# EUROPEAN PATENT SPECIFICATION

(11) **EP 2 718 261 B1**
(45) Date of publication and mention of the grant of the patent: **24.02.2016**
(21) Application number: 12728010.5
(22) Date of filing: 08.06.2012
(51) Int. Cl.: C07C 237/08, A61K 31/16, A61P 1/16, C07C 237/22, C07C 323/60, C07C 333/04, A61K 9/127

(54) **COMPOUNDS FOR TARGETING DRUG DELIVERY AND ENHANCING SIRNA ACTIVITY**
VERBINDUNGEN FÜR DIE GEZIELTE WIRKSTOFFFREISETZUNG UND VERBESSERUNG DER SIRNA-WIRKUNG
COMPOSÉS POUR L'ADMINISTRATION DE MÉDICAMENT CIBLÉE ET L'AUGMENTATION DE L'ACTIVITÉ ARNSI

(30) Priority: 08.06.2011 US 201161494710 P; 08.06.2011 US 201161494840 P
(43) Date of publication of application: 16.04.2014
(60) Divisional application: 15181729.3
(73) Proprietor: Nitto Denko Corporation, Ibaraki-shi Osaka 567-8680 (JP)
(72) Inventor: SRIDHAR, C. Nagarajan, San Diego, CA 92130 (US); KARMALI, Priya, San Diego, CA 92122 (US); NIITSU, Yoshiro, Hokkaido 064-0823 (JP); PAYNE, Joseph E., Oceanside, CA 92057 (US); HOU, Zheng, San Diego, CA 92129 (US); GAUDETTE, John A., Poway, CA 92064 (US); KNOPOV, Victor, Oceanside, CA 92056 (US); WITTE, Richard P., San Diego, CA 92110 (US); AHMADIAN, Mohammad, Carlsbad, CA 92009 (US); PERELMAN, Loren A., San Diego, CA 92119 (US); TANAKA, Yasunobu, Osaka, 567-8680 (JP); AKOPIAN, Violetta, Oceanside, CA 92056 (US)
(74) Representative: Hoffmann Eitle
(86) International application number: PCT/US2012/041753
(87) International publication number: WO 2012/170952

(56) References cited:
- WO-A1-2010/061880
- DATABASE CA [Online] CHEMICAL ABSTRACTS SERVICE, COLUMBUS, OHIO, US; HORIMOTO, HIKARI ET AL: "Liquid crystal compositions", XP002684897, retrieved from STN Database accession no. 1987:11469 & JP 61 089286 A (TOKUYAMA SODA CO., LTD., JAPAN) 7 May 1986 (1986-05-07)
- DATABASE CA [Online] CHEMICAL ABSTRACTS SERVICE, COLUMBUS, OHIO, US; HORIMOTO, HIKARI ET AL: "Liquid crystal compositions", XP002684898, retrieved from STN Database accession no. 1987:59428 & JP 61 136584 A (TOKUYAMA SODA CO., LTD., JAPAN) 24 June 1986 (1986-06-24)
- DATABASE CA [Online] CHEMICAL ABSTRACTS SERVICE, COLUMBUS, OHIO, US; TAGAMI, TATSUAKI ET AL: "Effect of siRNA in PEG-coated siRNA -lipoplex on anti-PEG IgM production", XP002684899, retrieved from STN Database accession no. 2009:776119 -& TAGAMI T ET AL: "Effect of siRNA in PEG-coated siRNA-lipoplex on anti-PEG IgM production", JOURNAL OF CONTROLLED RELEASE, ELSEVIER, AMSTERDAM, NL, vol. 137, no. 3, 4 August 2009 (2009-08-04), pages 234-240, XP026223536, ISSN: 0168-3659, DOI: 10.1016/J.JCONREL.2009.04.006 [retrieved on 2009-04-08]
- DATABASE CA [Online] CHEMICAL ABSTRACTS SERVICE, COLUMBUS, OHIO, US; 8 April 2011 (2011-04-08), HADA, MAGDOLNA ET AL: "The first synthesis of PEG-carotenoid conjugates", XP002690323, retrieved from STN Database accession no. 2011:648518 & MAGDOLNA HDA ET AL: "The first synthesis of PEGcarotenoid conjugates", TETRAHEDRON LETTERS, ELSEVIER, AMSTERDAM, NL, vol. 52, no. 25, 8 April 2011 (2011-04-08) , pages 3195-3197, XP028212363, ISSN: 0040-4039, DOI: 10.1016/J.TETLET.2011.04.039 [retrieved on 2011-04-15]
- DATABASE CA [Online] CHEMICAL ABSTRACTS SERVICE, COLUMBUS, OHIO, US; 2003, BENTLEY, MICHAEL DAVID ET AL: "Water-soluble polymer conjugates of retinoic acid", XP002690324, retrieved from STN Database accession no. 2003:356296 & WO 03/037385 A1 (SHEARWATER CORP [US]; BENTLEY MICHAEL DAVID [US]; ZHAO XUAN [US]; LEAC) 8 May 2003 (2003-05-08)
- DATABASE CA [Online] CHEMICAL ABSTRACTS SERVICE, COLUMBUS, OHIO, US; 1999, SCHREIBER, JORG ET AL: "Disperse multiphase systems linked by covalent bonds into a network", XP002690325, retrieved from STN Database accession no. 1999:12437
- DATABASE CA [Online] CHEMICAL ABSTRACTS SERVICE, COLUMBUS, OHIO, US; 1998, DIEC, KHIET HIEN ET AL: "Cosmetic or dermatological crosslinked microemulsions", XP002690326, retrieved from STN Database accession no. 1998:239092 & DE 196 41 672 A1 (BEIERSDORF A.-G., GERMANY; DIEC, KHIET HIEN; MEIER, WOLFGANG; SCHREIBE) 16 April 1998 (1998-04-16)
- DATABASE CA [Online] CHEMICAL ABSTRACTS SERVICE, COLUMBUS, OHIO, US; 1998, SCHREIBER, JOERG ET AL: "Crosslinked structures with double lipid membranes or based on peptides", XP002690327, retrieved from STN Database accession no. 1998:208404 -& WO 98/13025 A1 (BEIERSDORF A.-G., GERMANY; SCHREIBER, JOERG; MEIER, WOLFGANG) 2 April 1998 (1998-04-02)
- MOON ET AL: "Regulation of adipocyte differentiation by PEGylated all-trans retinoic acid: reduced cytotoxicity and attenuated lipid accumulation", JOURNAL OF NUTRITIONAL BIOCHEMISTRY, BUTTERWORTH PUBLISHERS, STONEHAM, GB, vol. 18, no. 5, 19 April 2007 (2007-04-19) , pages 322-331, XP022035106, ISSN: 0955-2863, DOI: 10.1016/J.JNUTBIO.2006.06.004
- LI JING ET AL: "Synthesis of polyethylene glycol (PEG) derivatives and PEGylated-peptide biopolymer conjugates", BIOMACROMOLECULES, AMERICAN CHEMICAL SOCIETY; US, vol. 4, no. 4, 17 May 2003 (2003-05-17), pages 1055-1067, XP002328259, ISSN: 1525-7797, DOI: 10.1021/BM034069L
- DATABASE CA [Online] CHEMICAL ABSTRACTS SERVICE, COLUMBUS, OHIO, US; NIITSU, YOSHIRO ET AL: "Retinoid-liposomes for enhancing modulation of human hsp47 expression for treatment of fibrosis", XP002690328, retrieved from STN Database accession no. 2012:1819002 & WO 2012/170957 A2 (NITTO DENKO CORP [JP]; QUARK PHARMACEUTICALS INC [US]; NNTSU YOSHIRO [) 13 December 2012 (2012-12-13)
- 'Print-out of Utah Valley University chemical dicitionary for entry "alkenyl"'
- 'Print-out of Wikipedia entry for "alkene" in English and German'
- "Advances in Oxygen Research and Application", 2013, ScholarlyEditions
- YANG K ET AL: "Systematic analysis of choline-containing phospholipids using multi-dimensional mass spectrometry-based shotgun lipidomics", JOURNAL OF CHROMATOGRAPHY B: BIOMEDICAL SCIENCES & APPLICATIONS, ELSEVIER, AMSTERDAM, NL, vol. 877, no. 26, 15 September 2009 (2009-09-15), pages 2924-2936, ISSN: 1570-0232 [retrieved on 2009-01-21]
- Keiko Yamada ET AL: "Studies on the composition of phospholipids in rat small intestinal smooth muscle", Journal of Biochemistry, 1 April 1976 (1976-04-01), pages 809-817, Retrieved from the Internet: URL:https://www.jstage.jst.go.jp/article/b iochemistry1922/79/4/79_4_809/_pdf [retrieved on 2015-07-01]
- NATALIA ZHUKOVA: "Lipids and Fatty Acids of Nudibranch Mollusks: Potential Sources of Bioactive Compounds", MARINE DRUGS, vol. 12, no. 8, 19 August 2014 (2014-08-19), pages 4578-4592, DOI: 10.3390/md12084578
- Wolfgang J Baumann ET AL: "Synthesis of long-chain alkyl and alkenyl bromides", Journal of Lipid Research, 1 July 1966 (1966-07-01), page 568, UNITED STATES Retrieved from the Internet: URL:http://www.jlr.org/cgi/content/abstrac t/7/4/568

## Description

### CROSS REFERENCE TO RELATED APPLICATIONS

This application claims the benefit of United States Provisional Application Nos. 61/494,840 and 61/494,710, filed June 8, 2011.

### TECHNICAL FIELD

The present invention is directed to the use of cationic lipids and fat-soluble vitamin compounds to target and enhance activity of therapeutic molecules, including siRNA.

### BACKGROUND

Fibrosis of the liver can be caused by activated hepatic stellate cells (HSC), resulting in a plurality of types of collagen molecules and fibronectin being deposited on interstitial tissue.. This can lead to hepatic cirrhosis, hepatic failure, and/or hepatocellular carcinoma. Further, chronic pancreatitis develops as a result of pancreatic fibrosis by the same mechanism as that for hepatic fibrosis (Madro, et al., 2004; Med Sci Monit. 10:RA166-70.; Jaster, 2004, Mol Cancer. 6:26). Furthermore, stellate cells are present in the vocal cord disorders of the vocal cord and larynx such as vocal cord scarring, vocal cord mucosal fibrosis, and laryngeal fibrosis.. To prevent or treat fibrosis in these organs and elsewhere in the body, there is a desire for the development of a drug carrier and drug carrier kit.

Stellate cells are one of the important target candidates for treating fibrosis (Fallowfield et al., 2004, Expert Opin Ther Targets. 8:423-35; Pinzani, et al., 2004, Dig Liver Dis.36:231-42). During fibrosis, stellate cells are activated by cytokines from nearby cells and are activated. Stellate cells are known as storage cells for vitamin A, and belong to the myofibroblast family, and produce many factors that cause hepatic fibrosis.

Therapeutic methods to prevent or treat fibrosis attempt to control collagen metabolism, promotion of the collagen degradation system, and inhibition of activation of stellate cells, However, in all cases, since the specificity of action and/or the organ specificity are low, there are problems with the limited efficacy and with adverse side effects.

Inhibition of collagen protein synthesis has not been established as a therapeutic method. The potency of molecules targeting collagen production is limited because of the possibility of causing side effects. Inhibiting collagen production directly would be an obvious therapeutic method to prevent or treat fibrosis. To do this would require control of one or more of the various types of collagen Types I to IV. A method for accomplishing this may be through HSP47, a collagen-specific molecular chaperone that is essential for intracellular transport and molecular maturation necessary for various types of collagen. Therefore, if the function of HSP47 can be controlled specifically in stellate cells of an organ, there is a possibility of inhibiting hepatic fibrosis in the organ.

A number of techniques are available for delivering a therapeutic agent such as siRNA into a cell, including the use of viral transfection systems and non-viral transfection systems. Non-viral transfection systems can include, for example, polymers, lipids, liposomes, micelles, dendrimers, and nanomaterials. Examples of polymers that have previously been studied for cell transfection include cationic polymers such as poly(L-lysine) (PLL), polyethyleneimine (PEI), chitosan, and poly(2-dimethylamino)ethyl methacrylate (pDMAEMA). WO 2010/061880 A1 A describes *inter alia* vesicles containing a cationic lipid that is represented by the following formula (I)

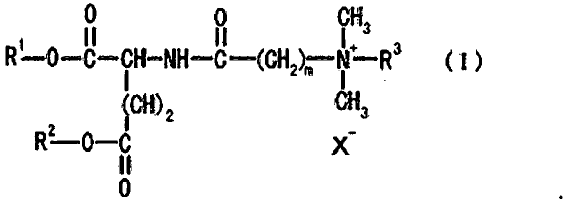

Each type of system has its respective advantages and drawbacks. For example, viral systems can yield high transfection efficiency, but may not be as safe as some non-viral systems. In addition, viral systems can be complicated and/or expensive to prepare. Non-viral transfection systems, such as cationic polymers, have been reported to transfer plasmid DNA into cells. However, some drawbacks to the use of cationic polymers include their toxicity to cells and/or their lack of stability.

As such, there is a pressing need for new compounds, compositions, and methods of using cationic components to improve delivery of therapeutic drugs, including nucleic acids, to cells, tissues and organisms.

### SUMMARY

One aspect of the description are compounds of formula I 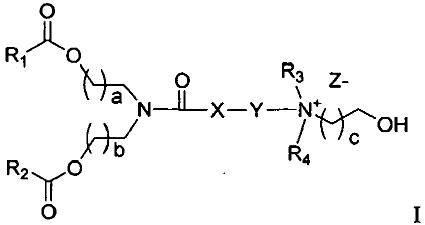 wherein R₁ and R₂ is independently selected from a group consisting of C₁₂ to C₁₈ alkyl, C₁₂ to C₁₈ alkenyl, and oleyl group; wherein R₃ and R₄ are independently selected from a group consisting of C₁ to C₆ alkyl, and C₂ to C₆ alkanol; wherein X is selected from a group consisting of-CH₂-, -S-, and -O-, or absent; wherein Y is selected from -(CH₂)ₙ, -S(CH₂)ₙ, -O(CH₂)ₙ-, thiophene, and -SO₂(CH₂)ₙ-, wherein n = 1-4; wherein a = 1-4; wherein b=1-4; wherein c=1-4; and wherein Z is a counterion. Further aspects of the present invention are specified in the appended claims.

Disclosed herein is also a compound for facilitating drug delivery to a target cell, consisting of the structure (targeting molecule)ₘ-linker-(targeting molecule)ₙ, wherein the targeting molecule is a retinoid having a specific receptor or activation/binding site on the target cell; wherein m and n are independently 0, 1, 2, or 3; and wherein the linker comprises a polyethylene glycol (PEG) or PEG-like molecule

According to one embodiment of the present disclosure, the retinoid is selected from the group consisting of vitamin A, retinoic acid, tretinoin, adapalene, 4-hydroxy(phenyl)retinamide (4-HPR), retinyl palmitate, retinal, saturated retinoic acid, and saturated, demethylated retinoic acid.

In another embodiment of the present disclosure, the linker is selected from the group consisting of bis-amido-PEG, tris-amido-PEG, tetra-amido-PEG, Lys-bis-amido-PEG Lys, Lys-tris-amido-PEG-Lys, Lys-tetr-amido-PEG-Lys, Lys-PEG-Lys, PEG2000, PEG1250, PEG1000, PEG750, PEG550, PEG-Glu, Glu, C6, Gly3, and GluNH.

In another embodiment of the present disclosure, the compound is selected from the group consisting of retinoid-PEG-retinoid, (retinoid)₂-PEG-(retinoid)₂, VA-PEG2000-VA, (retinoid)₂-bis-amido-PEG-(retinoid)₂, (retinoid)₂-Lys-bis-amido-PEG-Lys-(retinoid)₂.

In another embodiment of the present disclosure, the retinoid is selected from the group consisting of vitamin A, retinoic acid, tretinoin, adapalene, 4-hydroxy(phenyl)retinamide (4-HPR), retinyl palmitate, retinal, saturated retinoic acid, and saturated, demethylated retinoic acid.

In another embodiment of the present disclosure, the compound is a composition of the formula 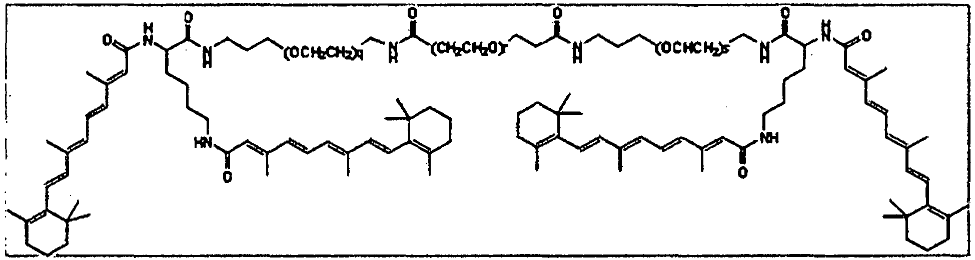 wherein q, r, and s are each independently 1, 2, 3, 4, 5, 6, 7, 8, 9, or 10.

In another embodiment of the present disclosure, the formula of the compound comprises

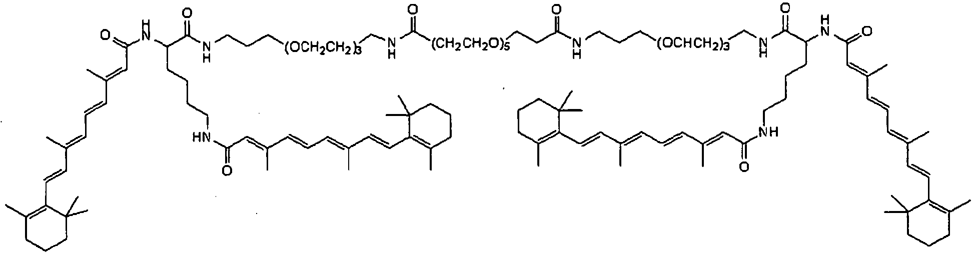

In another aspect, the present disclosure provides a stellate-cell-specific drug carrier comprising a stellate cell specific amount of a retinoid molecule consisting of the structure (retinoid)ₘ-linker-(retinoid)ₙ; wherein m and n are independently 0, 1, 2, or 3; and wherein the linker comprises a polyethylene glycol (PEG) or PEG-like molecule.

In another embodiment, the present invention provides a composition comprising a liposomal composition. In other embodiments of the present disclosure, the liposomal composition comprises a lipid vesicle comprising a bilayer of lipid molecules.

In certain embodiments of the present disclosure, the retinoid molecule is at least partially exposed on the exterior of the drug carrier before the drug carrier reaches the stellate cell.

In another embodiment of the present disclosure, the retinoid is 0.1 mol% to 20 mol% of the lipid molecules.

In the present invention also provides embodiments where the lipid molecules comprise one or more lipids as specified in the appended claims. Further disclosed are lipids selected from the group consisting of HEDC, DODC, HEDODC, DSPE, DOPE, and DC-6-14. In another embodiment, the lipid molecules further comprise S104.

In certain embodiments, the drug carrier comprises a nucleic acid.

In other embodiments, the nucleic acid is an siRNA that is capable of knocking down expression of hsp47 mRNA in the stellate cell.

In another aspect, the present disclosure provides a compound for facilitating drug delivery to a target cell, consisting of the structure (lipid)ₘ-linker-(targeting molecule)ₙ, wherein the targeting molecule is a retinoid or a fat soluble vitamin having a specific receptor or activation/binding site on the target cell; wherein m and n are independently 0, 1, 2, or 3; and wherein the linker comprises a polyethylene glycol (PEG) molecule.

In one embodiment of the present disclosure, the lipid is selected from one or more of the group consisting of DODC, HEDODC, DSPE, DOPE, and DC-6-14.

In another embodiment of the present disclosure, the retinoid is selected from the group consisting of vitamin A, retinoic acid, tretinoin, adapalene, 4-hydroxy(phenyl)retinamide (4-HPR), retinyl palmitate, retinal, saturated retinoic acid, and saturated, demethylated retinoic acid.

In another embodiment of the present disclosure, the fat-soluble vitamin is vitamin D, vitamin E, or vitamin K.

In another embodiment of the present disclosure, the linker is selected from the group consisting of bis-amido-PEG, tris-amido-PEG, tetra-amido-PEG, Lys-bis-amido-PEG Lys, Lys-tris-amido-PEG-Lys, Lys-tetr-amido-PEG-Lys, Lys-PEG-Lys, PEG2000, PEG1250, PEG1000, PEG750, PEG550, PEG-Glu, Glu, C6, Gly3, and GluNH.

In another embodiment of the present disclosure the compound is selected from the group consisting of DSPE-PEG-VA, DSPE-PEG2000-Glu-VA, DSPE-PEG550-VA, DOPE-VA, DOPE-Glu-VA, DOPE-Glu-NH-VA, DOPE-Gly3-VA, DC-VA, DC-6-VA, and AR-6-VA.

### BRIEF DESCRIPTION OF THE DRAWINGS

Fig. 1 depicts knockdown efficacy of certain embodiments of the description. This includes HEDC liposomes compared to DC-6-14 lipoplex controls.
Fig. 2 depicts an *in vitro* comparison of gene knockdown using cationic lipids.
Fig. 3 depicts an evaluation of gene expression *in vivo* with exemplary HEDODC liposome formulations of the invention (* indicates p<0.05).
Fig. 4 depicts an evaluation of gene expression *in vitro* with exemplary HEDC liposome formulations of the Example 15. Error bars indicate standard deviations (n=3). A sigmoidal dose-response curve is shown based on best fit. An EC₅₀ value was calculated from the curve. This is indicated to be 11.8 nM.
Fig. 5 shows the results of measurements *in vivo* using a rat DMNQ model. After subtracting background gp46 mRNA levels determined from the naive group, all test group values were normalized to the average gp46 mRNA of the vehicle group (expressed as a percent of the vehicle group). The mean gp46 mRNA level following treatment showed dose-dependent response and curve fitting to a sigmoidal dose response curve. The calculated ED₅₀ value is 0.79 mg/kg.
Fig. 6 shows the results of measurements *in vivo* using a rat DMNC model. After subtracting background gp46 mRNA levels determined from the naïve group, all test group values were normalized to the average gp46 mRNA of the vehicle group (expressed as a percent of the vehicle group). MRPL19 mRNA levels were determined by quantitative RT-PCR (TaqMan®) assay. mRNA levels for gp46 were normalized to MRPL19 levels. (*** indicates p<0.02.)
Fig. 7 shows the results of measurements *in vivo* using a rat pulmonary bleomycin model. The bar graph summarizes the fibrosis (T. Ashcroft) scoring of Azan-stained lung sections for each group. Statistical analysis was performed using a One way ANOVA, Bonferroni multi comparison test with Prism5 software.
Fig. 8 VA-conjugate addition to liposomes via decoration enhances siRNA activity
Fig. 9 VA-conjugate addition to liposomes via co-solubilization enhances siRNA activity
Fig. 10 VA-conjugate addition to liposomes via co-solubilization enhances siRNA activity
Fig. 11 VA-conjugate addition to lipoplexes via co-solubilization enhance siRNA activity
Fig. 12 VA-conjugate addition to lipoplexes via co-solubilization vs. decoration.

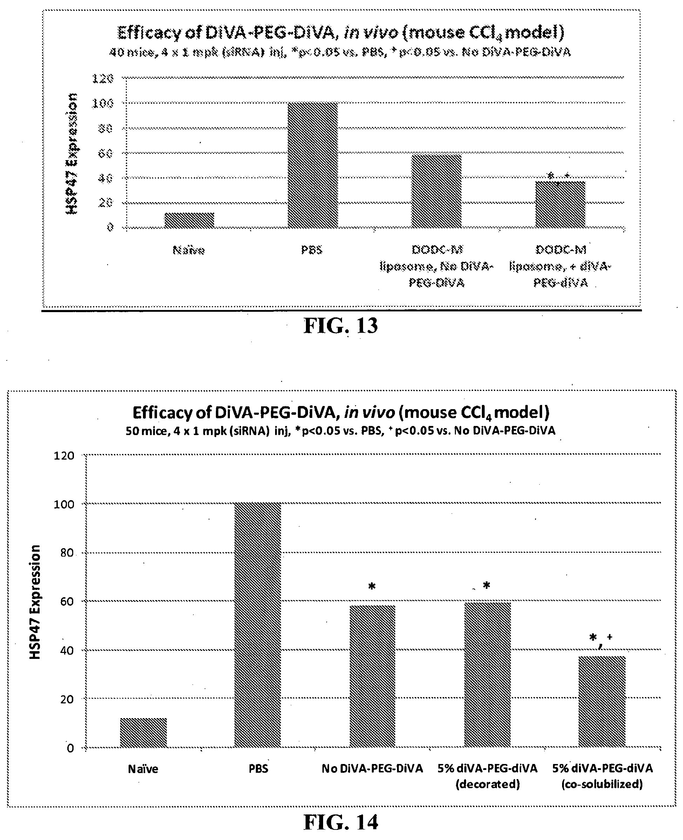

Fig. 13 *in vivo* efficacy in mouse, CCl₄ model
Fig. 14 *in vivo* efficacy of decorated vs. co-solubilized retinoid conjugates
Fig. 15 *in vitro* efficacy (pHSC), effect of retinoid conjugates in liposome formulations.
Fig. 16 shows the correlation of retinoid conjugate content (mol%) to *in vivo* (rat DMNQ) efficacy. Male Sprague-Dawley rats injected intravenously either with formulations containing 0, 0.25, 0.5, 1, and 2% DiVA at a dose of 0.75 mg/kg siRNA, or PBS (vehicle), one hour after the last injection of DMN.

### DETAILED DESCRIPTION OF ILLUSTRATIVE EMBODIMENTS

Within the scope of the invention are compounds of formula I 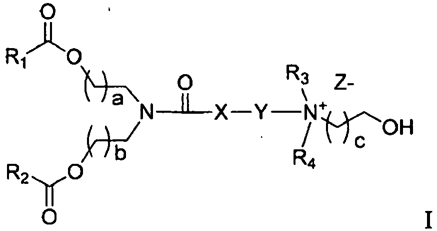 wherein R₁ and R₂ is independently selected from a group consisting of C₁₂ to C₁₈ alkyl, C₁₂ to C₁₈ alkenyl, and oleyl group; wherein R₃ and R₄ are independently selected from a group consisting of C₁ to C₆ alkyl, and C₂ to C₆ alkanol; wherein X is selected from a group consisting of -CH₂-, -S-, and -O-, or absent; wherein Y is selected from -(CH₂)ₙ, -S(CH₂)ₙ, -O(CH₂)ₙ-, thiophene, and -SO₂(CH₂)ₙ-, wherein n = 1-4; wherein a = 1-4; wherein b=1-4; wherein c=1-4; and wherein Z is a counterion.

Compounds of the invention are also referred to herein as being within the class of compounds known as "cationic lipids." Cationic lipids are compounds that include at least one lipid moiety and a positively charged nitrogen associated with a counterion. "Lipids" are understood in the art to be comprised of a hydrophobic alkyl or alkenyl moiety and a carboxylic acid or ester moiety.

It has heretofore been discovered that the amino-alkyl-hydroxyl (-N-alkyl-OH) moiety of the compounds of formula I imparts properties to the formulations of the invention not previously seen with other cationic lipids previously reported. Formulations of the invention that include compounds of formula I result in superior reduction in protein expression, as compared to formulations that do not include compounds of formula I. Particularly surprising is the ability of formulations of the invention that include compounds of formula I to reduce the expression of HSP47.

Preferred compounds of the invention include those wherein R₁ and R₂ are each independently C₁₂-C₁₈ alkyl. Particularly preferred embodiments include those wherein R₁ and R₂ are each independently C₁₃-C₁₇ alkyl. Most preferred are those compounds wherein R₁ and R₂ are each C₁₃ alkyl.

In other embodiments, R₁ and R₂ are each independently C₁₂-C₁₈ alkenyl. In yet other embodiments, R₁ and R₂ are each independently C₁₃-C₁₇ alkenyl. Most preferred compounds of the invention include those wherein R₁ and R₂ are each C₁₇ alkenyl.

Also for compounds of formula I, R₃ and R₄ are independently selected from a group consisting of C₁ to C₆ alkyl. In preferred embodiments, R₃ and R₄ are each independently C₁-C₃ alkyl. Most preferably, R₃ and R₄ are each methyl. In other embodiments, at least one of R₃ and R₄ are -CH₂CH₂OH.

Most preferred are those compounds of formula I wherein a = b = c = 1.

Z can be any nitrogen counterion, as that term is readily understood in the art. Preferred nitrogen counterions include halogens, with chloride and bromide being particularly preferred and mesylate (SO₃CH₃⁻). In contrast to other cationic lipids previously described wherein the effect of the cationic lipid depends on the counterion, the efficacy of compounds of formula I, surprisingly, do not appear to be related to the counterion selected.

Exemplary compounds of formula I include:

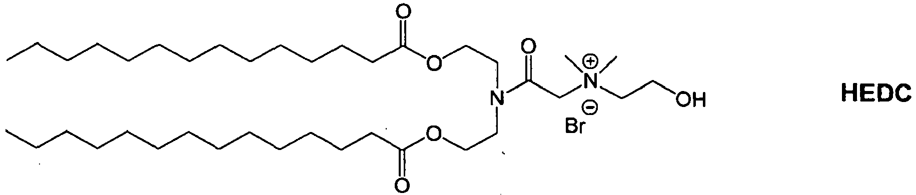

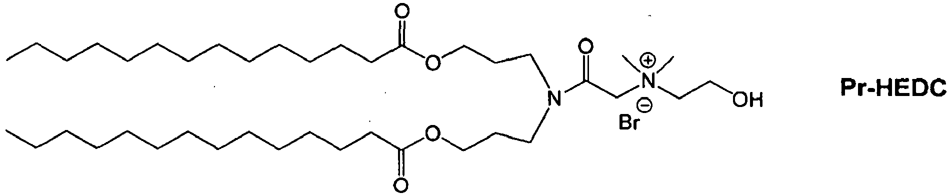

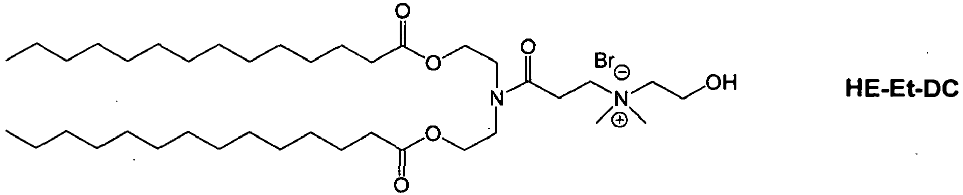

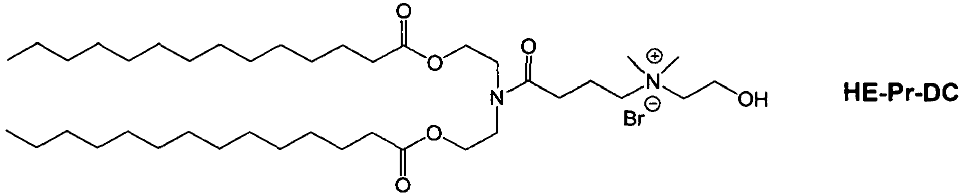

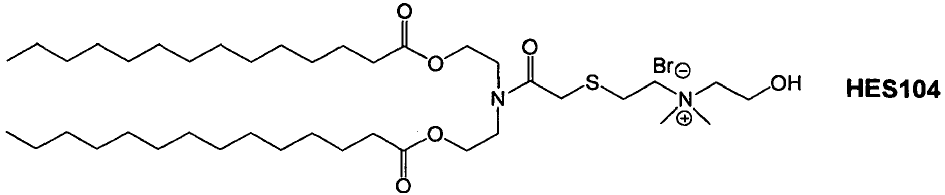

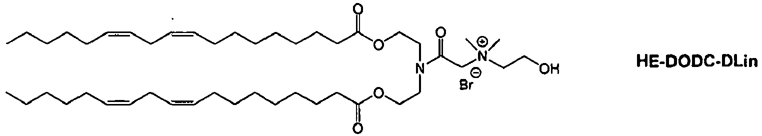

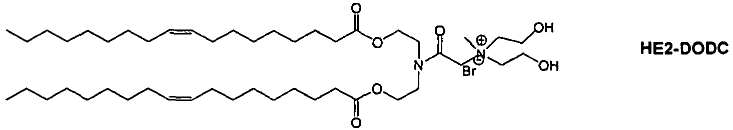

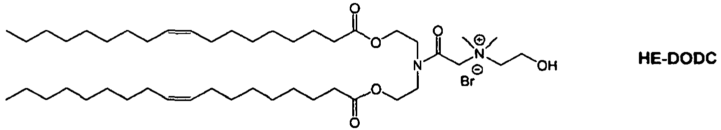

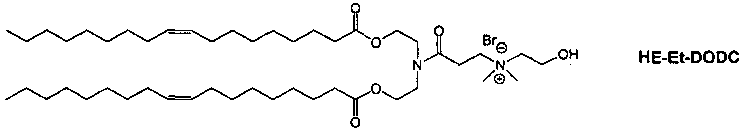

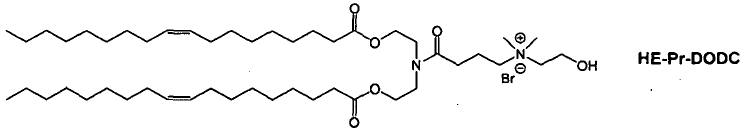

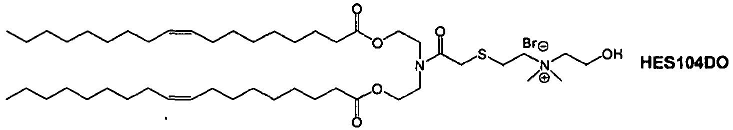

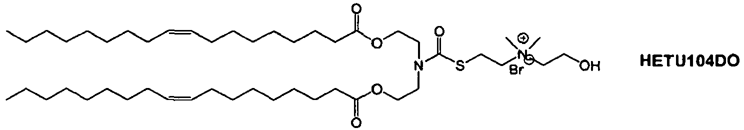

Disclosed herein is a compound for facilitating drug delivery to a target cell, consisting of the structure (targeting molecule)ₘ-linker-(targeting molecule)ₙ, wherein the targeting molecule is a retinoid or a fat soluble vitamin having a specific receptor or [activation/binding site] on the target cell; and wherein m and n are independently 0, 1, 2, or 3;and wherein the linker comprises a polyethylene glycol (PEG) or PEG-like molecule and is designated "Formula A".

The present disclosure also includes a compound for facilitating drug delivery to a target cell, consisting of the structure (lipid)ₘ-linker-(targeting molecule)ₙ, wherein the targeting molecule is a retinoid or a fat soluble vitamin having a specific receptor on the target cell; wherein m and n are independently 0, 1,2, or 3;and wherein the linker comprises a polyethylene glycol (PEG) PEG-like molecule and is designated "Formula B".

It has heretofore been discovered that the compounds of Formula A or Formula B impart properties to the formulations of the invention not previously seen. Formulations of the invention that include compounds of Formula A or Formula B result in superior reduction in gene expression, as compared to formulations that do not include these compounds. Particularly surprising is the ability of formulations disclosed herein that include compounds of Formula A to reduce the expression of HSP47.

In certain embodiments of the present disclosure, the retinoid is selected from the group consisting of vitamin A, retinoic acid, tretinoin, adapalene, 4-hydroxy(phenyl)retinamide (4-HPR), retinyl palmitate, retinal, saturated retinoic acid, and saturated, demethylated retinoic acid.

Embodiments of the present disclosure include compounds where the linker is selected from the group consisting of bis-amido-PEG, tris-amido-PEG, tetra-amido-PEG, Lys-bis-amido-PEG Lys, Lys-tris-amido-PEG-Lys, Lys-tetra-amido-PEG-Lys, Lys-PEG-Lys, PEG2000, PEG1250, PEG1000, PEG750, PEG550, PEG-Glu, Glu, C6, Gly3, and GluNH. In other embodiments, the PEG is mono disperse.

Disclosed herein is also a compound where Formula A is selected from the group consisting of retinoid-PEG-retinoid, (retinoid)₂-PEG-(retinoid)₂, VA-PEG2000-VA, (retinoid)₂-bis-amido-PEG-(retinoid)₂, (retinoid)₂-Lys-bis-amido-PEG-Lys-(retinoid)₂.

In another embodiment of the present disclosure, the compound is the formula 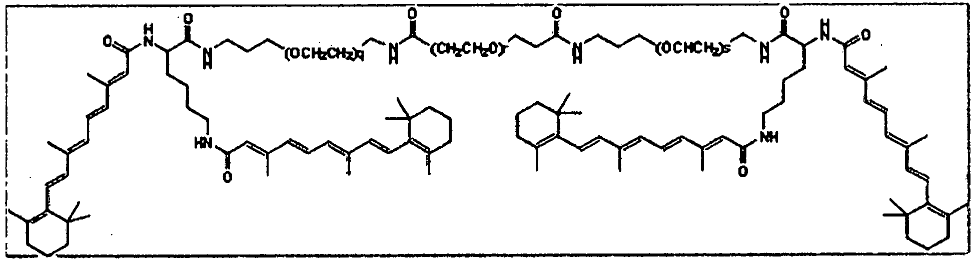 wherein q, r, and s are each independently 1, 2, 3, 4, 5, 6, 7, 8, 9, or 10.

In other embodiments of the present disclosure, the formula of the compound comprises

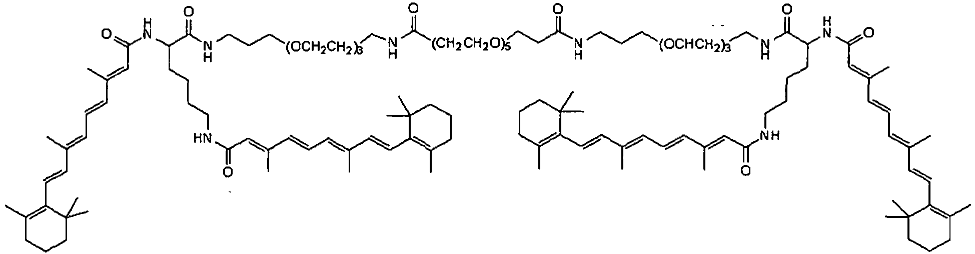

Other embodiments of the present disclosure include the structures shown in Table 1

**Table 1**

| Lipid Name | Compound Structure |
|---|---|
| SatDiVA | 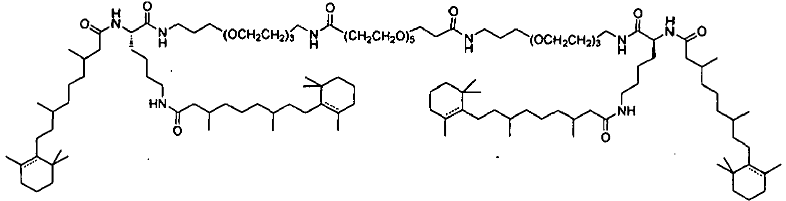 |
| SimDiVA | 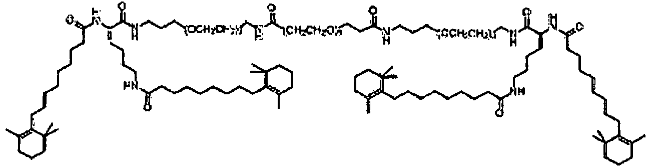 |
| DiVA-PEG18 | 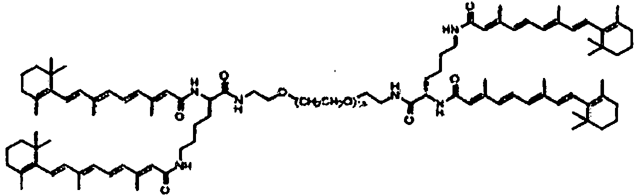 |
| TriVA | 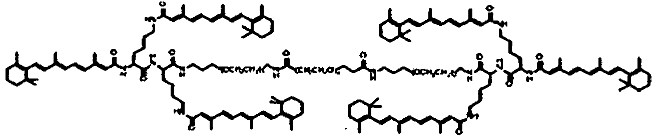 |
| 4TTNPB | 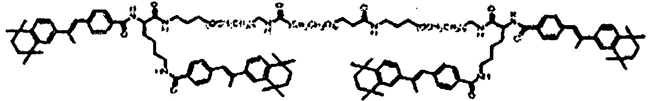 |
| 4Myr | 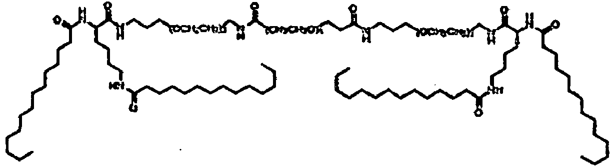 |
| DiVA-242 | 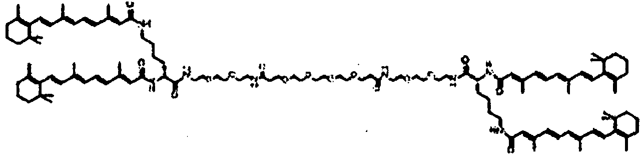 |

Compositions of the invention that include at least one compound of formula I and a liposome may further comprise one or more retinoid conjugates. In preferred embodiments of the invention, the retinoid conjugate will be present at a concentration of about 0.3 to about 30 weight percent, based on the total weight of the composition or formulation, which is equivalent to about 0.1 to about 10 mol.%, which is equivalent to a molar ratio of about 0.1 to about 10. Preferably, the retinoid conjugate is a retinoid-linker-lipid molecule or a retinoid-linker-retinoid molecule.

An example of a retinoid conjugates include those compounds of formula II: 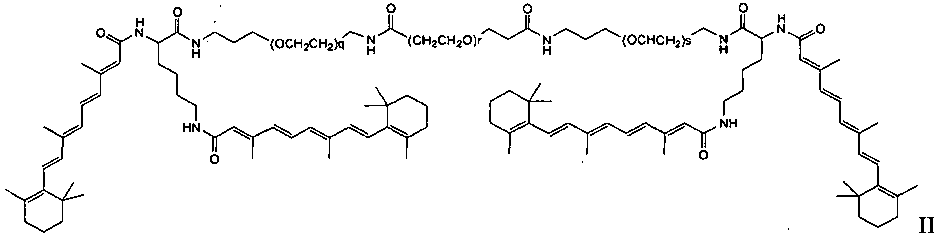 wherein q, r, and s are each independently 1, 2, 3, 4, 5, 6, 7, 8, 9, or 10, and enantiomers and diastereomers thereof.

Preferred compounds of formula II include those wherein q, r, and s are each independently 1, 2, 3, 4, 5, 6, or 7. More preferred are those compounds of formula II wherein q, r, and s are each independently 3, 4, or 5. Most preferred are those compounds of formula II wherein q is 3, r is 5, and s is 3. One example of a compound of formula II is

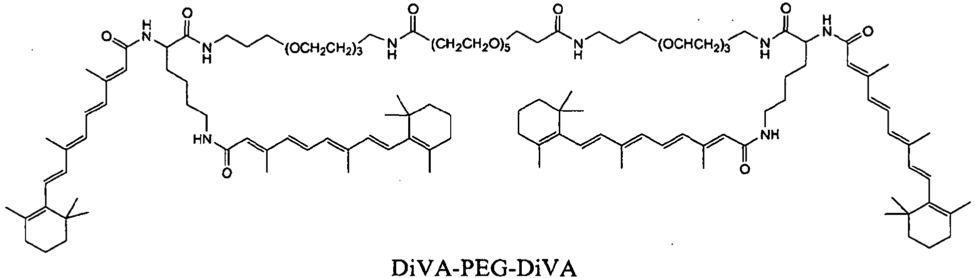

DiVA-PEG-DiVA includes stereocenters and all enantiomers and diastereomers are considered to be within the scope of the invention.

The concentration of cationic lipids of formula I in compositions of the invention, can be from 1 to about 80 weight percent, based on the total weight of the lipid composition. More preferably, the concentration is from 1 to about 75 weight percent. Even more preferably, the concentration is from about 30 to about 75 weight percent. A concentration of from about 30 to about 75 weight percent corresponds to about 30 to 60 mol.% and a molar ratio of about 30-60. Most preferred are those compositions having a cationic lipid concentration of about 50 weight percent. In formulations that contain a mixture of an ionizable cationic lipid and a quaternary amine cationic lipid of formula I, the preferred mol% is from 5% to 45 mol%, with even more preferred mixture of approximately 20 mol% of the ionizable cationic lipid and 20 mol% of the quaternary amine cationic lipid for formula I.

Such compositions may also include an aqueous medium. The cationic lipids, including those of formula I, can be encapsulated within the liposome in such embodiments and may be inaccessible to the aqueous medium. Furthermore, the cationic lipids, including those of formula I, can be localized on the outer surface of the liposome and be accessible to the aqueous medium.

Compositions of the invention that include at least one compound of formula I and a liposome, and optionally a retinoid conjugate such as a compound of formula II, can also include siRNA. Also within the scope of the invention are formulations including at least one compound of formula I and further comprising at least one compound of Formula A or B and siRNA.

It is envisioned that any siRNA molecule can be used within the scope of the invention. The siRNA may include an antisense sequence to the mRNA coding sequence for human hsp47 exemplified by SEQ ID NO:1, which is shown as follows.

For example,
Sense (5'->3') GGACAGGCCUCUACAACUATT (SEQ. ID. NO. 2)
Antisense (3'->5') TTCCUGUCCGGAGAUGUUGAU (SEQ. ID. NO. 3).

Such compositions may also include an aqueous medium. Preferably, such compositions consist essentially of at least one compound of formula I in a charge complex with the siRNA. Such compositions including a compound of formula I and an siRNA can further comprise a liquid medium. In one embodiment, the liquid medium is suitable for injection into a living organism. Liquid mediums within the scope of any of the described embodiments of the invention can be aqueous, that is, be comprises entirely of an aqueous solvent, and to include salts, buffers, and/or other pharmaceutical excipients. In another embodiment, the liquid medium may consist of an aqueous solvent in combination with another liquid solvent such as, for example, an organic solvent. Liquid mediums within the scope of any of the described embodiments of the invention can also include at least one organic solvent. Organic solvents are known in the art *per se* and include C₁ to C₄ alcohols, dimethyl sulfoxide ("DMSO"), and the like. Those liquid mediums that include a mixture of water and at least one organic solvent are also within the scope of any of the described embodiments of the invention.

Also within the scope of the invention are compositions that comprise at least one compound of formula I and a liposome. Some embodiments can include mixtures of compounds of formula I and a liposome. Other embodiments can include a liposome and one or more compounds of formula I in addition to cationic lipids that are not within the scope of formula I.

In some embodiments, the siRNA will be encapsulated by the liposome so that the siRNA is inaccessible to the aqueous medium. In other embodiments, the siRNA will not be encapsulated by the liposome. In such embodiments, the siRNA can be complexed on the outer surface of the liposome. In these embodiments, the siRNA is accessible to the aqueous medium.

Other embodiments include a stellate-cell-specific drug carrier comprising a liposomal composition. The liposomal composition can comprise a lipid vesicle comprising a bilayer of lipid molecules. In certain embodiments it may preferred that the retinoid molecule is at least partially exposed on the exterior of the drug carrier before the drug carrier reaches the stellate cell.

Certain embodiments of the present disclosure provide that the lipid molecules comprise one or more lipids selected from the group consisting of HEDC, DODC, HEDODC, DSPE, DOPE, and DC-6-14. In other embodiments, the lipid molecules can further comprise S104.

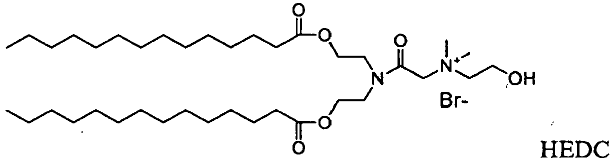

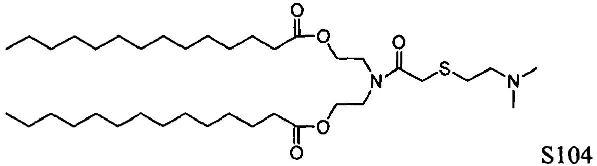

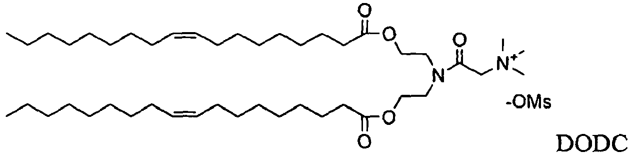

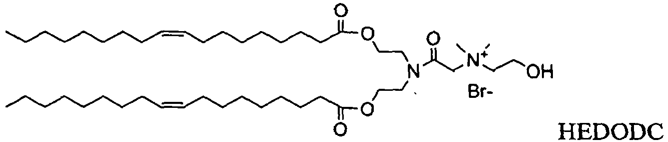

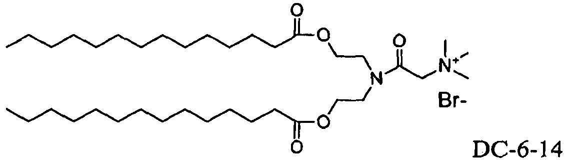

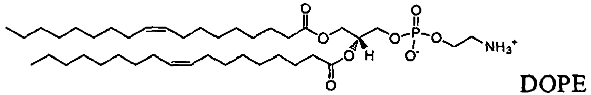

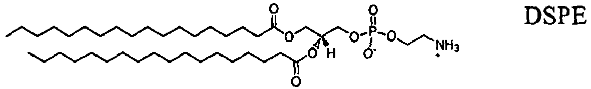

In some embodiments, the siRNA will be encapsulated by the liposome so that the siRNA is inaccessible to the aqueous medium. In other embodiments, the siRNA will not be encapsulated by the liposome. In such embodiments, the siRNA can be complexed on the outer surface of the liposome. In these embodiments, the siRNA is accessible to the aqueous medium.

Other embodiments include stellate-cell-specific drug carrier comprising a liposomal composition. The liposomal composition can comprise a lipid vesicle comprising a bilayer of lipid molecules. In other embodiments, the retinoid molecule is at least partially exposed on the exterior of the drug carrier before the drug carrier reaches the stellate cell.

In certain preferred embodiments, the retinoid is 0.1 mol% to 20 mol% of the lipid molecules.

The forgoing compositions can also include PEG-conjugated lipids, which are known in the art *per se,* including PEG-phospholipids and PEG-ceramides, including one or more molecules selected from the following: PEG2000-DSPE, PEG2000-DPPE, PEG2000-DMPE, PEG2000-DOPE, PEG1000-DSPE, PEG1000-DPPE, PEG1000-DMPE, PEG1000-DOPE, PEG550-DSPE, PEG550-DPPE, PEG-550DMPE, PEG-1000DOPE, PEG-cholesterol, PEG2000-ceramide, PEG1000-ceramide, PEG750-ceramide, and PEG550-ceramide.

The foregoing compositions of the invention can include one or more phospholipids such as, for example, 1,2-Distearoyl-sn-glycero-3-phosphocholine ("DSPC"), dipalmitoylphosphatidylcholine ("DPPC"), 1,2-Dipalmitoyl-*sn*-glycero-3-phosphoethanolamine ("DPPE"), and 1,2-Dioleoyl-*sn*-glycero-3-phosphoethanolamine ("DOPE"). Preferably, the helper phospholipid is DOPE.

For example, liposomes within the scope of the invention were prepared using various PEG-lipids, incorporated using co-solubilization methods described herein. These formulations comprised cationic lipid:DOPE:cholesterol:DiVA-PEG-DiVA:PEG-Lipid (50:10:38:5:2 molar ratio) and each formulation was tested in the pHSC *in vitro* assay described herein using human/rat HSP-47-C siRNA at a concentration of 200 nM. The results are shown in the following table:

| **PEG-Lipid** | **gp46 Gene Knockdown (%)** | **Std. Dev.** |
|---|---|---|
| Untreated | 0 | 5.6 |
| RNAimax Control | 50.0 | 7.9 |
| PEG-BML | 95.5 | 1.7 |
| PEG1000-DMPE | 93.2 | 0.8 |
| PEG1000-DPPE | 92.8 | 1.3 |
| PEG1000-DSPE | 93.5 | 0.8 |
| PEG1000-DOPE | 90.7 | 2.5 |
| PEG2000-Ceramide | 91.8 | 1.0 |
| PEG2000-DMPE | 93.7 | 3.4 |
| PEG2000-DPPE | 91.1 | 1.4 |
| PEG2000-DSPE | 89.4 | 1.7 |

The foregoing compositions of the invention can include one or more phospholipids such as, for example, 1,2-distearoyl-sn-glycero-3-phosphocholine ("DSPC"), dipalmitoylphosphatidylcholine ("DPPC"), 1,2-dipalmitoyl-*sn*-glycero-3-phosphoethanolamine ("DPPE"), and 1,2-dioleoyl-*sn*-glycero-3-phosphoethanolamine ("DOPE"). Preferably, the helper lipid is DOPE.

In addition to the cationic lipid of Formula I, other lipids may be useful. These include ionizable cationic lipids, including S104, shown below.

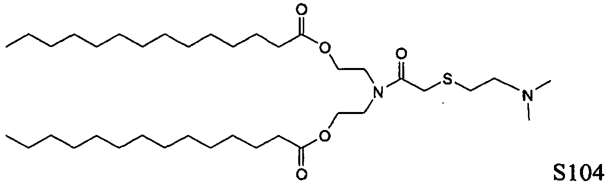

Delivery formulations may consist of a cationic lipid of Formula I in combination with an ionizable cationic lipid. An ionizable cationic lipid may include, e.g., S104. The ionizable cationic lipid may be present at a concentration of 0 to 45 mol %, including a concentration selected from 5, 10, 15, 20, 25, 30, 35, 40, and 45 mol %.

Also within the scope of the present disclosure are pharmaceutical formulations that include any of the aforementioned compositions in addition to a pharmaceutically acceptable carrier or diluent. Pharmaceutical formulations of the present disclosure will include at least one therapeutic agent. Preferably, the therapeutic agent is an siRNA. It is envisioned that any siRNA molecule can be used within the scope of the present disclosure.

Also within the scope of the present disclosure are pharmaceutical formulations that include any of the aforementioned compounds in addition to a pharmaceutically acceptable carrier or diluent. Pharmaceutical formulations of the present disclosure will include at least one therapeutic agent. Preferably, the therapeutic agent is an siRNA. It is envisioned that any siRNA molecule can be used within the scope of the present disclosure. As previously described, siRNA include the sequences of shown as SEQ ID NO: 1, SEQ ID NO: 2, SEQ ID NO: 3, and SEQ ID NO: 4.

In preferred formulations of the present disclosure including siRNA, the siRNA is encapsulated by the liposome. In other embodiments, the siRNA can be outside of the liposome. In those embodiments, the siRNA can be complexed to the outside of the liposome.

A useful range of cationic lipid:siRNA (lipid nitrogen to siRNA phosphate ratio, "N:P") is 0.2 to 5.0. Particularly preferred range of N:P is 1.5 to 2.5 for compositions and formulations of the present disclosure.

Preferred formulations of the invention include those comprising HEDC:S104:DOPE:Cholesterol:PEG-DMPE:DiVA-PEG-DiVA (20:20:30:25:5:2 molar ratio) and HEDC:S104:DOPE:Cholesterol:PEG-DMPE:DiVA-PEG-DiVA (20:20:30:25:5:2 molar ratio) wherein DiVA-PEG-DiVA is co-solubilized. Also disclosed are DODC:DOPE:cholesterol:Peg-Lipid:DiVA-PEG-DiVA (50:10:38:2:5 molar ratio) and DODC:DOPE:cholesterol:Peg-Lipid:DiVA-PEG-DiVA formulations wherein the DiVA-PEG-DiVA is co-solubilized.

Other formulations of the invention include those comprising HEDODC:DOPE:cholesterol-PEG-lipid:DiVA-PEG-DiVA (50:10:38:2:5 molar ratio) and HEDODC:DOPE:cholesterol-PEG-lipid:DiVA-PEG-DiVA formulations wherein the DiVA-PEG-DiVA is co-solubilized.

Other preferred formulations of the present disclosure include those comprising DC-6-14:DOPE:cholesterol: DiVA-PEG-DiVA (40:30:30:5, molar ratios) and DC-6-14:DOPE:cholesterol: DiVA-PEG-DiVA, wherein the DiVA-PEG-DiVA that is co-solubilized.

Also within the scope of the present disclosure are methods of delivering a therapeutic agent to a patient. These methods comprise providing a pharmaceutical formulation including any of the foregoing compositions and a pharmaceutically acceptable carrier or diluent; and administering the pharmaceutical formulation to the patient.

### DEFINITIONS

As used herein, "alkyl" refers to a straight or branched fully saturated (no double or triple bonds) hydrocarbon group, for example, a group having the general formula-CₙH₂ₙ₊₁. The alkyl group may have 1 to 50 carbon atoms (whenever it appears herein, a numerical range such as "1 to 50" refers to each integer in the given range; *e.g*., "1 to 50 carbon atoms" means that the alkyl group may consist of 1 carbon atom, 2 carbon atoms, 3 carbon atoms, *etc.,* up to and including 50 carbon atoms, although the present definition also covers the occurrence of the term "alkyl" where no numerical range is designated). The alkyl group may also be a medium size alkyl having 1 to 30 carbon atoms. The alkyl group could also be a lower alkyl having 1 to 5 carbon atoms. The alkyl group of the compounds may be designated as "C₁-C₄ alkyl" or similar designations. By way of example only, "C₁-C₄ alkyl" indicates that there are one to four carbon atoms in the alkyl chain, i.e., the alkyl chain is selected from the group consisting of methyl, ethyl, propyl, iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl, hexyl and the like.

As used herein, "alkenyl" refers to an alkyl group that contains in the straight or branched hydrocarbon chain one or more double bonds. An alkenyl group may be unsubstituted or substituted. When substituted, the substituent(s) may be selected from the same groups disclosed above with regard to alkyl group substitution unless otherwise indicated.

As used herein, "alkynyl" refers to an alkyl group that contains in the straight or branched hydrocarbon chain one or more triple bonds. An alkynyl group may be unsubstituted or substituted. When substituted, the substituent(s) may be selected from the same groups disclosed above with regard to alkyl group substitution unless otherwise indicated.

As used herein, "halogen" refers to F, Cl, Br, and I.

As used herein, "mesylate" refers to -OSO₂CH₃.

As used herein, the term "pharmaceutical formulation" refers to a mixture of a composition disclosed herein with one or more other chemical components, such as diluents or additional pharmaceutical carriers. The pharmaceutical formulation facilitates administration of the composition to an organism. Multiple techniques of administering a pharmaceutical formulation exist in the art including, but not limited to injection and parenteral administration.

As used herein, the term "pharmaceutical carrier" refers to a chemical compound that facilitates the incorporation of a compound into cells or tissues. For example dimethyl sulfoxide (DMSO) is a commonly utilized carrier as it facilitates the uptake of many organic compounds into the cells or tissues of an organism

As used herein, the term "diluent" refers to chemical compounds diluted in water that will dissolve the formulation of interest (e.g., the formulation that can include a compound, a retinoid, a second lipid, a stabilizing agent, and/or a therapeutic agent) as well as stabilize the biologically active form of the formulation. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of the formulation. As used herein, an "excipient" refers to an inert substance that is added to a formulation to provide, without limitation, bulk, consistency, stability, binding ability, lubrication, disintegrating ability, etc., to the composition. A "diluent" is a type of excipient.

As used herein, "therapeutic agent" refers to a compound that, upon administration to a mammal in a therapeutically effective amount, provides a therapeutic benefit to the mammal: A therapeutic agent may be referred to herein as a drug. Those skilled in the art will appreciate that the term "therapeutic agent" is not limited to drugs that have received regulatory approval. A "therapeutic agent" can be operatively associated with a compound as described herein, a retinoid, and/or a second lipid. For example, a second lipid as described herein can form a liposome, and the therapeutic agent can be operatively associated with the liposome, e.g., as described herein.

As used herein, "lipoplex formulations" refer to those formulations wherein the siRNA is outside of the liposome. In preferred lipoplex formulations, the siRNA is complexed to the outside of the liposome. Other preferred lipoplex formulations include those wherein the siRNA is accessible to any medium present outside of the liposome.

As used herein, "liposome formulations" refer to those formulations wherein the siRNA is encapsulated within the liposome. In preferred liposome formulations, the siRNA is inaccessible to any medium present outside of the liposome.

As used herein, the term "co-solubized" refers to the addition of a component to the cationic lipid mixture before the empty vesicle is formed.

As used herein, the term "decorated" refers to the addition of a component after vesicle formation.

As used herein, "DC-6-14" refers to the following cationic lipid compound:

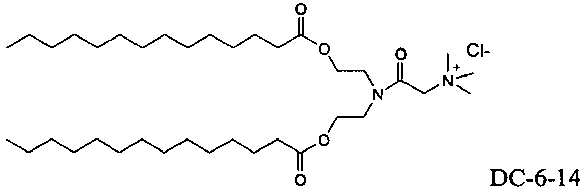

As used herein, "DODC" refers to the following cationic lipid compound:

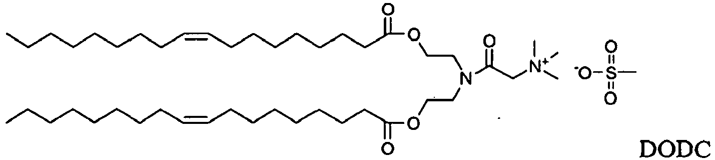

As used herein, "HEDODC" refers to the following cationic lipid compound:

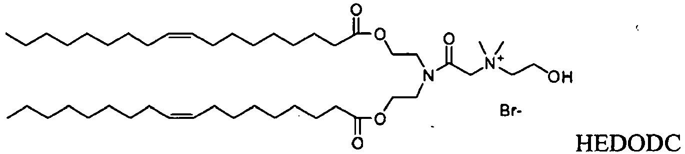

As used herein, a "retinoid" is a member of the class of compounds consisting of four isoprenoid units joined in a head-to-tail manner, see G. P. Moss, "biochemical Nomenclature and Related Documents," 2nd Ed. Portland Press, pp. 247-251 (1992). "Vitamin A" is the generic descriptor for retinoids exhibiting qualitatively the biological activity of retinol. As used herein, retinoid refers to natural and synthetic retinoids including first generation, second generation, and third generation retinoids. Examples of naturally occurring retinoids include, but are not limited to, (1) 11-*cis*-retinal, (2) all*-trans* retinol, (3) retinyl palmitate, (4) all-*trans* retinoic acid, and (5) 13-*cis*-retinoic acids. Furthermore, the term "retinoid" encompasses retinols, retinals, retinoic acids, rexinoids, demethylated and/or saturated retinoic acids, and derivatives thereof.

As used herein, "retinoid conjugate" refers to a molecule that includes at least one retinoid moiety.

As used herein, "retinoid-linker-lipid molecule" refers to a molecule that includes at least one retinoid moiety attached to at least one lipid moiety through at least one linker such as, for example, a PEG moiety.

As used herein, "retinoid linker-retinoid molecule" refers to a molecule that includes at least one retinoid moiety attached to at least one other retinoid moiety (which may be the same or different) through at least one linker such as, for example, a PEG moiety.

As used herein, "Vitamin D" is a generic descriptor for a group of vitamins having antirachitic activity. The vitamin D group includes: vitamin D₂ (calciferol), vitamin D₃ (irradiated 22-dihydroergosterol), vitamin D₄ (irradiated dehydrositosterol) and vitamin D₅ (irradiated dehydrositosterol).

As used herein, "Vitamin E" is a generic descriptor for a group of molecules with antioxidant activity. The vitamin E family includes α-tocopherol, β-tocopherol, γ-tocopherol and δ-tocopherol, with α-tocopherol being the most prevalent. (Brigelius-Flohe and Traber, The FASEB Journal. 1999;13:1145-1155).

As used herein, "Vitamin K" is generic descriptor for an antihemorrahgic factor and includes vitamin K₁ (phytonodione), vitamin K₂ (menaquinone), vitamin K₃, vitamin K₄ and vitamin K₅. Vitamins K₁ and K₂ are natural, while K3-5 are synthetic.

As used herein, "retinoid-linker-lipid molecule" refers to a molecule that includes at least one retinoid moiety attached to at least one lipid moiety through at least one linker such as, for example, a PEG moiety.

As used herein, "retinoid linker-retinoid molecule" refers to a molecule that includes at least one retinoid moiety attached to at least one other retinoid moiety (which may be the same or different) through at least one linker such as, for example, a PEG moiety.

As used herein, the terms "lipid" and "lipophilic" are used herein in their ordinary meanings as understood by those skilled in the art. Non-limiting examples of lipids and lipophilic groups include fatty acids, sterols, C₂-C₅₀ alkyl, C₂-C₅₀ heteroalkyl, C₂-C₅₀ alkenyl, C₂-C₅₀ heteroalkenyl, C₅-C₅₀ aryl, C₅-C₅₀ heteroaryl, C₂-C₅₀ alkynyl, C₂-C₅₀ heteroalkynyl, C₂-C₅₀ carboxyalkenyl, and C₂-C₅₀ carboxyheteroalkenyl. A fatty acid is a saturated or unsaturated long-chain monocarboxylic acid that contains, for example, 12 to 24 carbon atoms A lipid is characterized as being essentially water insoluble, having a solubility in water of less than about 0.01% (weight basis). As used herein, the terms "lipid moiety" and "lipophilic moiety" refers to a lipid or portion thereof that has become attached to another group. For example, a lipid group may become attached to another compound (e.g., a monomer) by a chemical reaction between a functional group (such as a carboxylic acid group) of the lipid and an appropriate functional group of a monomer.

As used herein, "siRNA" refers to small interfering RNA, also known in the art as short interfering RNA or silencing RNA. siRNA is a class of double stranded RNA molecules that have a variety of effects known in the art, the most notable being the interference with the expression of specific genes and protein expression.

As used herein, "encapsulated by the liposome" refers to a component being substantially or entirely within the liposome structure.

As used herein, "accessible to the aqueous medium" refers to a component being able to be in contact with the aqueous medium.

As used herein, "inaccessible to the aqueous medium" refers to a component not being able to be in contact with the aqueous medium.

As used herein, "complexed on the outer surface of the liposome" refers to refers to a component being operatively associated with the outer surface of the liposome.

As used herein, "localized on the outer surface of the liposome" refers to a component being at or near the outer surface of the liposome.

As used herein, "charge complexed" refers to an electrostatic association.

As used herein, the term "operatively associated" refers to an electronic interaction between a compound as described herein, a therapeutic agent, a retinoid, and/or a second lipid. Such interaction may take the form of a chemical bond, including, but not limited to, a covalent bond, a polar covalent bond, an ionic bond, an electrostatic association, a coordinate covalent bond, an aromatic bond, a hydrogen bond, a dipole, or a van der Waals interaction. Those of ordinary skill in the art understand that the relative strengths of such interactions may vary widely.

The term "liposome" is used herein in its ordinary meaning as understood by those skilled in the art, and refers to a lipid bilayer structure that contains lipids attached to polar, hydrophilic groups which form a substantially closed structure in aqueous media. In some embodiments, the liposome can be operatively associated with one or more compounds, such as a therapeutic agent and a retinoid or retinoid conjugate. A liposome may be comprised of a single lipid bilayer (i.e., unilamellar) or it may comprised of two or more concentric lipid bilayers (i.e., multilamellar). Additionally, a liposome can be approximately spherical or ellipsoidal in shape.

The term "facilitating drug delivery to a target cell" refers the enhanced ability of the present retinoid or fat soluble vitamin compounds to enhance delivery of a therapeutic molecule such as siRNA to a cell. While not intending to be bound by theory, the retinoid or fat-soluble vitamin compound interacts with a specific receptor or [activation/binding site] on a target cell with specificity that can be measured. For example, binding is generally consider specific when binding affinity (Kₐ) of 10⁶ M⁻¹ or greater, preferably 10⁷ M⁻¹ or greater, more preferably 10⁸ M⁻¹ or greater, and most preferably 10⁹ M⁻¹ or greater. The binding affinity of an antibody can be readily determined by one of ordinary skill in the art, for example, by Scatchard analysis (Scatchard, Ann. NY Acad. Sci. 51:660, 1949)

Nucleic acid delivery systems may include, for example, aqueous and nonaqueous gels, creams, multiple emulsions, microemulsions, liposomes, ointments, aqueous and nonaqueous solutions, lotions, aerosols, hydrocarbon bases and powders, and can contain excipients such as solubilizers, permeation enhancers (*e.g.*, fatty acids, fatty acid esters, fatty alcohols and amino acids), and hydrophilic polymers (*e.g.,* polycarbophil and polyvinylpyrolidone).

In addition to the cationic lipid of Formula I, other lipids may be useful. These include ionizable cationic lipids, including

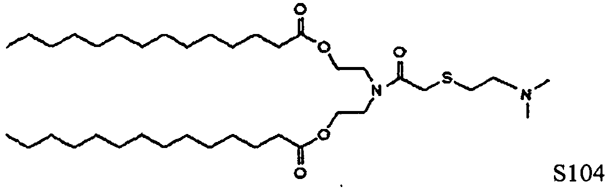

Delivery formulations may consist of a cationic lipid of Formula I in combination with an ionizable cationic lipid. An ionizable cationic lipid may include, e.g., S104. The ionizable cationic lipid may be present at a concentration of 0 to 45 mol %, including a concentration selected from 5, 10, 15, 20, 25, 30, 35, 40, and 45 mol %.

A lipid conjugated to a polyethylene glycol molecule (PEG), may be present in the liposome particles. PEG-lipids include
- 1,2-dimyristoleoyl-sn-glycero-3-phosphoethanolamine-N-PEG (PEG-DMPE)
- 1,2-dipalmitoyl-sn-glycero-3-phosphoethanolamine-N-PEG (PEG-DPPE),
- 1,2-distearoyl-sn-glycero-3-phosphoethanolamine-N-PEG (PEG-DSPE), or
- 1,2-dioleoyl-sn-glycero-3-phosphoethanolamine-N-PEG (PEG-DOPE) and/or
- PEG-ceramide.

Delivery formulations may consist of a cationic lipid of Formula I in combination with a PEG-lipid. The PEG-lipid may be present at a concentration of 0 to 15 mol %, preferably 1 to 10 mol %, including a concentration selected from 1, 2, 3, 4, 5, 6, 7, 8, 9, and 10 mole %.

Non-limiting examples of non-cationic lipids include phospholipids such as lecithin, phosphatidylethanolamine, lysolecithin, lysophosphatidylethanolamine, phosphatidylserine, phosphatidylinositol, sphingomyelin, egg sphingomyelin (ESM), cephalin, cardiolipin, phosphatidic acid, cerebrosides, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoyl-phosphatidylcholine (POPC), palmitoyloleoyl-phosphatidylethanolamine (POPE), palmitoyloleyol-phosphatidylglycerol (POPG), dioleoylphosphatidylethanolamine 4-(N-maleimidomethyl)-cyclohexane-1-carboxylate (DOPE-mal), dipalmitoyl-phosphatidylethanolamine (DPPE), dimyristoylphosphatidylethanolamine (DMPE), distearoyl-phosphatidylethanolamine (DSPE), monomethylphosphatidylethanolamine, dimethyl-phosphatidylethanolamine, dielaidoylphosphatidylethanolamine (DEPE), stearoyloleoyl-phosphatidylethanolamine (SOPE), lysophosphatidylcholine, dilinoleoylphosphatidylcholine, and mixtures thereof. Other diacylphosphatidylcholine and diacylphosphatidylethanolamine phospholipids can also be used. The acyl groups in these lipids are preferably acyl groups derived from fatty acids having C₁₀-C₂₄ carbon chains, e.g., lauroyl, myristoyl, palmitoyl, stearoyl, or oleoyl.

In certain embodiments, the amount of phospholipid present in particles comprises from about 0 mol % to about 55 mol %, more specifically at a concentration selected from the 5, 10, 15, 20, 25, 30, 35, 40, 45, 50, and 55%. As a non-limiting example, the phospholipid is DOPE.

Additional examples of non-cationic lipids include sterols such as cholesterol and derivatives thereof such as cholestanol, cholestanone, cholestenone, coprostanol, cholesteryl-2'-hydroxyethyl ether, cholesteryl-4'-hydroxybutyl ether, and mixtures thereof.

In certain embodiments, the cholesterol or cholesterol derivative present in particles comprises from about 0 mol % to about 55 mol %, more specifically at a concentration selected from the 5, 10, 15, 20, 25, 30, 35, 40, 45, 50, and 55 mol %. As a non-limiting example, cholesterol is present in the lipid particles.

In certain other embodiments, the cholesterol present in lipid particles containing a mixture of phospholipid and cholesterol comprises from about 30 mol % to about 40 mol %, from about 30 mol % to about 35 mol %, or from about 35 mol % to about 40 mol % of the total lipid present in the particle. As a non-limiting example, a lipid particle comprising a mixture of phospholipid and cholesterol may comprise cholesterol at about 34 mol % of the total lipid present in the particle.

In further embodiments, the cholesterol present in lipid particles containing a mixture of phospholipid and cholesterol comprises from about 10 mol % to about 30 mol %, from about 15 mol % to about 25 mol %, or from about 17 mol % to about 23 mol % of the total lipid present in the particle. As a non-limiting example, a lipid particle comprising a mixture of phospholipid and cholesterol may comprise cholesterol at about 20 mol % of the total lipid present in the particle.

The retinoid or retinoid conjugate useful for delivery of nucleic acid is in a state in which it is dissolved in or mixed with a medium that can dissolve or retain it.

Any retinoid or retinoid conjugate may be used in the present description as long as it is actively accumulated by stellate cells; examples of retinoid include, but are not limited to, tretinoin, adapalene, retinol palmitate, and in particular vitamin A, saturated vitamin A, retinoic acid, and retinal. Examples of the retinoid-conjugate include PEG-retinoid conjugates. The present description utilizes the property of stellate cells to positively incorporate a retinoid and/or a retinoid conjugate, and by using the retinoid and/or retinoid conjugate as a drug carrier or by bonding to or being included in another drug carrier component, a desired material or body is transported specifically to stellate cells. A retinoid is a member of the class of compounds having a skeleton in which four isoprenoid units are bonded in a head-to-tail manner. See G. P. Moss, "Biochemical Nomenclature and Related Documents," 2nd Ed. Portland Press, pp. 247-251 (1992). Vitamin A is a generic descriptor for a retinoid qualitatively showing the biological activity of retinol. The retinoid in the present description promotes specific substance delivery to a cancer cell and a CAF (that is, the substance is targeted at these cells). Such a retinoid is not particularly limited, and examples thereof include retinol, Vitamin A, saturated Vitamin A, retinal, retinoic acid, an ester of retinol and a fatty acid, an ester of an aliphatic alcohol and retinoic acid, etretinate, tretinoin, isotretinoin, adapalene, acitretine, tazarotene, and retinol palmitate, and vitamin A analogues such as fenretinide, and bexarotene. Retinoid-conjugates include PEG-conjugates, e.g., diVA-PEG-diVA and VA-PEG-VA.

The drug carrier of the present description therefore may contain a drug carrier component other than a retinoid and/or retinoid-conjugate. Such a component is not particularly limited, and any component known in the fields of medicine and pharmacy may be used, but it is preferable for it to be capable of including a retinoid and/or retinoid conjugate. Furthermore, the drug carrier of the present description may contain a substance that improves incorporation into stellate cells, for example, retinol-binding protein (RBP). The bonding or inclusion of the retinoid and/or retinoid conjugate with the drug carrier of the present description may also be carried out by bonding or including the retinoid and/or retinoid conjugate with another component of the drug carrier by chemical and/or physical methods. Alternatively, bonding or inclusion of the retinoid and/or retinoid conjugate with the drug carrier of the present description may also be carried out by mixing the retinoid and/or retinoid conjugate having formationaffinity and basic components of the drug carrier, into the drug carrier components during preparation of the drug carrier.

The amount of retinoid and/or retinoid conjugate bonded to or included in the drug carrier of the present description may be 0.1 mol % to 20 mol% as a ratio by weight relative to the drug carrier components, preferably 0.2% to 10%, and more preferably 0.5% to 5 mol %, including a concentration selected from the values 0.25, 0.5, 1.0, 1.5, 2.0. 2.5, 3.0, 3.5, 4.0, 4.5, and 5.0 mol %.

In certain embodiments, the present invention provides for a liposome to be produced via mixing in a chamber. This includes a process that provides an aqueous solution comprising a nucleic acid such as an interfering RNA in a first reservoir, providing an organic lipid solution in a second reservoir, and mixing the aqueous solution with the organic lipid solution such that the organic lipid solution mixes with the aqueous solution so as to produce a liposome encapsulating the nucleic acid (e.g., siRNA) in a gradient of organic solvent concentration.

The liposome formed using the mixing method typically have a size of from about 40 nm to about 250 nm, from about 50 nm to about 150 nm, from about 60 nm to about 150 nm. The particles thus formed do not aggregate and are optionally sized to achieve a uniform particle size.

The drug carrier of the present description may be in any form as long as a desired material or body can be transported to target stellate cells, and examples of the form include, but are not limited to, polymer micelle, liposome, emulsion, microsphere, and nanosphere. Furthermore, the drug carrier of the present description may include in its interior the substance that is to be transported, be attached to the exterior of the substance that is to be transported, or be mixed with the substance that is to be transported as long as the retinoid and/or retinoid conjugate included therein is at least partially exposed on the exterior of the preparation before it reaches the stellate cells at the latest.

The drug carrier of the present description specifically targets stellate cells and enables a desired effect such as, for example, inhibition or prevention of fibrosis to be exhibited with the maximum effect and minimum side effects by efficiently transporting to stellate cells a desired material or body such as, for example, a drug for controlling the activity or growth of stellate cells. The material or body that the present drug carrier delivers is not particularly limited, but it preferably has a size that enables physical movement in a living body from an administration site to the liver, pancreas, etc., where stellate cells are present. The drug carrier of the present description therefore can transport not only a material such as an atom, a molecule, a compound, a protein, or a nucleic acid but also a body such as a vector, a virus particle, a cell, a drug release system constituted from one or more elements, or a micromachine. The material or body preferably has the property of exerting some effect on stellate cells, and examples thereof include one that labels stellate cells and one that controls the activity or growth of stellate cells.

Therefore, in one embodiment of the present description, it is a drug for controlling the activity or growth of stellate cells that the drug carrier delivers. This may be any drug that directly or indirectly inhibits the physicochemical actions of stellate cells involved in the promotion of fibrosis, and examples thereof include, but are not limited to, TGFβ activity inhibitors such as a truncated TGFβ type II receptor and a soluble TGFβ type II receptor, growth factor preparations such as HGF and expression vectors therefor, MMP production promoters such as an MMP gene-containing adenovirus vector, TIMP production inhibitors such as an antisense TIMP nucleic acid, a PPARγ ligand, cell activation inhibitors and/or cell growth inhibitors such as an angiotensin activity inhibitor, a PDGF activity inhibitor, and a sodium channel inhibitor, and also apoptosis inducers such as compound 861 and gliotoxin, adiponectin, and a compound having Rho kinase inhibitory activity such as (+)-trans-4-(1-aminoethyl)-1-(4-pyridylcarbamoyl)cyclohexane. Furthermore, the drug for controlling the activity or growth of stellate cells' in the present description may be any drug that directly or indirectly promotes the physicochemical actions of stellate cells directly or indirectly involved in the inhibition of fibrosis, and examples thereof include, but are not limited to, a drug for promoting a collagen degradation system, e.g., MMP production promoters such as an MMP expression vector, HGF, and drugs having HGF-like activity such as HGF analogues and expression vectors therefor.

Other examples of the drug for controlling the activity or growth of stellate cells in the present description include a drug for controlling the metabolism of an extracellular matrix such as collagen, for example, a substance having an effect in inhibiting the expression of a target molecule, such as siRNA, ribozyme, and antisense nucleic acid (including RNA, DNA, PNA, and a composite thereof), a substance having a dominant negative effect, and vectors expressing same, that target, for example, an extracellular matrix constituent molecule produced by stellate cells or target one or more molecules that have the function of producing or secreting the extracellular matrix constituent molecule.

The present description also relates to a medicine for treating a stellate cell-related disorder, the medicine containing the drug carrier and the drug for controlling the activity or growth of stellate cells, and relates to the use of the drug carrier in the production of a pharmaceutical composition for treating a stellate cell-related disorder. The stellate cell-related disorder referred to here means a disorder in which stellate cells are directly or indirectly involved in the process of the disorder, that is, the onset, exacerbation, improvement, remission, cure, etc. of the disorder, and examples thereof include hepatic disorders such as hepatitis, in particular chronic hepatitis, hepatic fibrosis, hepatic cirrhosis, and liver cancer, and pancreatic disorders such as pancreatitis, in particular chronic pancreatitis, pancreatic fibrosis, and pancreatic cancer.

In the medicine of the present description, the drug carrier may include a drug in its interior, be attached to the exterior of a drug-containing substance, or be mixed with a drug as long as the retinoid and/or retinoid-conjugate included in the drug carrier is at least partially exposed on the exterior of the preparation before it reaches the stellate cells at the latest. Therefore, depending on the route of administration or manner in which the drug is released, the medicine may be covered with an appropriate material, such as, for example, an enteric coating or a material that disintegrates over time, or may be incorporated into an appropriate drug release system.

The present description therefore includes a drug carrier or medicine preparation kit containing one or more containers containing one or more of a drug carrier constituent, a retinoid and/or a retinoid conjugate, and/or a drug, and also includes an essential component for the drug carrier or the medicine provided in the form of such a kit. The kit of the present description may contain, in addition to those described above, a description, etc. in which a preparation method or an administration method for the drug carrier and the medicine of the present description is described. Furthermore, the kit of the present description may contain all components for completing the drug carrier or the medicine of the present description but need not necessarily contain all of the components. The kit of the present description therefore need not contain a reagent or a solvent that is normally available at a place of medical treatment, an experimental facility, etc. such as, for example, sterile water, saline, or a glucose solution.

The present description further relates to a method for treating a stellate cell-related disorder, the method including administering an effective amount of the medicine to a subject in need thereof. The effective amount referred to here is an amount that suppresses onset of the target disorder, reduces symptoms thereof, or prevents progression thereof, and is preferably an amount that prevents onset of the target disorder or cures the target disorder. It is also preferably an amount that does not cause an adverse effect that exceeds the benefit from administration. Such an amount may be determined as appropriate by an *in vitro* test using cultured cells, etc. or by a test in a model animal such as a mouse, a rat, a dog, or a pig, and such test methods are well known to a person skilled in the art.

In the method of the present description, the term 'subject' means any living individual, preferably an animal, more preferably a mammal, and yet more preferably a human individual. In the present description, the subject may be healthy or affected with some disorder, and in the case of treatment of a disorder being intended, the subject typically means a subject affected with the disorder or having a risk of being affected.

Furthermore, the term 'treatment' includes all types of medically acceptable prophylactic and/or therapeutic intervention for the purpose of the cure, temporary remission, prevention, etc. of a disorder. For example, when the disorder is hepatic fibrosis, the term 'treatment' includes medically acceptable intervention for various purposes including delaying or halting the progression of fibrosis, regression or disappearance of lesions, prevention of the onset of fibrosis, or prevention of recurrence.

The present description also relates to a method for delivering a drug to stellate cells using the drug carrier. This method includes, but is not limited to, a step of supporting a substance to be delivered on the drug carrier, and a step of administering or adding the drug carrier carrying the substance to be delivered to a stellate cell-containing living body or medium, such as, for example, a culture medium. These steps may be achieved as appropriate in accordance with any known method, the method described in the present specification, etc. This delivery method may be combined with another delivery method, for example, another delivery method in which an organ where stellate cells are present is the target, etc.

Nucleic acid molecules may be adapted for use to prevent or treat fibroses (e.g., liver, kidney, peritoneal, and pulmonary) diseases, traits, conditions and/or disorders, and/or any other trait, disease, disorder or condition that is related to or will respond to the levels of hsp47 in a cell or tissue, alone or in combination with other therapies. A nucleic acid molecule may include a delivery vehicle, including liposomes, for administration to a subject, carriers and diluents and their salts, and/or can be present in pharmaceutically acceptable formulations.

The nucleic acid molecules of the description may include sequences shown in Table 3. Examples of such nucleic acid molecules consist essentially of sequences provided in Table 3.

The nucleic acid molecules may be administered via pulmonary delivery, such as by inhalation of an aerosol or spray dried formulation administered by an inhalation device or nebulizer, providing rapid local uptake of the nucleic acid molecules into relevant pulmonary tissues. Solid particulate compositions containing respirable dry particles of micronized nucleic acid compositions can be prepared by grinding dried or lyophilized nucleic acid compositions, and then passing the micronized composition through, for example, a 400 mesh screen to break up or separate out large agglomerates. A solid particulate composition comprising the nucleic acid compositions of the description can optionally contain a dispersant which serves to facilitate the formation of an aerosol as well as other therapeutic compounds. A suitable dispersant is lactose, which can be blended with the nucleic acid compound in any suitable ratio, such as a 1 to 1 ratio by weight.

Aerosols of liquid particles may include a nucleic acid molecules disclosed herein and can be produced by any suitable means, such as with a nebulizer (see *e.g.,* U.S. Pat. No. 4,501,729). Nebulizers are commercially available devices which transform solutions or suspensions of an active ingredient into a therapeutic aerosol mist either by means of acceleration of a compressed gas, typically air or oxygen, through a narrow venturi orifice or by means of ultrasonic agitation. Suitable formulations for use in nebulizers include the active ingredient in a liquid carrier in an amount of up to 40% w/w preferably less than 20% w/w of the formulation. The carrier is typically water or a dilute aqueous alcoholic solution, preferably made isotonic with body fluids by the addition of, *e.g*., sodium chloride or other suitable salts. Optional additives include preservatives if the formulation is not prepared sterile, *e.g*., methyl hydroxybenzoate, anti-oxidants, flavorings, volatile oils, buffering agents and emulsifiers and other formulation surfactants. The aerosols of solid particles including the active composition and surfactant can likewise be produced with any solid particulate aerosol generator. Aerosol generators for administering solid particulate therapeutics to a subject produce particles which are respirable, as explained above, and generate a volume of aerosol containing a predetermined metered dose of a therapeutic composition at a rate suitable for human administration. One illustrative type of solid particulate aerosol generator is an insufflator. Suitable formulations for administration by insufflation include finely comminuted powders which can be delivered by means of an insufflator. In the insufflator, the powder, *e.g*., a metered dose thereof effective to carry out the treatments described herein, is contained in capsules or cartridges, typically made of gelatin or plastic, which are either pierced or opened in situ and the powder delivered by air drawn through the device upon inhalation or by means of a manually-operated pump. The powder employed in the insufflator consists either solely of the active ingredient or of a powder blend comprising the active ingredient, a suitable powder diluent, such as lactose, and an optional surfactant. The active ingredient typically includes from 0.1 to 100 w/w of the formulation. A second type of illustrative aerosol generator includes a metered dose inhaler. Metered dose inhalers are pressurized aerosol dispensers, typically containing a suspension or solution formulation of the active ingredient in a liquefied propellant. During use these devices discharge the formulation through a valve adapted to deliver a metered volume to produce a fine particle spray containing the active ingredient. Suitable propellants include certain chlorofluorocarbon compounds, *e.g.*, dichlorodifluoromethane, trichlorofluoromethane, dichlorotetrafluoroethane and mixtures thereof. The formulation can additionally contain one or more co-solvents, for example, ethanol, emulsifiers and other formulation surfactants, such as oleic acid or sorbitan trioleate, anti-oxidants and suitable flavoring agents. Other methods for pulmonary delivery are described in, *e.g*., US20040037780, US6592904, US6582728, and US6565885. WO08132723 relates to aerosol delivery of oligonucleotides in general, and of siRNA in particular, to the respiratory system.

Nucleic acid molecules may be administered to the central nervous system (CNS) or peripheral nervous system (PNS). Experiments have demonstrated the efficient *in vivo* uptake of nucleic acids by neurons. See *e.g.,* Sommer et al., 1998, Antisense Nuc. Acid Drug Dev., 8:75; Epa et al., 2000, Antisense Nuc. Acid Drug Dev., 10:469; Broaddus et al., 1998, J. Neurosurg., 88:734; Karle et al., 1997, Eur. J. Pharmocol., 340:153; Bannai et al., 1998, Brain Research, 784:304; Rajakumar et al., 1997, Synapse, 26:199; Wu-pong et al., 1999, BioPharm, 12:32; Bannai et al., 1998, Brain Res. Protoc., 3:83; and Simantov et al., 1996, Neuroscience, 74:39. Nucleic acid molecules are therefore amenable to delivery to and uptake by cells in the CNS and/or PNS.

Delivery of nucleic acid molecules to the CNS is provided by a variety of different strategies. Traditional approaches to CNS delivery that can be used include, but are not limited to, intrathecal and intracerebroventricular administration, implantation of catheters and pumps, direct injection or perfusion at the site of injury or lesion, injection into the brain arterial system, or by chemical or osmotic opening of the blood-brain barrier. Other approaches can include the use of various transport and carrier systems, for example though the use of conjugates and biodegradable polymers. Furthermore, gene therapy approaches, *e.g*., as described in Kaplitt et al., U.S. Pat. No. 6,180,613 and Davidson, WO 04/013280, can be used to express nucleic acid molecules in the CNS.

Nucleic acid molecules may be formulated or complexed with polyethylenimine (*e*.*g*., linear or branched PEI) and/or polyethylenimine derivatives, including for example grafted PEIs such as galactose PEI, cholesterol PEI, antibody derivatized PEI, and polyethylene glycol PEI (PEG-PEI) derivatives thereof (see for example Ogris et al., 2001, AAPA PharmSci, 3, 1-11; Furgeson et al., 2003, Bioconjugate Chem., 14, 840-847; Kunath et al., 2002, Pharm Res 19:810-17; Choi et al., 2001, Bull. Korean Chem. Soc., 22:46-52; Bettinger et al., 1999, Bioconjugate Chem., 10:558-561; Peterson et al., 2002, Bioconjugate Chem. 13:845-54; Erbacher et al., 1999, J Gene Med 1:1-18; Godbey et al., 1999., PNAS, 96:5177-81; Godbey et al., 1999, J Controlled Release, 60:149-60; Diebold et al., 1999, J Biol Chem, 274:19087-94; Thomas et al., 2002, PNAS, 99, 14640-45; and Sagara, U.S. Pat. No. 6,586,524).

Nucleic acid molecules may include a bioconjugate, for example a nucleic acid conjugate as described in Vargeese et al., U.S. Ser. No. 10/427,160; U.S. Pat. No. 6,528,631; U.S. Pat. No. 6,335,434; U.S. Pat. No. 6,235,886; U.S. Pat. No. 6,153,737; U.S. Pat. No. 5,214,136; U.S. Pat. No. 5,138,045.

Compositions, methods and kits disclosed herein may include an expression vector that includes a nucleic acid sequence encoding at least one nucleic acid molecule of the description in a manner that allows expression of the nucleic acid molecule. Methods of introducing nucleic acid molecules or one or more vectors capable of expressing the strands of dsRNA into the environment of the cell will depend on the type of cell and the make-up of its environment. The nucleic acid molecule or the vector construct may be directly introduced into the cell (i.e., intracellularly); or introduced extracellularly into a cavity, interstitial space, into the circulation of an organism, introduced orally, or may be introduced by bathing an organism or a cell in a solution containing dsRNA. The cell is preferably a mammalian cell; more preferably a human cell. The nucleic acid molecule of the expression vector can include a sense region and an antisense region. The antisense region can include a sequence complementary to an RNA or DNA sequence encoding hsp47 and the sense region can include a sequence complementary to the antisense region. The nucleic acid molecule can include two distinct strands having complementary sense and antisense regions. The nucleic acid molecule can include a single strand having complementary sense and antisense regions.

Nucleic acid molecules that interact with target RNA molecules and down-regulate gene encoding target RNA molecules (*e.g*., target RNA molecules referred to by Genbank Accession numbers herein) may be expressed from transcription units inserted into DNA or RNA vectors. Recombinant vectors can be DNA plasmids or viral vectors. Nucleic acid molecule expressing viral vectors can be constructed based on, but not limited to, adeno-associated virus, retrovirus, adenovirus, or alphavirus. The recombinant vectors capable of expressing the nucleic acid molecules can be delivered as described herein, and persist in target cells. Alternatively, viral vectors can be used that provide for transient expression of nucleic acid molecules. Such vectors can be repeatedly administered as necessary. Once expressed, the nucleic acid molecules bind and down-regulate gene function or expression via RNA interference (RNAi). Delivery of nucleic acid molecule expressing vectors can be systemic, such as by intravenous or intramuscular administration, by administration to target cells ex-planted from a subject followed by reintroduction into the subject, or by any other means that would allow for introduction into the desired target cell.

In another aspect, the present disclosure relates to a pharmaceutical formulation comprising one or more physiologically acceptable surface active agents, pharmaceutical carriers, diluents, excipients, and suspension agents, or a combination thereof; and a formulation (e.g., the formulation that can include a compound, a retinoid, a second lipid, a stabilizing agent, and/or a therapeutic agent) disclosed herein. Acceptable additional pharmaceutical carriers or diluents for therapeutic use are well known in the pharmaceutical art, and are described, for example, in Remington's Pharmaceutical Sciences, 18th Ed., Mack Publishing Co., Easton, PA (1990). Preservatives, stabilizers, dyes, and the like may be provided in the pharmaceutical formulation. For example, sodium benzoate, ascorbic acid and esters of p-hydroxybenzoic acid may be added as preservatives. In addition, antioxidants and suspending agents may be used. In various embodiments, alcohols, esters, sulfated aliphatic alcohols, and the like may be used as surface active agents; sucrose, glucose, lactose, starch, crystallized cellulose, mannitol, light anhydrous silicate, magnesium aluminate, magnesium metasilicate aluminate, synthetic aluminum silicate, calcium carbonate, sodium acid carbonate, calcium hydrogen phosphate, calcium carboxymethyl cellulose, and the like may be used as excipients; coconut oil, olive oil, sesame oil, peanut oil, soya may be used as suspension agents or lubricants; cellulose acetate phthalate as a derivative of a carbohydrate such as cellulose or sugar, or methylacetate-methacrylate copolymer as a derivative of polyvinyl may be used as suspension agents; and plasticizers such as ester phthalates and the like may be used as suspension agents.

The pharmaceutical formulations described herein can be administered to a human patient *per se,* or in pharmaceutical formulations where they are mixed with other active ingredients, as in combination therapy, or suitable pharmaceutical carriers or excipient(s). Techniques for formulation and administration of the compounds of the instant application may be found in "Remington's Pharmaceutical Sciences," Mack Publishing Co., Easton, PA, 18th edition, 1990.

Suitable routes of administration may include, for example, parenteral delivery, including intramuscular, subcutaneous, intravenous, intramedullary injections, as well as intrathecal, direct intraventricular, intraperitoneal, intranasal, or intraocular injections. The formulation (e.g., the formulation that can include a compound, a retinoid, a second lipid, a stabilizing agent, and/or a therapeutic agent) can also be administered in sustained or controlled release dosage forms, including depot injections, osmotic pumps, and the like, for prolonged and/or timed, pulsed administration at a predetermined rate. Additionally, the route of administration may be local or systemic.

The pharmaceutical formulations may be manufactured in a manner that is itself known, e.g., by means of conventional mixing, dissolving, granulating, dragee-making, levigating, emulsifying, encapsulating, entrapping or tableting processes.

Pharmaceutical formulations may be formulated in any conventional manner using one or more physiologically acceptable pharmaceutical carriers comprising excipients and auxiliaries which facilitate processing of the active compounds into preparations which can be used pharmaceutically. Proper formulation is dependent upon the route of administration chosen. Any of the well-known techniques, pharmaceutical carriers, and excipients may be used as suitable and as understood in the art; *e.g.,* in Remington's Pharmaceutical Sciences, above.

Injectables can be prepared in conventional forms, either as liquid solutions or suspensions, solid forms suitable for solution or suspension in liquid prior to injection, or as emulsions. Suitable excipients are, for example, water, saline, sucrose, glucose, dextrose, mannitol, lactose, lecithin, albumin, sodium glutamate, cysteine hydrochloride, and the like. In addition, if desired, the injectable pharmaceutical formulations may contain minor amounts of nontoxic auxiliary substances, such as wetting agents, pH buffering agents, and the like. Physiologically compatible buffers include, but are not limited to, Hanks's solution, Ringer's solution, or physiological saline buffer. If desired, absorption enhancing preparations may be utilized.

Pharmaceutical formulations for parenteral administration, *e.g*., by bolus injection or continuous infusion, include aqueous solutions of the active formulation (*e.g.*, the formulation that can include a compound, a retinoid, a second lipid, a stabilizing agent, and/or a therapeutic agent) in water-soluble form. Additionally, suspensions of the active compounds may be prepared as appropriate oily injection suspensions. Aqueous injection suspensions may contain substances which increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions. Formulations for injection may be presented in unit dosage form, *e.g.,* in ampoules or in multi-dose containers, with an added preservative. The formulations may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g.,* sterile pyrogen-free water, before use.

In addition to the preparations described previously, the formulations may also be formulated as a depot preparation. Such long acting formulations may be administered by intramuscular injection. Thus, for example, the formulations (e.g., the formulation that can include a compound, a retinoid, a second lipid, a stabilizing agent, and/or a therapeutic agent) may be formulated with suitable polymeric or hydrophobic materials (for example as an emulsion in an acceptable oil) or ion exchange resins, or as sparingly soluble derivatives, for example, as a sparingly soluble salt.

Some embodiments of the disclosure herein are directed to a method of delivering a therapeutic agent to a cell. For example, some embodiments are directed to a method of delivering a therapeutic agent such as siRNA into a cell. Suitable cells for use according to the methods described herein include prokaryotes, yeast, or higher eukaryotic cells, including plant and animal cells (e.g., mammalian cells). In some embodiments, the cells can be human fibrosarcoma cells (e.g., HT1080 cell line). In other embodiments, the cells can be cancer cells. Cell lines which are model systems for cancer may be used, including but not limited to breast cancer (MCF-7, MDA-MB-438 cell lines), U87 glioblastoma cell line, B16F0 cells (melanoma), HeLa cells (cervical cancer), A549 cells (lung cancer), and rat tumor cell lines GH3 and 9L. In these embodiments, the formulations described herein can be used to transfect a cell. These embodiments may include contacting the cell with a formulation described herein that includes a therapeutic agent, to thereby deliver a therapeutic agent to the cell.

Disclosed herein are methods for treating a condition characterized by abnormal fibrosis, which may include administering a therapeutically effective amount of a formulation described herein. Conditions characterized by abnormal fibrosis may include cancer and/or a fibrotic disease. Types of cancer that may be treated or ameliorated by a formulation described herein include, but are not limited to, lung cancer, pancreatic cancer, breast cancer, liver cancer, stomach cancer, and colon cancer. In an embodiment, the cancer that may be treated or ameliorated is pancreatic cancer. In another embodiment, the cancer that may be treated or ameliorated is lung cancer. Types of fibrotic disease that may be treated or ameliorated by a formulation described herein include, but are not limited to, hepatic fibrosis, hepatic cirrhosis, pancreatitis, pancreatic fibrosis, cystic fibrosis, vocal cord scarring, vocal cord mucosal fibrosis, laryngeal fibrosis, pulmonary fibrosis, idiopathic pulmonary fibrosis, cystic fibrosis, myelofibrosis, retroperitoneal fibrosis, and nephrogenic systemic fibrosis. In an embodiment, the condition that may be treated or ameliorated is hepatic fibrosis.

The formulations or pharmaceutical compositions described herein may be administered to the subject by any suitable means. Non-limiting examples of methods of administration include, among others, (a) administration via injection, subcutaneously, intraperitoneally, intravenously, intramuscularly, intradermally, intraorbitally, intracapsularly, intraspinally, intrasternally, or the like, including infusion pump delivery; (b) administration locally such as by injection directly in the renal or cardiac area, e.g., by depot implantation; as well as as deemed appropriate by those of skill in the art for bringing the active compound into contact with living tissue.

Pharmaceutical compositions suitable for administration include formulations (e.g., the formulation that can include a compound, a retinoid, a second lipid, a stabilizing agent, and/or a therapeutic agent) where the active ingredients are contained in an amount effective to achieve its intended purpose. The therapeutically effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors which those skilled in the medical arts will recognize. More specifically, a therapeutically effective amount means an amount of composition effective to prevent, alleviate or ameliorate symptoms of disease or prolong the survival of the subject being treated. Determination of a therapeutically effective amount is well within the capability of those skilled in the art, especially in light of the detailed disclosure provided herein.

As will be readily apparent to one skilled in the art, the useful *in vivo* dosage to be administered and the particular mode of administration will vary depending upon the age, weight and mammalian species treated, the particular compounds employed, and the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Alternatively, acceptable *in vitro* studies can be used to establish useful doses and routes of administration of the compositions identified by the present methods using established pharmacological methods.

In non-human animal studies, applications of potential products are commenced at higher dosage levels, with dosage being decreased until the desired effect is no longer achieved or adverse side effects disappear. The dosage may range broadly, depending upon the desired effects and the therapeutic indication. Typically, dosages may be about 10 microgram/kg to about 100 mg/kg body weight, preferably about 100 microgram/kg to about 10 mg/kg body weight. Alternatively dosages may be based and calculated upon the surface area of the patient, as understood by those of skill in the art.

The exact formulation, route of administration and dosage for the pharmaceutical compositions can be chosen by the individual physician in view of the patient's condition. (See *e.g.,* Fingl et al. 1975, in "The Pharmacological Basis of Therapeutics", with particular reference to Ch. 1, p. 1). Typically, the dose range of the composition administered to the patient can be from about 0.5 to about 1000 mg/kg of the patient's body weight. The dosage may be a single one or a series of two or more given in the course of one or more days, as is needed by the patient. In instances where human dosages for compounds have been established for at least some condition, the dosages will be about the same, or dosages that are about 0.1 % to about 500%, more preferably about 25% to about 250% of the established human dosage. Where no human dosage is established, as will be the case for newly-discovered pharmaceutical compositions, a suitable human dosage can be inferred from ED₅₀ or ID₅₀ values, or other appropriate values derived from *in vitro* or *in vivo* studies, as qualified by toxicity studies and efficacy studies in animals.

It should be noted that the attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Although the exact dosage will be determined on a drug-by-drug basis, in most cases, some generalizations regarding the dosage can be made. The daily dosage regimen for an adult human patient may be, for example, a dose of about 0.1 mg to 2000 mg of each active ingredient, preferably about 1 mg to about 500 mg, e.g. 5 to 200 mg. In other embodiments, an intravenous, subcutaneous, or intramuscular dose of each active ingredient of about 0.01 mg to about 100 mg, preferably about 0.1 mg to about 60 mg, e.g. about 1 to about 40 mg is used. In cases of administration of a pharmaceutically acceptable salt, dosages may be calculated as the free base. In some embodiments, the formulation is administered 1 to 4 times per day. Alternatively the formulations may be administered by continuous intravenous infusion, preferably at a dose of each active ingredient up to about 1000 mg per day. As will be understood by those of skill in the art, in certain situations it may be necessary to administer the formulations disclosed herein in amounts that exceed, or even far exceed, the above-stated, preferred dosage range in order to effectively and aggressively treat particularly aggressive diseases or infections. In some embodiments, the formulations will be administered for a period of continuous therapy, for example for a week or more, or for months or years.

Dosage amount and interval may be adjusted individually to provide plasma levels of the active moiety which are sufficient to maintain the modulating effects, or minimal effective concentration (MEC). The MEC will vary for each compound but can be estimated from in vitro data. Dosages necessary to achieve the MEC will depend on individual characteristics and route of administration. However, HPLC assays or bioassays can be used to determine plasma concentrations.

Dosage intervals can also be determined using MEC value. Compositions should be administered using a regimen which maintains plasma levels above the MEC for 10-90% of the time, preferably between 30-90% and most preferably between 50-90%.

In cases of local administration or selective uptake, the effective local concentration of the drug may not be related to plasma concentration.

The amount of formulation administered may be dependent on the subject being treated, on the subject's weight, the severity of the affliction, the manner of administration and the judgment of the prescribing physician.

Formulations disclosed herein (e.g., the formulation that can include a compound, a retinoid, a second lipid, a stabilizing agent, and/or a therapeutic agent) can be evaluated for efficacy and toxicity using known methods. For example, the toxicology of a particular compound, or of a subset of the compounds, sharing certain chemical moieties, may be established by determining *in vitro* toxicity towards a cell line, such as a mammalian, and preferably human, cell line. The results of such studies are often predictive of toxicity in animals, such as mammals, or more specifically, humans. Alternatively, the toxicity of particular compounds in an animal model, such as mice, rats, rabbits, or monkeys, may be determined using known methods. The efficacy of a particular compound may be established using several recognized methods, such as *in vitro* methods, animal models, or human clinical trials. Recognized *in vitro* models exist for nearly every class of condition, including but not limited to cancer, cardiovascular disease, and various immune dysfunction. Similarly, acceptable animal models may be used to establish efficacy of chemicals to treat such conditions. When selecting a model to determine efficacy, the skilled artisan can be guided by the state of the art to choose an appropriate model, dose, and route of administration, and regime. Of course, human clinical trials can also be used to determine the efficacy of a compound in humans.

The formulations may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions comprising a compound formulated in a compatible pharmaceutical carrier may also be prepared, placed in an appropriate container, and labeled for treatment of an indicated condition.

It is understood that, in any compound described herein having one or more stereocenters, if an absolute stereochemistry is not expressly indicated, then each center may independently be of R-configuration or S-configuration or a mixture thereof. Thus, the compounds provided herein may be enantiomerically pure or be stereoisomeric mixtures. In addition it is understood that, in any compound having one or more double bond(s) generating geometrical isomers that can be defined as E or Z each double bond may independently be E or Z a mixture thereof. Likewise, all tautomeric forms are also intended to be included.

The invention can be further exemplified by reference to the following examples. These examples are illustrative, only, and are not intended to limit the invention.

### EXAMPLES

### Example 1: Preparation of 2-(bis(2-(tetradecanoyloxy)ethyl)amino)-N-(2-hydroxyethyl)-N,N-dimethyl-2-oxoethan-aminium bromide (HEDC)

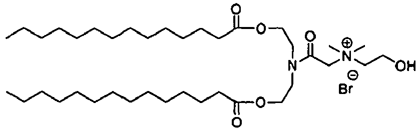

Preparation of Intermediate 1: 2,2'-(tert-butoxycarbonylazanediyl)bis(ethane-2,1-diyl) ditetradecanoate (HEDC-BOC-IN)

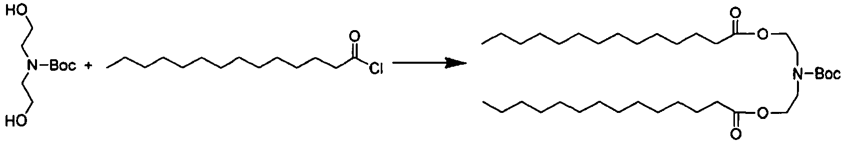

A solution of *N*-BOC-diethanolamine (194 g, 0.946 mol), triethylamine (201 g, 2.03 mol) and diaminopyridine (23.1 g, 0.19 mol) in DCM (1750 mL) was cooled to 0°C. A solution of myristoyl chloride (491 g, 1.99 mol) in DCM (440 mL) was added over a period of 50 minutes at 0-10°C, and the mixture was allowed to warm to ambient temperature. Full conversion was indicated by TLC after 1.5 hours at 20-24°C. Water (1750 mL) was added and pH 8.3 was measured by pH meter. The organic phase was separated, washed with (1) 6% NaHCO₃ (500 mL), (2) 0.3 M HCl (1700 mL), (3) 12.5% sodium chloride (1700 mL), and dried with anhydrous magnesium sulphate (120 g). Evaporation of the filtrate at 50°C and 50 mBar gave 622 g of 2,2'-(tert-butoxycarbonylazanediyl)bis(ethane-2,1-diyl) ditetradecanoate, (HEDC-BOC-IN). This distillation residue, which solidified on standing, was used in the next step.

Preparation of Intermediate 2: 2,2'-(tert-butoxycarbonylazanediyl)bis(ethane-2,1-diyl) ditetradecanoate (HEDC-amine-IN) TFA salt 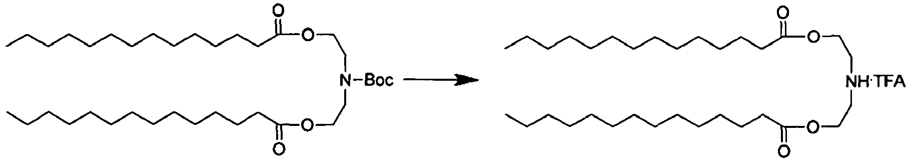 HEDC-BOC-IN (620 g, 0.990 mol) was transformed into a liquid by brief heating into a 50°C-bath and then cooled below 25°C. TFA (940 mL, 1.39 kg, 1.18 mol) was added to the liquid over a period of 30 minutes, using moderate cooling in order to maintain a temperature not higher than 25°C. Having added two thirds of the amount of TFA, significant gas evolution was observed. The reaction mixture was stirred overnight at ambient temperature. TLC indicated traces of HEDC-BOC-IN. The reaction mixture was heated for distillation of TFA under reduced pressure (125-60 mBar) from a water bath of 50-55°C, and distillation was continued until it became very slow. TFA fumes were absorbed in a scrubber with 10% sodium hydroxide. Heptane (2000 mL) was added, stirred, and distilled off under reduced pressure. Heptane (2000 mL) was added to the partly solidified residue, and the mixture was heated to 45°C, at which temperature a slightly turbid solution was formed. The solution was cooled, seeded at 40°C, and precipitate was formed by stirring for a period of 25 minutes at 40-36°C. Having cooled and stirred for a period of 40 minutes at ambient temperature, the precipitate of heavy crystals was isolated by filtration, and the filter cake was washed with heptane (1000 mL). The wet filter cake (914 g) was dried overnight at ambient temperature under reduced pressure (<1 mBar) to give 635 g (100%) of 2,2'-(tert-butoxycarbonylazanediyl)bis(ethane-2,1-diyl) ditetradecanoate (HEDC-amine-IN) TFA salt as white crystals.

Preparation of Intermediate 3: 2,2'-(2-(dimethylamino)acetylazanediyl)bis(ethane-2,1-diyl) ditetradeca-noate (HEDC-DiMeGly-IN) 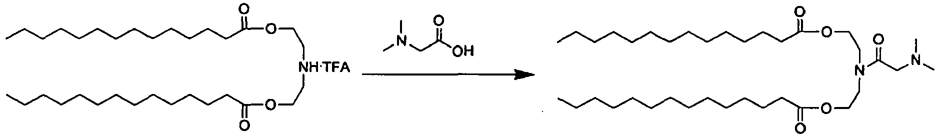 N,N-Dimethylglycine (56.6 g, 548 mmol), HOBt hydrate (83.9 g, 548 mmol) and EDC hydrochloride (105 g, 548 mmol) were added to DMF (3.5 L), and the mixture was stirred at ambient temperature for a period of one hour. A clear solution was formed. HEDC-amine-IN TFA salt (270 g, 442 mmol) was mixed with DCM (1.15 L) and 6% sodium bicarbonate (1.15 L). The separated organic phase with the free amine was added to the coupling mixture in DMF, and a precipitation as well as a temperature increase of approx. 9°C was observed. Triethylamine (47.0 g, 464 mmol) was added, and the reaction mixture was stirred at 25-30°C for a period of five hours. TLC indicated incomplete conversion, and additional EDC hydrochloride (29.5 g, 154 mmol) was added. Having stirred overnight at ambient temperature, a clear solution was observed, and TLC now indicated full conversion. The reaction mixture was mixed with DCM (2.3 L) and 2% sodium bicarbonate (7 L). The organic phase was washed twice with 1.25% sodium chloride (5 L each) and dried with anhydrous magnesium sulphate (186 g). The filtrate was evaporated at 50°C and 30 mBar to give 253 g of crude oil. The crude material was loaded to a column packed with 2.6 kg of Silica Gel 60 (40-63µ). The product was eluted with toluene:ethyl acetate (8:2) (4 L), and followed with ethyl acetate:methanol (1:1) (5 L). The product containing fractions (3.5-8 L) was evaporated (50°C/250-20 mBar) to give 208 g (66%) of 2,2'-(2-(dimethylamino)acetylazanediyl)bis(ethane-2,1-diyl) ditetradecanoate (HEDC-DiMeGly-IN) as an oil.

Preparation of HEDC: 2-(bis(2-(tetradecanoyloxy)ethyl)amino)-N-(2-hydroxyethyl)-N,N-dimethyl-2-oxethanaminium bromide

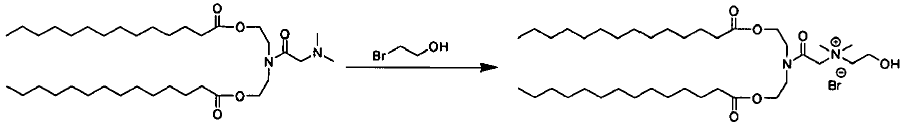

A mixture of HEDC-DiMeGly-IN (206 g, 337 mmol) and 2-bromoethanol (274 g, 2.19 mol) was stirred at 80°C for a period of two hours. HPLC indicated 8.1 % of unreacted dimethylglycin-intermediate. After an additional period of 40 minutes at 80°C, HPLC indicated 7.8% of unreacted dimethylglycin intermediate. Warm (65°C) ethyl acetate (2 L) was added. A blank filtration of the hot solution was washed with hot ethyl acetate (0.5 L). The combined filtrates were cooled to 0°C, and crystallization was initiated by seeding. The product suspension was cooled slowly and stirred at -16 to -18°C for a period of 40 minutes. The precipitate was isolated by filtration, and the filter cake was washed with cold ethyl acetate (200 mL). Drying overnight (20°C/<lmBar) gave 211 g of crude material. The material was then recrystallized from a mixture of ethyl acetate (2.1 L) and ethanol (105 mL) by heating to 35°C and seeding at 25°C. The precipitate was isolated at 10°C, washed with cold ethyl acetate (300 mL) and dried (20°C/<1 mBar) overnight to give 161 g (66%) of 2-(bis(2-(tetradecanoyloxy)ethyl)amino)-N-(2-hydroxyethyl)-N,N-dimethyl-2-oxoethanaminium bromide (HEDC). HPLC indicated 99.5% purity. QTOF MS ESI+: m/z 655.6 (M + H).

### Example 2: Preparation of 2-(bis(3-(tetradecanoyloxy)propyl)amino)-N-(2-hydroxyethyl)-N,N-dimethyl-2-oxo-ethanaminium bromide (Pr-HEDC)

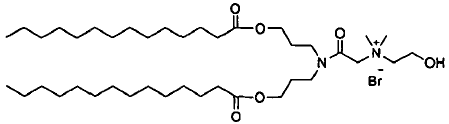

Preparation of Intermediate 1: 3,3'-azanediylbis(propan-1-ol)

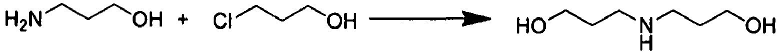

A mixture of 3-amino-1-propanol (14.5 mL, 19.0 mmol), 1-chloro-3-hydroxy propane (8 mL, 95.6 mmol) and H₂O (∼50 mL) was refluxed over 24 hours. Potassium hydroxide (5.40 g) was then added. After dissolution, the whole of the H₂O was evaporated to leave viscous oil and large quantities of potassium chloride. These were filtered and washed with dry acetone and dichloromethane. The organic phase was dried over Na₂SO₄, filtered, and concentrated. Product was then purified via silica gel chromatography with a DCM/MeOH gradient to yield 12.5 g 3,3'-azanediylbis(propan-1-ol).

Preparation of Intermediate 2: *tert*-butyl bis(3-hydroxypropyl)carbamate

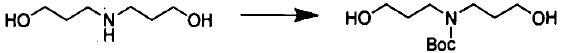

3,3'-Azanediylbis(propan-1-ol) (12.5g, 95.4mmol) was diluted in DCM (25mL). A solution of di-tert-butyl dicarbonate (26g, 119.25mmol) in DCM (25mL) was slowly added while stirring under a blanket of Ar gas. Reaction was allowed to stir overnight. The reaction mixture was concentrated. Purification by silica gel chromatography with a DCM/MeOH gradient yielded tert-butyl bis(3-hydroxypropyl)carbamate.

Preparation of Intermediate 3: ((tert-butoxycarbonyl)azanediyl)bis(propane-3,1-diyl) ditetradecanoate 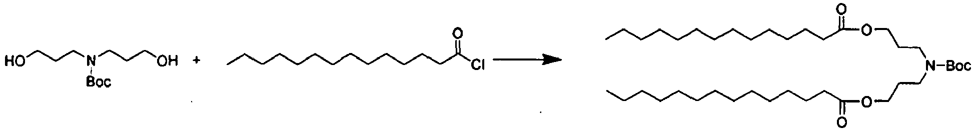 *tert*-Butyl bis(3-hydroxypropyl)carbamate (4.00 g, 17.3 mmol), triethylamine (4.80 ml, 34.6 mmol) and 4-dimethylaminopyridine (529 mg, 4.33 mmol) were dissolved in chloroform (50 mL). While being stirred in an ice-bath, a solution of myristoyl chloride was added in ∼15 min. The addition was carried out in such a way that the temperature of the reaction did not exceed 30°C. The reaction was allowed to stir at room temperature overnight. Next day, MeOH (50 mL) and 0.9% saline solution (50 mL) was added to quench the reaction. The organic layer was separated and washed with 1M NaHCO₃. Solvent was dried with Na₂SO₄, filtered, and concentrated *in vacuo* to yield ((tert-butoxycarbonyl)azanediyl)bis(propane-3,1-diyl) ditetradecanoate as an oil.

Preparation of Intermediate 4: azanediylbis(propane-3,1-diyl) ditetradecanoate TFA salt 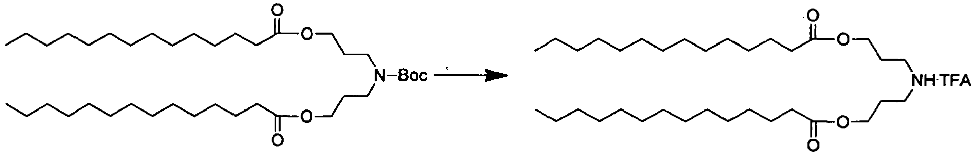 ((*tert*-butoxycarbonyl)azanediyl)bis(propane-3,1-diyl) ditetradecanoate (11.3g, 17.3 mmol) was dissolved in TFA/CHCl₃ (1:1, 20mL) and the mixture was allowed to stir at room temperature for 15 minutes. The mixture was then concentrated *in vacuo.* This was repeated a second time. Residue was then dissolved in DCM and washed with H₂O, dried with Na₂SO₄, and concentrated in vacuo.Purification by silica gel chromatography with a DCM/MeOH gradient yielded azanediylbis(propane-3,1-diyl) ditetradecanoate as a TFA salt (750mg).

Preparation of Intermediate 5: ((2-(dimethylamino)acetyl)azanediyl)bis(propane-3,1-diyl) ditetradecanoate 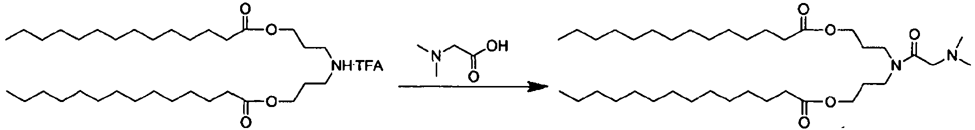 Azanediylbis(propane-3,1-diyl) ditetradecanoate TFA salt (750mg, 1.35 mmol) was diluted with DCM (5mL) and added to a pre-activated mixture ofN,N-dimethylglycine (154mg, 1.49mmol), HATU (616mg, 1.62mmol) and DIEA (495uL, 2.84mmol) in DCM (5mL). Product was flushed with argon and stirred at room temperature overnight, and then concentrated. Purification by silica gel chromatography with a DCM/MeOH gradient yielded 465 mg ((2-(dimethylamino)acetyl)azanediyl)bis(propane-3,1 -diyl)ditetradecanoate.

Preparation of Pr-HEDC: 2-(bis(3-(tetradecanoyloxy)propyl)amino)-N-(2-hydroxyethyl)-N,N-dimethyl-2-oxoethanaminium bromide

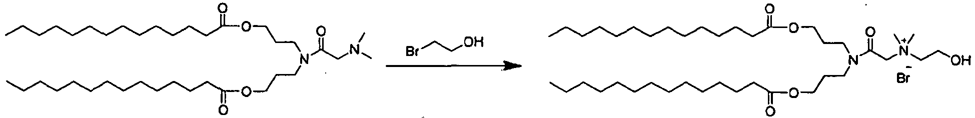

In a sealed system, ((2-(dimethylamino)acetyl)azanediyl)bis(propane-3,1-diyl) ditetradecanoate (246 mg, 0.385 mmol) was dissolved in ACN (10 mL), and 2-bromoethanol (500 uL) was added. The reaction vessel was flushed with inert gas and then sealed. The mixture was heated to 80°C, stirred overnight, and then cooled and concentrated *in vacuo.* Purification by silica gel chromatography with a DCM/MeOH gradient yielded 99 mg 2-(bis(3-(tetradecanoyloxy)propyl)amino)-N-(2-hydroxyethyl)-N,N-dimethyl-2-oxoethanaminium bromide. QTOF MS ESI+: *m*/*z* 683.6 (M + H).

### Example 3: Preparation of 2-(bis(3-(oleoyloxy)propyl)amino)-N-(2-hydroxyethyl)-N,N-dimethyl-2-oxoethanaminium bromide (Pr-HE-DODC)

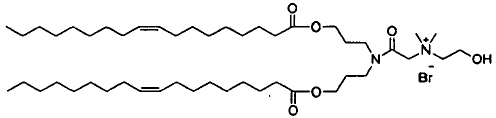

Preparation of Intermediate 1: (Z)-((tert-butoxycarbonyl)azanediyl)bis(propane-3,1-diyl) dioleate 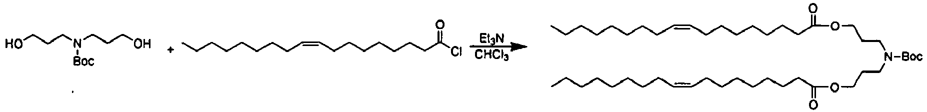 *tert*-Butyl bis(3-hydroxypropyl)carbamate (synthesis previously described), triethylamine and DMAP were dissolved in chloroform. While being stirred in an ice-bath, a solution of oleoyl chloride was added in 15 min. The addition was carried out in such a way that the temperature of the reaction did not exceed 30°C. The reaction was allowed to stir at room temperature overnight. Next day, MeOH (50 mL) and 0.9% saline solution (50mL) was added to quench the reaction. The organic layer was separated and washed with 1M NaHCO₃. Solvent was dried with Na₂SO₄, filtered and concentrated to yield an oil. (Z)-((tert-butoxycarbonyl)azanediyl)bis(propane-3,1-diyl) dioleate was carried forward without and further purification.

Preparation of Intermediate 2: (Z)-azanediylbis(propane-3,1-diyl) dioleate TFA salt 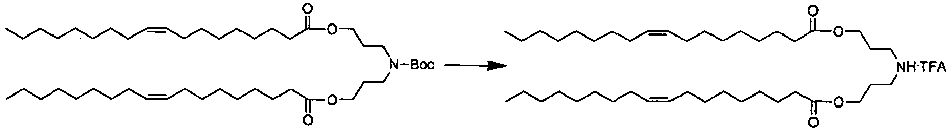 (Z)-((*tert*-Butoxycarbonyl)azanediyl)bis(propane-3,1-diyl) dioleate (13.2 g, 17.3 mmol) was dissolved in TFA/CHCl₃ (1:1, 20 mL) and the mixture was allowed to stir at room temperature for 15 min. The mixture was then concentrated in vacuo. This was repeated a second time. Residue was then dissolved in DCM and washed with H₂O, dried with Na₂SO₄ and concentrated. Purification by silica gel chromatography with a DCM/MeOH gradient yielded (Z)-azanediylbis(propane-3,1-diyl) dioleate TFA salt (750 mg).

Preparation of Intermediate3: (Z)-((2-(dimethylamino)acetyl)azanediyl)bis(propane-3,1-diyl) dioleate 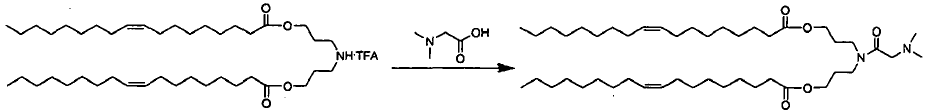 (Z)-azanediylbis(propane-3,1-diyl) dioleate TFA salt (750 mg, 1.13 mmol) was diluted with DCM (5 mL) and added to a pre-activated mixture of N,N-dimethylglycine (128 mg, 1.24 mmol), HATU (517 mg, 1.36 mmol) and DIEA (413 uL, 2.37 mmol) in DCM (5 mL). Flask was flushed with argon and allowed to stir at room temperature overnight. The reaction mixture was concentrated, and subjected to silica gel chromatography with a DCM/MeOH gradient to yield (Z)-((2-(dimethylamino)acetyl)azanediyl)bis(propane-3,1-diyl) dioleate.

Preparation of Pr-HE-DODC: 2-(bis(3-(oleoyloxy)propyl)amino)-N-(2-hydroxyethyl)-N,N-dimethyl-2-oxoethanaminium bromide

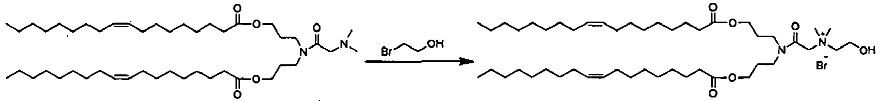

In a sealed system, (Z)-((2-(dimethylamino)acetyl)azanediyl)bis(propane-3,1-diyl) dioleate (269 mg, 0.360 mmol) was dissolved in ACN (10 mL) an d 2-Bromoethanol (200 uL) was added. The reaction vessel was flushed with inert gas and then sealed. Reaction was heated to 80°C and allowed to stir overnight. The reaction mixture was cooled and concentrated. Purification by silica gel chromatography with a DCM/MeOH gradient yielded 2-(bis(3-(oleoyloxy)propyl)amino)-N-(2-hydroxyethyl)-N,N-dimethyl-2-oxoethanaminium bromide (129 mg). QTOF MS ESI+: m/z 791.7 (M + H).

### Example 4: Preparation of 3-(bis(2-(tetradecanoyloxy)ethyl)amino)-N-(2-hydroxyethyl)-N,N-dimethyl-3-oxopro-pan-1-aminium bromide (HE-Et-DC)

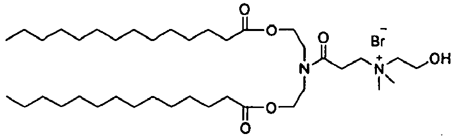

Preparation of Intermediate 1: ((3-(dimethylamino)propanoyl)azanediyl)bis(ethane-2,1-diyl) ditetradeca-noate

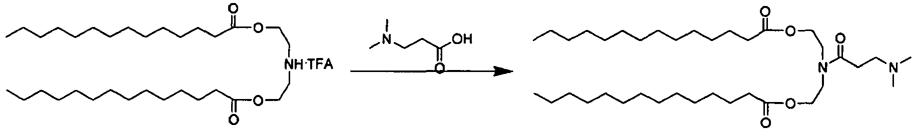

Synthesis of azanediylbis(ethane-2,1-diyl) ditetradecanoate TFA salt previously described. Azanediylbis(ethane-2,1-diyl) ditetradecanoate TFA salt (1.5 g, 2.85 mmol) was diluted with DCM (10 mL) and added to a pre-activated mixture of 3-(dimethylamino)propionic acid HCl salt (482mg, 3.14 mmol), HATU (1.30 g, 3.42 mmol) and DIEA (1.04 mL, 5.98 mmol) in DCM (10 mL). The round-bottomed flask was flushed with argon and the reaction mixture was allowed to stir at room temperature overnight. The reaction mixture was concentrated. Purification by silica gel chromatography with a DCM/MeOH gradient yielded ((3-(dimethylamino)propanoyl)azanediyl)bis(ethane-2,1-diyl) ditetradecanoate.

Preparation of HE-Et-DC: 3-(*bis*(2-(tetradecanoyloxy)ethyl)amino)-N-(2-hydroxyethyl)-N,N-dimethyl-3-oxopropan-1-aminium bromide

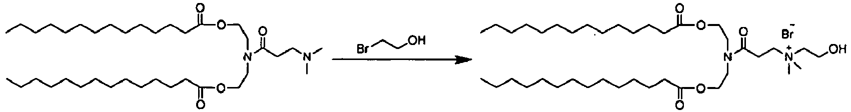

In a sealed system, ((3-(dimethylamino)propanoyl)azanediyl)bis(ethane-2,1-diyl) ditetradecanoate (606mg, 0.970mmol) was dissolved in ACN (10 mL) and 2-Bromoethanol (500 uL) was added. The reaction vessel was flushed with inert gas and then sealed. Reaction was heated to 80°C and stirred overnight, then cooled and concentrated. Purification by silica gel chromatography with a DCM/MeOH gradient yielded 3-(bis(2-(tetradecanoyloxy)ethyl)amino)-N-(2-hydroxyethyl)-N,N-dimethyl-3-oxopropan-1-aminium bromide (80 mg). QTOF MS ESI+: m/z 669.6 (M + H).

### Example 5: Preparation of 3-(bis(2-(oleoyloxy)ethyl)amino)-N-(2-hydroxyethyl)-N,N-dimethyl-3-oxopropan-1-aminium bromide (HE-Et-DODC)

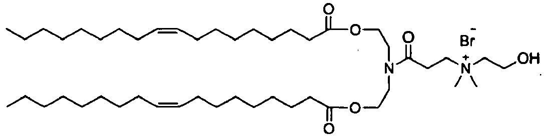

Preparation of Intermediate 1: (Z)-((*tert*-butoxycarbonyl)azanediyl)bis(ethane-2,1-diyl) dioleate

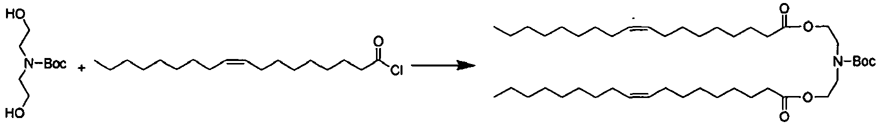

*N*-Boc diethanolamine (Aldrich 15268, Lot# 0001406013, MW 205.25; 17.81 g, 0.087 mole), triethylamine (Aldrich, MW 101.19; 24.4 ml, 0.176 mole) and 4-(dimethylamino)pyridine (Aldrich, MW 122.17; 2.76 g, 1.3 g, 0.023 mole) were dissolved in 350 ml of chloroform. While being stirred, a solution of oleoyl chloride (MW 300.91; 61.6 g, 0.174 mole) in 100 ml of chloroform was added in 10 min (Alternatively, the chloroform solution of N-Boc diethanolamine was immersed in an ice/water bath while oleoyl chloride was added). The addition was carried out in such a way that the temperature of the reaction mixture does not exceed 50°C. The reaction mixture was stirred at room temperature for 2 hrs. A mixture of 200 ml of methanol (EMD MX0485-5, Lot 50350) and 200 ml of 0.9% saline (Sodium chloride, BDH, BDH8014, Lot# 92717) was added to quench the reaction. The organic layer was separated and was washed with 2 x 100 ml of dilute aqueous sodium bicarbonate (Aldrich S6014, Batch# 095K0143). The solvent was removed by rotary evaporation to afford 59.5 g of crude product as pale yellow oil (MW 734.14; 59.5 g, 0.081 mole, 100% yield). This material was used for the next step without further purification. 1H NMR (400 MHz, CDC13) 0.87 (t,6H, CH3), 1.20-1.40 (m, 40H, CH2), 1.45 (s, 9H, tBu CH3), 1.59 (m, 4H, CH2CH2C(=O)), 2.00(m, 8H, CH2CH=CH), 2.33 (t, 4H, CH2C(=O)), 3.48 (m, 4H, NC**H**2CH2O), 4.18 (m, 4H,NCH2CH2O), 5.33 (m, 4H, CH=CH).

Preparation of Intermediate 2: (Z)-azanediylbis(ethane-2,1-diyl) dioleate TFA salt

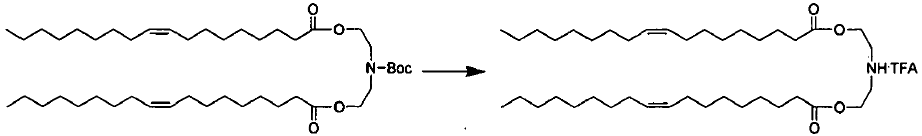

The (Z)-((*tert*-butoxycarbonyl)azanediyl)bis(ethane-2,1-diyl) dioleate (59.5 g, 0.081 mole) was treated twice with 100 ml trifluoroacetic acid (Alfa Aesar Stock# A12198, Lot# D07W005, MW 114.02; 100 ml, 1.35 mole) and 100 ml of chloroform (Aldrich 154733, Lot# KBF5943V). Each consisted of stirring at room temperature for ten minutes, and the solvent was removed by rotary evaporation at the end of each treatment. After the second treatment, the reaction mixture was concentrated by rotary evaporation. The residue was dissolved in 200 ml of methylene chloride and the mixture had been washed with 100 ml of water twice. The residue was purified by silica gel chromatography using a mixture of methanol (EMD MX0485-5, Lot 50350) and methylene chloride (EMD DX0835-5, Lot 51090) as eluent to yield 44 g of (Z)-azanediylbis(ethane-2,1-diyl) dioleate TFA salt (44.0 g). 1H NMR (400 MHz, CDC13) 0.87 (t, 6H, CH3), 1.20-1.40 (m, 40H, CH2), 1.59 (m, 4H, C**H**2CH2C(=O)),2.00 (m, 8H, C**H**2CH=CH), 2.33 (t, 4H, C**H**2C(=O)), 3.31 (m, 4H, NC**H**2CH2O), 4.38 (m, 4H, NCH2C**H**2O), 5.33 (m, 4H, CH=CH).

Preparation of Intermediate 3: (((Z)-((3-(dimethylamino)propanoyl)azanediyl)bis(ethane-2,1-diyl) dioleate 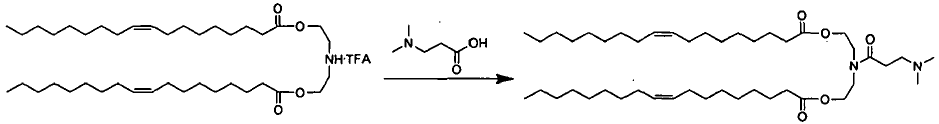 (Z)-azanediylbis(ethane-2,1-diyl) dioleate TFA salt (1.50 g, 2.37 mmol) was diluted with DCM (10 mL) and added to a pre-activated mixture of 3-(dimethylamino)propionic acid HCl salt (383 mg, 2.49 mmol), HATU (1034 mg, 2.72 mmol) and DIEA (831 uL, 4.77 mmol) in DCM (10 mL). The round-bottomed flask was flushed with argon and the reaction mixture was allowed to stir at room temperature overnight. The reaction mixture was concentrated. Purification by silica gel chromatography with a DCM/MeOH gradient yielded (Z)-((3-(dimethylamino)propanoyl)azanediyl)bis(ethane-2,1-diyl) dioleate.

Preparation of HE-Et-DODC: 3-(bis(2-(oleoyloxy)ethyl)amino)-N-(2-hydroxyethyl)-N,N-dimethyl-3-oxo-propan-1-aminium bromide

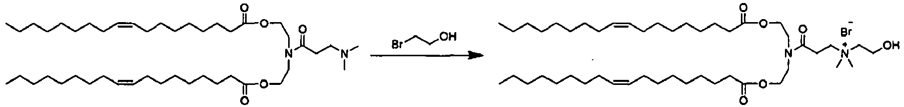

In a sealed system, (((Z)-((3-(dimethylamino)propanoyl)azanediyl)bis(ethane-2,1-diyl) dioleate (588 mg, 0.802 mmol) was dissolved in ACN (10 mL) and 2-bromoethanol (200 uL) was added. The reaction vessel was flushed with inert gas and then sealed. Reaction was heated to 80°C and stirred overnight, then cooled and concentrated *in vacuo.* Purification by silica gel chromatography with a DCM/MeOH gradient yielded 3-(bis(2-(oleoyloxy)ethyl)amino)-N-(2-hydroxyethyl)-N,N-dimethyl-3-oxopropan-1-aminium bromide (160 mg). QTOF MS ESI+: m/z 764.3 (M + H).

### Example 6: Preparation of 4-(bis(2-(tetradecanoyloxy)ethyl)amino)-N-(2-hydroxyethyl)-N,N-dimethyl-4-oxobutan-1-aminium bromide (HE-Pr-DC)

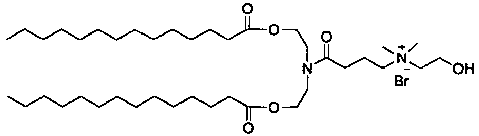

Preparation of Intermediate 1: ((4-(dimethylamino)butanoyl)azanediyl)bis(ethane-2,1-diyl) ditetra-decanoate 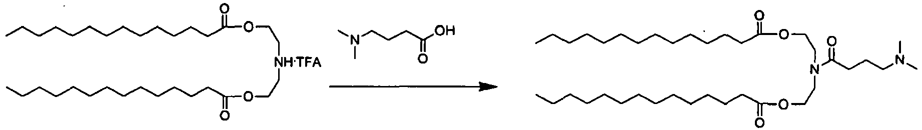 Synthesis of azanediylbis(ethane-2,1-diyl) ditetradecanoate TFA salt previously described. Azanediylbis(ethane-2,1-diyl) ditetradecanoate TFA salt (1.00 g, 1.90 mmol) was diluted with DCM (5 mL) and added to a pre-activated mixture of 4-(dimethylamino) butyric acid HCl salt (382 mg, 2.28 mmol), HATU (867 mg, 2.28mmol) and DIEA (728 uL, 4.18 mmol) in DCM (5 mL). The flask was flushed with argon and the reaction mixture was stirred at room temperature overnight, then concentrated. Purification by silica gel chromatography with a DCM/MeOH gradient yielded ((4-(dimethylamino)butanoyl)azanediyl)bis(ethane-2,1-diyl) ditetradecanoate.

Preparation of HE-Pr-DC: 4-(bis(2-(tetradecanoyloxy)ethyl)amino)-N-(2-hydroxyethyl)-N,N-dimethyl-4-oxobutan-1-aminium bromide

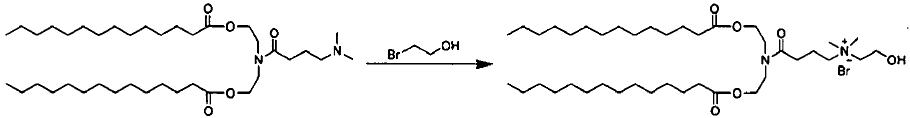

In a sealed system, ((4-(dimethylamino)butanoyl)azanediyl)bis(ethane-2,1-diyl) ditetradecanoate (300 mg, 0.469 mmol) was dissolved in ACN (5 mL) and 2-bromoethanol (500 uL) was added. The reaction vessel was flushed with inert gas and then sealed. Reaction was heated to 80°C and stirred overnight, then concentrated. Purification by silica gel chromatography with a DCM/MeOH gradient yielded 4-(bis(2-(tetradecanoyloxy)ethyl)amino)-N-(2-hydroxyethyl)-N,N-dimethyl-4-oxobutan-1-aminium bromide (140 mg). LCMS ESI+: *m*/*z* 684.4 (M + H).

### Example 7: Preparation of 4-(bis(2-(oleoyloxy)ethyl)amino)-N-(2-hydroxyethyl)-N,N-dimethyl-4-oxobutan-1-aminium bromide (HE-Pr-DODC)

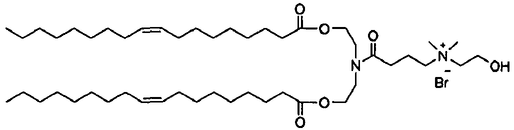

Preparation of Intermediate 1: (Z)-((4-(dimethylamino)butanoyl)azanediyl)bis(ethane-2,1-diyl) dioleate 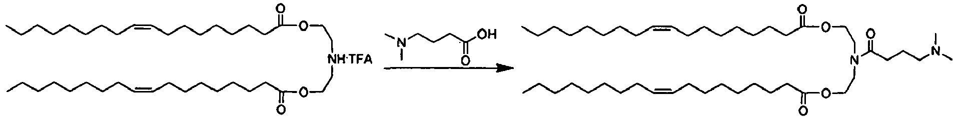 Synthesis of (Z)-azanediylbis(ethane-2,1-diyl) dioleate TFA salt previously described. (Z)-azanediylbis(ethane-2,1-diyl) dioleate TFA salt (1.00 g, 1.58 mmol) was diluted with DCM (5 mL) and added to a pre-activated mixture of 4-(Dimethylamino) butyric acid HCl salt (317 mg, 1.89 mmol), HATU (719 mg, 1.89 mmol) and DIEA (606 uL, 3.48 mmol) in DCM (5 mL). The flask was flushed with argon and the reaction mixture stirred at room temperature overnight, then concentrated. Purification by silica gel chromatography with a DCM/MeOH gradient yielded (Z)-((4-(dimethylamino)butanoyl)azanediyl)bis(ethane-2,1-diyl) dioleate.

Preparation of HE-Pr-DODC: 4-(bis(2-(oleoyloxy)ethyl)amino)-N-(2-hydroxyethyl)-N,N-dimethyl-4-oxobutan-1-aminium bromide

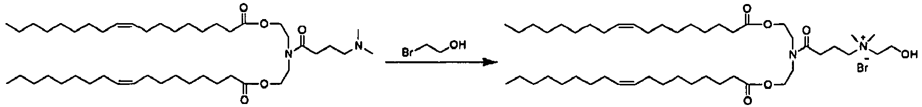

In a sealed system, ((4-(dimethylamino)butanoyl)azanediyl)bis(ethane-2,1-diyl) ditetradecanoate (400 mg, 0.535 mmol) was dissolved in ACN (5 mL) and 2-Bromoethanol (500 uL) was added. The reaction vessel was flushed with inert gas and then sealed. Reaction was heated to 80°C and stirred overnight, then concentrated. Purification by silica gel chromatography with DCM/MeOH gradient yielded 4-(bis(2-(oleoyloxy)ethyl)amino)-N-(2-hydroxyethyl)-N,N-dimethyl-4-oxobutan-1-aminium bromide (255 mg). LCMS ESI+: m/z 792.5 (M + H).

### Example 8: Preparation of 2-(bis(2-(oleoyloxy)ethyl)amino)-N,N-bis(2-hydroxyethyl)-N-methyl-2-oxoethanaminium bromide (HE2DODC)

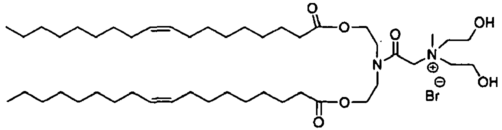

Preparation of Intermediate 1: (Z)-((2-bromoacetyl)azanediyl)bis(ethane-2,1-diyl) dioleate

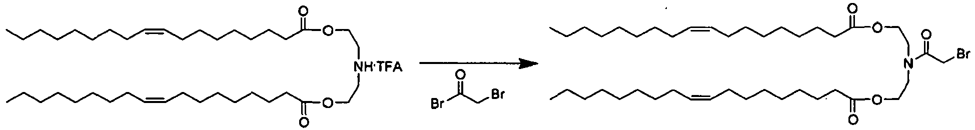

Synthesis of (Z)-azanediylbis(ethane-2,1-diyl) dioleate TFA salt previously described. (Z)-azanediylbis(ethane-2,1-diyl) dioleate TFA salt (1.50 g, 2.34 mmol) was dissolved in DCM (20mL) and placed in an ice-bath. Bromoacetyl bromide (214 uL, 2.46 mmol) was added followed by triethylamine (685 uL, 4.91 mmol). The ice-bath was removed and the reaction was allowed to stir overnight at room temperature under inert gas, then diluted with DCM to 100 mL, and washed with a 1M HCl (75 mL), H₂O (75 mL), saturated NaHCO₃ solution (75 mL) and saturated brine solution (75 mL). All aqueous washes were back extracted with DCM (25 mL). Dried organics with MgSO₄, filtered and concentrated *in vacuo.* Purification by silica gel chromatography with ethyl acetate yielded (Z)-((2-bromoacetyl)azanediyl)bis(ethane-2,1-diyl) dioleate (1.22 g).

Preparation of HE2DODC: 2-(bis(2-(oleoyloxy)ethyl)amino)-N,N-bis(2-hydroxyethyl)-N-methyl-2-oxoethanaminium bromide

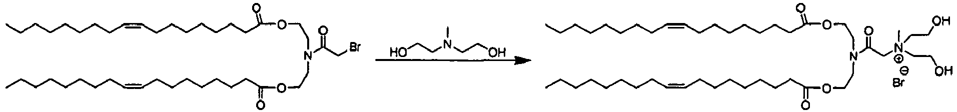

In a sealed system, (Z)-((2-bromoacetyl)azanediyl)bis(ethane-2,1-diyl) dioleate (2.08 g, 2.75 mmol) was combined with N-methyldiethylamine (1.58 mL, 13.8 mmol) was added. The reaction vessel was flushed with inert gas and then sealed. Reaction was heated to 50°C and stirred overnight, and then concentrated. Purification by silica gel chromatography with DCM/MeOH gradient yielded 2-(bis(2-(oleoyloxy)ethyl)amino)-N,N-bis(2-hydroxyethyl)-N-methyl-2-oxoethanaminium bromide (479 mg). LCMS ESI+: m/z 793.7 (M + H).

### Example 9: Preparation of 2-(bis(2-((9Z,12Z)-octadeca-9,12-dienoyloxy)ethyl)amino)-N-(2-hydroxyethyl)-N,N-dimethyl-2-oxoethanaminium bromide (HEDC-DLin)

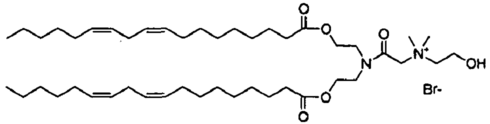

2-(bis(2-((9Z,12Z)-octadeca-9,12-dienoyloxy)ethyl)amino)-N-(2-hydroxyethyl)-N,N-dimethyl-2-oxoethanaminium bromide was prepared in similar fashion as HEDC with the substitution of (9Z,12Z)-octadeca-9,12-dienoyl chloride for myristoyl chloride.

### Example 10: Preparation of 2-(bis(2-(dodecanoyloxy)ethyl)amino)-N-(2-hydroxyethyl)-N,N-dimethyl-2-oxoethanaminium bromide (HEDC-12) (comparative example)

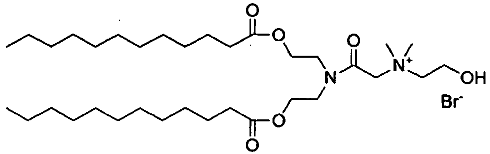

2-(bis(2-(Dodecanoyloxy)ethyl)amino)-N-(2-hydroxyethyl)-N,N-dimethyl-2-oxoethanaminium bromide was prepared in similar fashion as HEDC with the substitution of dodecanoyl chloride for myristoyl chloride.

### Example 11: Preparation of 2-((2-(bis(2-(tetradecanoyloxy)ethyl)amino)-2-oxoethyl)thio)-N-(2-hydroxyethyl)-N,N-dimethylethanaminium bromide (HES104)

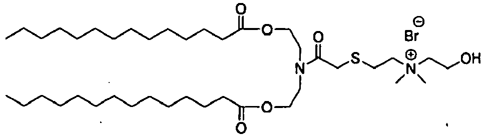

Preparation of Intermediate 1: ((2-((2-(dimethylamino)ethyl)thio)acetyl)azanediyl)bis(ethane-2,1-diyl) ditetradecanoate (S104).

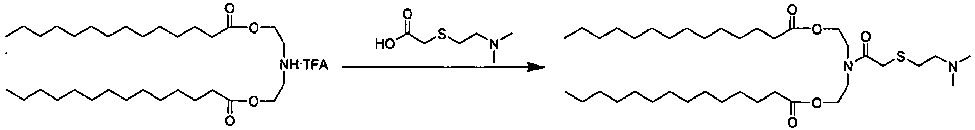

Synthesis of azanediylbis(ethane-2,1-diyl) ditetradecanoate TFA salt previously described. Azanediylbis(ethane-2,1-diyl) ditetradecanoate TFA salt (152 g, 238 mmol) was stirred with DCM (2.3 L) and 10% potassium bicarbonate (1.15 L) at 0-5°C. The organic phase was separated and the aqueous phase is further extracted with DCM (1.15 L). The combined organic phases were stirred with magnesium sulfate hydrate (236 g) for a period of 30 minutes at 0-5°C, filtrated and washed with DCM (1.15 L). To the combined filtrates were added 2-((2-(dimethylamino)ethyl)thio)acetic acid hydrochloride (57.0 g, 285 mmol), EDC hydrochloride (68.4 g, 357 mmol) and DMAP (2.91 g, 23.8 mmol), and the suspension was stirred overnight at ambient temperature, after which period of time a clear solution was formed. MQ-water (2.3 L) and methanol (460 mL) were added and after having stirred for a period of 10 minutes the clear organic phase was separated. The turbid aqueous phase (pH 3.0) was extracted with DCM (575 mL). The combined organic extracts were concentrated yielding 143 g of crude material as the hydrochloride salt. The crude material (142.6g) was transferred to a distillation flask with DCM (500 mL), and ethyl acetate (1 L) was added. The solution was heated to distillation at atmospheric pressure, and distillation was continued over a period of 70 minutes in order to obtain a temperature of the residue of 76°C. A total volume of 1.4 L was obtained by addition of ethyl acetate (800 mL), and ethanol (70 mL) was added. The clear solution at 50°C was cooled to 37°C and seed crystals were added. Having observed initiation of significant crystallization over a period of 10 minutes at 37-35°C, the suspension was cooled and stirred at 0°C overnight and the precipitate was isolated by filtration, and washed with cold ethyl acetate (210 mL). Drying to a constant weight at ambient temperature in oil pump vacuum over a period of 4.5 hours gave 134 g of recrystallized material as the hydrochloride salt, white crystalline solid. Tripotassium phosphate (85 g, 0.40 mol) and dipotassium hydrogen phosphate (226 g, 1.30 mol) was added to purified water (1.7 L), and the solution formed with pH 10.9 was cooled to 18-20°C. DCM (1.3 L) and recrystallized S104 hydrochloride (133 g, 0.188 mol) were added, and the mixture was stirred for a period of 10 min minutes. A clear organic phase was separated at moderate rate (over a period of 35 minutes), and the turbid aqueous phase was further extracted with DCM (650 mL). The combined organic phases were stirred with anhydrous magnesium sulfate (65 g) for a period of 40 min, and the mixture was filtered, washing with DCM (200 mL). The combined filtrates were evaporated from a 50°C water bath under reduced pressure (down to 20 mBar, at which pressure evaporation was continued for one hour). Additional evaporation from a 15-20°C water bath at oil pump vacuum, resulted in 126 g partially solidified oil. Cooling in - 20°C cooling bath gave complete solidification, and after drying at -20°C under an oil pump vacuum, we have obtained 126 g of ((2-((2-(dimethylamino)ethyl)thio)acetyl)azanediyl)bis(ethane-2,1-diyl) ditetradecanoate (S104). HPLC indicated 98.1 % purity.

Preparation of HES104: 2-((2-(bis(2-(tetradecanoyloxy)ethyl)amino)-2-oxoethyl)thio)-N-(2-hydroxyethyl)-N,N-dimethylethanaminium bromide 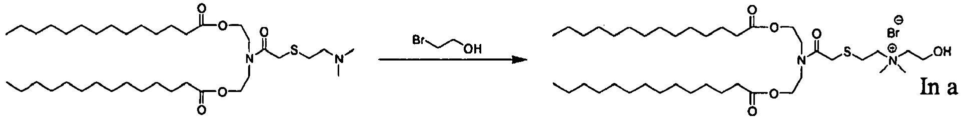 sealed system, ((2-((2-(dimethylamino)ethyl)thio)acetyl)azanediyl)bis(ethane-2,1-diyl) ditetradecanoate (1.00 g, 1.49 mmol) was combined with 2-bromoethanol (687 uL, 9.69 mmol) was added. The reaction vessel was flushed with inert gas and then sealed. Reaction was heated to 75°C and allowed to stir overnight, then cooled, and concentrated *in vacuo.* Purification by silica gel chromatography with DCM/MeOH gradient yielded 2-((2-(bis(2-(tetradecanoyloxy)ethyl)amino)-2-oxoethyl)thio)-N-(2-hydroxyethyl)-N,N-dimethylethanaminium bromide (HES104) (790 mg). LCMS ESI+: m/z 715.7 (M + H).

### Example 12: Preparation of 2-((2-(bis(2-(oleoyloxy)ethyl)aniino)-2-oxoethyl)thio)-N-(2-hydroxyethyl)-N,N-dimethylethanaminium bromide (HES104-DO)

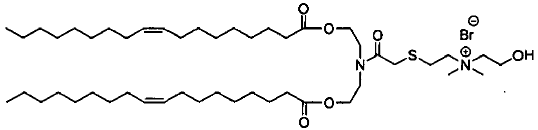

Preparation of Intermediate 1: (Z)-((2-((2-(dimethylamino)ethyl)thio)acetyl)azanediyl)bis(ethane-2,1-diyl) dioleate

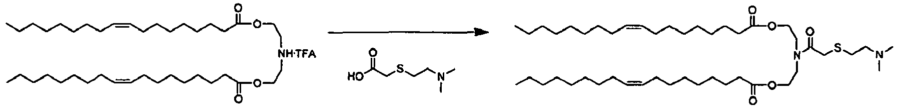

Synthesis of (Z)-azanediylbis(ethane-2,1-diyl) dioleate TFA salt previously described. (Z)-azanediylbis(ethane-2,1-diyl) dioleate TFA salt (4.06 g, 6.41 mmol) was stirred in DCM (60 mL) with 10% K₂CO₃ (30 mL) at 0-5°C. After 30 min, the organic phase was separated and the aqueous phase was further extracted with DCM (30 mL). The combined organic phases were stirred with anhydrous MgSO₄ for a period of 30 minutes at 0-5°C, filtered, and washed with DCM (30 mL). To the combined filtrates were added to 2-((2-(dimethylamino)ethyl)thio)acetic acid (1.26 g, 7.70 mmol), EDC HCl salt (1.84 g, 9.62 mmol), DMAP (78.3 mg, 0.64 mmol). The thin suspension was stirred overnight at room temperature; after which a period of time, the solution became clear. Next day, deionized water (60 mL) and methanol (30 mL) were added. After stirring for 10 minutes, the clear organic layer was isolated. The turbid aqueous phase is extracted with DCM. The combined organic extracts were concentrated. Crude material was filtered through a plug of silica and taken up in DCM (40 mL) and PBS (pH=11, 50 mL) was added. The mixture was stirred at room temperature for 10 min. Then, the organic phase was separated and the aqueous phase is extracted again with DCM (15 mL). The combined organic phases were stirred with anhydrous MSO4 for a period of 30 min. The mixture was then filtered, and washed with DCM. The combined filtrates were concentrated in vacuo to yield (Z)-((2-((2-(dimethylamino)ethyl)thio)acetyl)azanediyl)bis(ethane-2,1-diyl) dioleate (3.44 g).

Preparation of HES104-DO

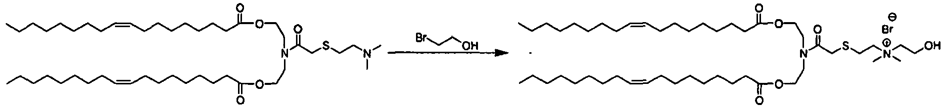

In a sealed system, (Z)-((2-((2-(dimethylamino)ethyl)thio)acetyl)azanediyl)bis(ethane-2,1-diyl) dioleate (540mg, 0.693mmol) was combined with 2-bromoethanol (319uL, 4.50) was added. The reaction vessel was flushed with inert gas and then sealed. Reaction was heated to 75°C and allowed to stir overnight. Next day, cooled and concentrated *in vacuo.* Purification by silica gel chromatography with DCM/MeOH gradient yielded 2-((2-(bis(2-(oleoyloxy)ethyl)amino)-2-oxoethyl)thio)-N-(2-hydroxyethyl)-N,N-dimethylethanaminium bromide (324mg).
LCMS ESI+: m/z 823.8 (M + H).

### Example 13: Preparation of 2-((bis(2-(oleoyloxy)ethyl)carbamoyl)thio)-N-(2-hydroxyethyl)-N,N-dimethylethan-aminium bromide (HETU104-DO)

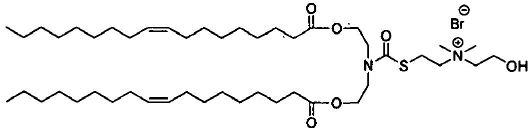

Preparation of Intermediate 1: (Z)-((((2-(dimethylamino)ethyl)thio)carbonyl)azanediyl)bis(ethane-2,1-diyl) dioleate

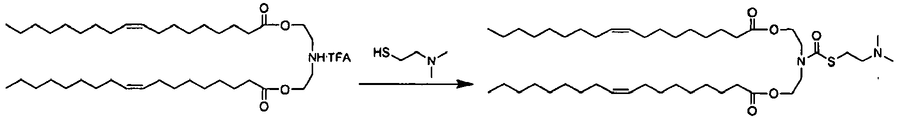

Synthesis of (Z)-((tert-butoxycarbonyl)azanediyl)bis(ethane-2,1-diyl) dioleate previously described. (Z)-((tert-butoxycarbonyl)azanediyl)bis(ethane-2,1-diyl) dioleate (4.2 g, 5.72 mmol) was dissolved in DCM (20mL) and cooled to 0°C in an ice bath. TFA (20mL) was added and the mixture was allowed to stir under a blanket of inert gas for 20 minutes. Afterwards, the mixture was concentrated in vacuo. The residue was partitioned between 10% K₂CO₃ (20 mL) and DCM (20 mL). The mixture was stirred in an ice bath for 20 minutes. Organic portion was collected, and turbid aqueous layer was extracted with DCM (2 x 10 mL). The combined organic extracts were added with anhydrous MgSO₄ and stirred at 0°C for 20 minutes. The suspension was filtered and washed DCM (10 mL). Diphosgene (1.38 mL, 11.4 mmol) was added to (Z)-azanediylbis(ethane-2,1-diyl) dioleate material in DCM and stirred under a blanket of inert gas at room temperature. Next day, DCM and excess diphosgene was removed in vacuo. 2-(dimethylamino)ethane thiol HCl salt (4.05 g, 28.6 mmol) was taken up in DCM (50 mL) and triethylamine (5.2 mL, 37.2 mmol) and added to (Z)-((chlorocarbonyl)azanediyl)bis(ethane-2,1-diyl) dioleate residue. Reaction mixture was allowed to stir overnight at room temperature. Next day, the mixture was diluted with DCM and washed with 0.3M HCl (75 mL), water (75 mL) and 10% K₂CO₃ (75mL). Back extracted all aqueous washes with DCM (25mL). The organics was dried over anhydrous MgSO₄, filtered, and concentrated *in vacuo.* Purification by silica gel chromatography with DCM/MeOH gradient yielded (Z)-((((2-(dimethylamino)ethyl)thio)carbonyl)azanediyl)bis(ethane-2,1-diyl) dioleate (1.90 g).

Preparation of HETU104DO

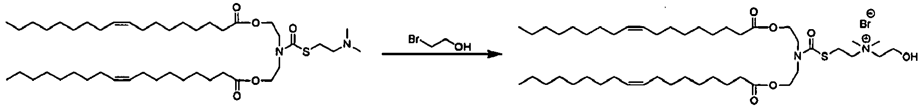

In a sealed system, (Z)-((((2-(dimethylamino)ethyl)thio)carbonyl)azanediyl)bis(ethane-2,1-diyl) dioleate (615 mg, 0.804 mmol) was combined with 2-Bromoethanol (370 uL, 5.22 mmol) was added. The reaction vessel was flushed with inert gas and then sealed. Reaction was heated to 75°C and allowed to stir overnight, then cooled and concentrated *in vacuo.* Purified by silica gel chromatography with a DCM/MeOH gradient yielded 2-((bis(2-(oleoyloxy)ethyl)carbamoyl)thio)-N-(2-hydroxyethyl)-N,N-dimethylethanaminium bromide (473 mg). LCMS ESI+: m/z 809.8 (M + H).

### Example 14: Synthesis of Dope-Glu-VA (preparative example)

### Preparation of (Z)-(2R)-3-(((2-(5-(((2E,4E,6E,8E)-3,7-dimethyl-9-(2,6,6-trimethylcyclohex-1-en-1-yl)nona-2,4,6,8-tetraen-1-yl)oxy)-5-oxopentanamido)ethoxy)(hydroxy)phosphoryl)oxy)propane-1,2-diyl dioleate (DOPE-Glu-VA)

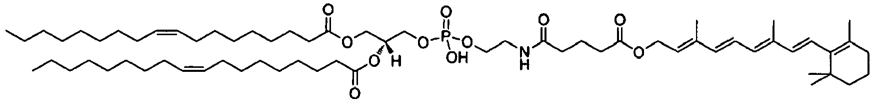

Preparation of Intermediate 1: 5-(((2E,4E,6E,8E)-3,7-dimethyl-9-(2,6,6-trimethylcyclohex-1-en-1-yl)nona-2,4,6,8-tetraen-1-yl)oxy)-5-oxopentanoic acid

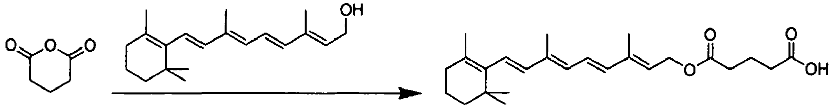

Glutaric anhydride (220 mg, 1.93 mmol) and retinol (500 mg, 1.75 mmol) were dissolved in dichloromethane (5 mL) in an amber-colored vial. Triethylamine (513 *u*l, 3.68 mmol) was added and the vial was flushed with argon. Reaction mixture was allowed to stir at room temperature for 4 hours. The material was concentrated and purified by silica gel chromatography with a dichloromethane/methanol gradient. Fractions were pooled and concentrated to yield yellowish oil (700 mg). The product was verified by NMR.

Preparation of DOPE-Glu-VA: (Z)-(2R)-3-(((2-(5-(((2E,4E,6E,8E)-3,7-dimethyl-9-(2,6,6-trimethylcyclohex-1-en-1-yl)nona-2,4,6, 8-tetraen-1-yl)oxy)-5-oxopentanamido)ethoxy)(hydroxy)phosphoryl)oxy)propane-1,2-diyl dioleate

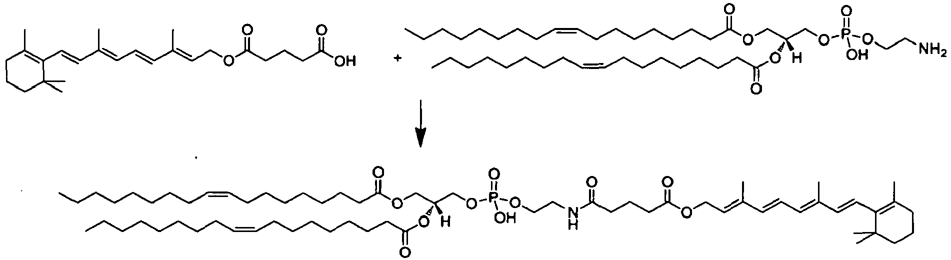

1,2-dioleoyl-sn-glycero-3-phosphoethanolamine (500 mg, 0.672 mmol), N,N,N',N'-Tetramethyl-O-(7-azabenzotriazol-1-yl)uronium hexafluorophosphate (306.5mg, 0.806 mmol) and 5-(((2E,4E,6E,8E)-3,7-dimethyl-9-(2,6,6-trimethylcyclohex-1-en-1-yl)nona-2,4,6,8-tetraen-1-yl)oxy)-5-oxopentanoic acid (269 mg, 0.672 mmol) was dissolved in chloroform/DMF (10 mL, 1:1 mixture) in an amber-colored vial flushed with argon and N,N-Diisopropylethylamine (300 *u*L, 1.68 mmol) was added. Reaction mixture was allowed to stir overnight at room temperature. The reaction mixture was concentrated and then purified by silica gel chromatography using a dichloromethane/methanol gradient. The fractions were pooled and concentrated to yield yellowish oil (460 mg, 61%). Verified product by NMR. ¹H NMR (400 MHz), δ_{H}: 8.6 (d, 1H), 8.27 (d, 1H), 6.57-6.61 (dd, 1H), 6.08-6.25 (m, 4H), 5.57 (t, 1H), 5.30-5.34 (m, 4H), 5.18 (m, 1H), 4.68-4.70 (d, 2H), 4.28-4.35 (m, 1H), 4.05-4.15 (m, 1H), 3.81-3.97 (m, 4H), 3.52-3.62 (m, 1H), 3.35-3.45 (m, 2H), 2.95-3.05 (m, 1H), 2.33-2.35 (t, 3H), 2.2-2.3 (m, 7H), 1.9-2.05 (m, 17H), 1.85 (s, 3H), 1.69 (s, 3H), 1.5-1.65 (m, 6H), 1.4-1.5 (m, 2H), 1.18-1.38 (m, ∼40H), 1.01 (s, 3H), 0.84-0.88 (m, 12H).

### Example 15: DOPE-Glu-NH-VA (preparative example)

Preparation of (Z)-(2R)-3-(((2-(4-((2E,4E,6E,8E)-3,7-dimethyl-9-(2,6,6-trimethylcyclohex-1-en-1-yl)nona-2,4,6,8-tetraenamido)butanamido)ethoxy)(hydroxy)phosphoryl)oxy)propane-1,2-diyl dioleate (DOPE-Glu-NH-VA)

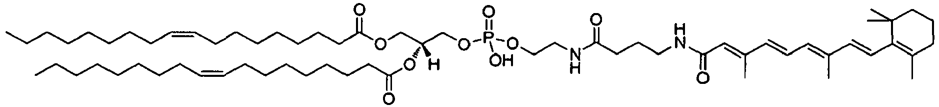

Preparation of Intermediate 1: (Z)-(2R)-3-(((2-(4-aminobutanamido)ethoxy)(hydroxy)phosphoryl)oxy)propane-1,2-diyl dioleate

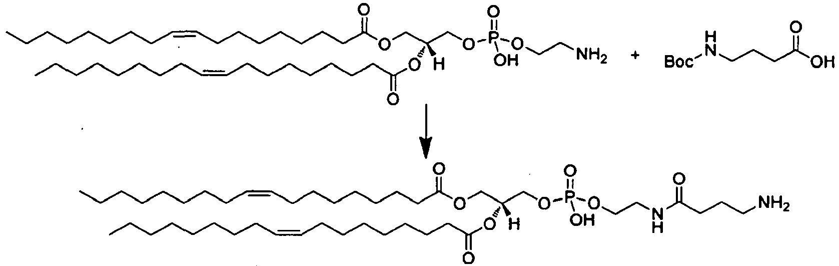

1,2-dioleoyl-sn-glycero-3-phosphoethanolamine (2500 mg, 3.36 mmol), Boc-GABA-OH (751 mg, 3.70 mmol) and N,N,N',N'-Tetramethyl-O-(7-azabenzotriazol-1-yl)uronium hexafluorophosphate (1531 mg, 4.03 mmol) were dissolved in a DMF/chloroform (25 mL, 1:1 mixture). N,N-Diisopropylethylamine (880 *u*L, 5.05 mmol) was added and the mixture was allowed to stir at room temperature overnight under a blanket of argon. The reaction mixture was diluted with ∼200 mL H₂O and product was extracted with dichloromethane (3x100 ml). The product was washed with ∼75 mL pH 4.0 PBS buffer, dried organics with sodium sulfate, filtered and concentrated. Material was then purified via silica gel chromatography with a dichloromethane/methanol gradient, and concentrated to yield colorless oil (2.01 g, 64%). The product was verified by NMR. Material was then taken up in 30 mL of 2 M HCl/diethyl ether. Reaction was allowed to stir at room temperature in a H₂O bath. After 2 hours, the solution was concentrated to yield (Z)-(2R)-3-(((2-(4-aminobutanamido)ethoxy)(hydroxy)phosphoryl)oxy)propane-1,2-diyl dioleate.

Preparation of DOPE-Glu-NH-VA: (Z)-(2R)-3-(((2-(4-((2E,4E,6E,8E)-3,7-dimethyl-9-(2,6,6-trimethylcyclohex-1-en-1-yl)nona-2,4,6,8-tetraenamido)butanamido)ethoxy)(hydroxy)phosphoryl)oxy)propane-1,2-diyl dioleate

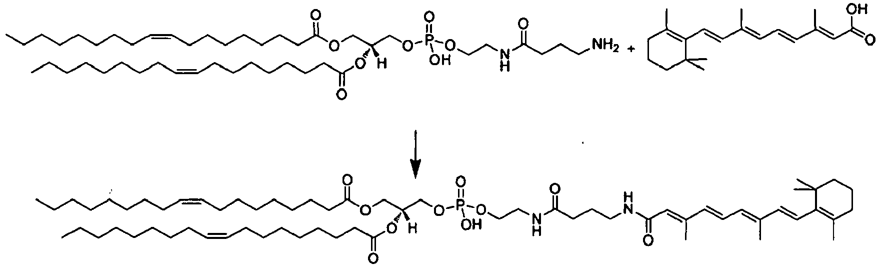

(Z)-(2R)-3-(((2-(4-aminobutanamido)ethoxy)(hydroxy)phosphoryl)oxy)propane-1,2-diyl dioleate (1200 mg, 1.45 mmol), retinoic acid (500 mg, 1.66 mmol) and N,N,N',N'-Tetramethyl-O-(7-azabenzotriazol-1-yl)uronium hexafluorophosphate (689 mg, 1.81 mmol) was suspended in DMF/chloroform (10 mL, 1:1 mixture). N,N-Diisopropylethylamine (758 *u*L, 4.35 mmol) was added. The round bottom flask was flushed with argon and covered with aluminum foil. Reaction mixture was stirred at room temperature for 4 hours, partitioned in dichloromethane (75 mL) and H₂O (75 mL), extracted with dichloromethane, dried (sodium sulfate), filtered and concentrated. Purification by silica gel chromatography using a dichloromethane/methanol gradient yielded (Z)-(2R)-3-(((2-(4-((2E,4E,6E,8E)-3,7-dimethyl-9-(2,6,6-trimethylcyclohex-1-en-1-yl)nona-2,4,6,8-tetraenamido)butanamido)ethoxy)(hydroxy)phosphoryl)oxy)propane-1,2-diyl dioleate (292 mg, 18%). The product was characterized by LCMS & NMR. ¹H NMR (400 MHz), δ_{H}: 8.55 (s, 1H), 8.2 (d, 1H), 7.3 (s, 1H), 6.6 (dd, 1H), 6.10-6.27 (m, 5H), 5.5 (t, 1H), 5.31 (s, 4H), 5.1-5.2 (m, 2H), 4.68 (d, 2H), 4.3 (d, 2H), 4.1 (m, 2H), 3.9 (m, 8H), 3.58 (q, 4H), 3.4 (s, 4H), 3.0 (q, 4H), 2.33-2.35 (t, 3H), 2.2-2.3 (m, 7H), 1.9-2.05 (m, 17H), 1.85 (s, 3H), 1.69 (s, 3H), 1.5-1.65 (m, 6H), 1.4-1.5 (m, 2H), 1.18-1.38 (m, ∼40H), 1.01 (s, 3H), 0.84-0.88 (m, 12H). MS: *m*/*z* 1112.44 (M + H⁺).

### Example 16: DSPE-PEG550-VA (preparative example)

Preparation of (2R)-3-(((((45E,47E,49E,51E)-46,50-dimethyl-4,44-dioxo-52-(2,6,6-trimethylcyclohex-1-en-1-yl)-7,10,13,16,19,22,25,28,31,34,37,40-dodecaoxa-3,43-diazadopentaconta-45,47,49,51-tetraen-1-yl)oxy)(hydroxy)phosphoryl)oxy)propane-1,2-diyl distearate (DSPE-PEG550-VA)

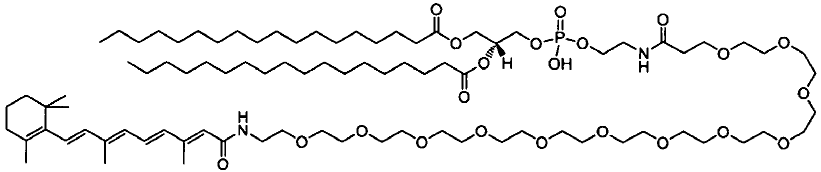

Preparation of Intermediate 1: (2R)-3-((((2,2-dimethyl-4,44-dioxo-3,8,11,14,17,20,23,26,29,32,35,38,41-tridecaoxa-5,45-diazaheptatetracontan-47-yl)oxy)(hydroxy)phosphoryl)oxy)propane-1,2-diyl distearate

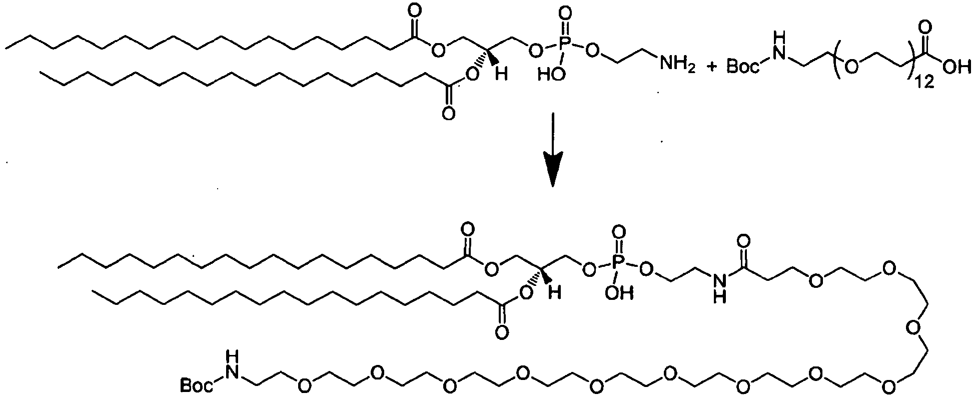

1,2-distearoyl-sn-glycero-3-phosphoethanolamine (200 mg, 0.267 mmol), t-Boc-N-amido-dPEG₁₂-acid (211 mg, 0.294 mmol) and N,N,N',N'-Tetramethyl-O-(7-azabenzotriazol-1-yl)uronium hexafluorophos-phate (122 mg, 0.320 mmol) were dissolved in a chloroform/methanol/H₂O (6 mL, 65:35:8) in a 20 mL scintillation vial flushed with argon. N,N-Diisopropylethylamine (116 *u*L, 0.668 mmol) was added. Reaction was allowed to stir at 25°C for 4 hours and concentrated. Material was then purified via silica gel chromatography with a dichloromethane/methanol gradient to yield (2R)-3-((((2,2-dimethyl-4,44-dioxo-3,8,11,14,17,20,23,26,29,32,35,38,41-tridecaoxa-5,45-diazaheptatetracontan-47-yl)oxy)(hydroxy)phosphoryl)oxy)propane-1,2-diyl distearate as an oil (252 mg, 65%).

Preparation of DSPE-PEG550-VA: (2R)-3-(((((45E,47E,49E,51E)-46,50-dimethyl-4,44-dioxo-52-(2,6,6-trimethylcyclohex-1-en-1-yl)-7,10,13,16,19,22,25,28,31,34,37,40-dodecaoxa-3,43-diazadopentaconta-45,47,49,51-tetraen-1-yl)oxy)(hydroxy)phosphoryl)oxy)propane-1,2-diyl distearate

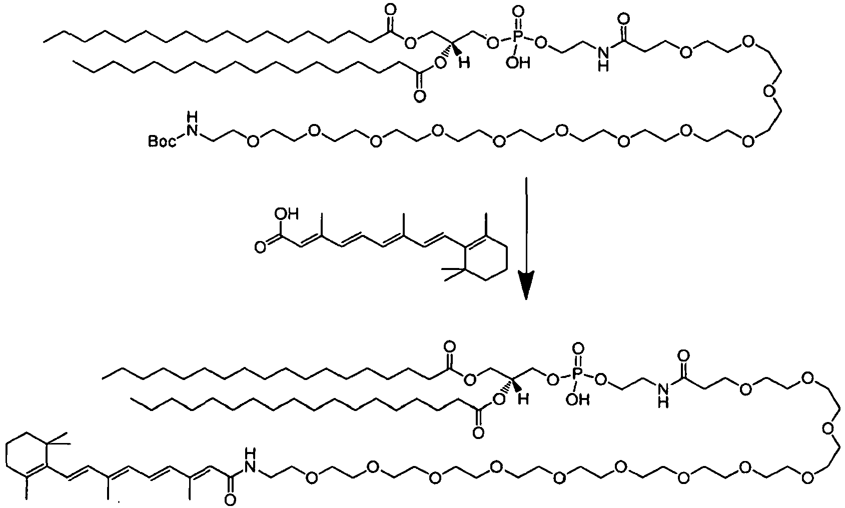

(2R)-3-((((2,2-dimethyl-4,44-dioxo-3,8,11,14,17,20,23,26,29,32,35,38,41-tridecaoxa-5,45-diazaheptatetracontan-47-yl)oxy)(hydroxy)phosphoryl)oxy)propane-1,2-diyl distearate (252 mg, 0.174 mmol) was dissolved in diethyl ether (5 mL). Reaction was placed in a H₂O bath at room temperature. 2 M HCl/diethyl ether (2 mL, 4 mmol) was added and the mixture was allowed to stir for approximately 1 hour. Afterwards, solvent and excess HCl were removed *in vacuo.* Suspended material in 2 mL N,N-Dimethylformamide in a round bottom flask flushed with argon. Retinoic acid (57.5 mg, 0.191 mmol), N,N,N',N'-Tetramethyl-O-(7-azabenzotriazol-1-yl)uronium hexafluorophosphate (79 mg, 0.209 mmol) and N,N-Diisopropylethylamine (106 *u*L, 0.609 mmol) were added. The material did not fully dissolve thus added more chloroform/methanol/H₂O (1 mL, 65:35:8 v:v:v mixture) to get reaction homogeneous. After 3.5 hours, the reaction mixture was concentrated. Material was then purified via silica gel chromatography with a dichloromethane/methanol gradient to yield (2R)-3-(((((45E,47E,49E,51E)-46,50-dimethyl-4,44-dioxo-52-(2,6,6-trimethylcyclohex-1-en-1-yl)-7,10,13,16,19,22,25,28,31,34,37,40-dodecaoxa-3,43-diazadopentaconta-45,47,49,51-tetraen-1-yl)oxy)(hydroxy)phosphoryl)oxy)propane-1,2-diyl distearate as a tan solid (210 mg, 74%). Verified product by NMR & LCMS. ¹H NMR (400 MHz), δ_{H}: 8.6 (s, 1H), 8.25 (d, 1H), 6.8-6.9 (dd, 1H), 6.3-6.4 (m, 1H), 6.12-6.25 (dd, 5H), 5.71 (s, 1H), 5.18 (m, 2H), 4.33 (dd, 2H), 4.13 (m, 2H), 3.95 (m, 2H), 3.74 (m, 8H), 3.63 (s, ∼48H), 3.0 (q, 2H), 2.5 (t, 3H), 2.35 (s, 3H), 2.25 (t, 8H), 1.97 (m, 7H), 1.7 (3, 3H), 1.5 (m, 2H), 1.36 (m, 12H), 1.23 (m, ∼56H), 1.01 (s, 6H), 0.86 (t, 12H). MS: *m*/*z* 1630.28 (M + H⁺).

### Example 17: DSPE-PEG2000-Glu-VA (preparative example)

Preparation of DSPE-PEG2000-Glu-VA

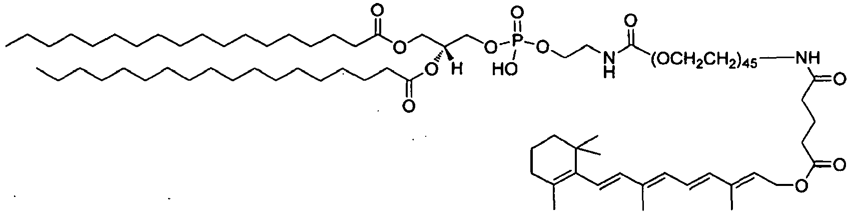

Preparation of Intermediate 1: 5-(((2E,4E,6E,8E)-3,7-dimethyl-9-(2,6,6-trimethylcyclohex-1-en-1-yl)nona-2,4,6,8-tetraen-1-yl)oxy)-5-oxopentanoic acid

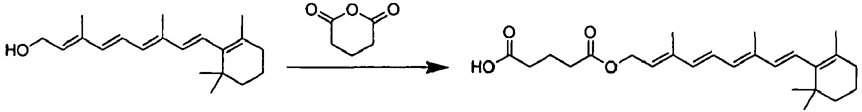

Glutaric anhydride (115 mg, 1.01 mmol) and retinol (240 mg, 0.838 mmol) were dissolved in dichloromethane (3 mL) in an amber-colored vial. Triethylamine (257 *u*l, 1.84 mmol) was added and the vial was flushed with argon. Reaction was allowed to stir at room temperature overnight. The reaction mixture was concentrated arid then purified via silica gel chromatography with a dichloromethane/methanol gradient to yield 5-(((2E,4E,6E,8E)-3,7-dimethyl-9-(2,6,6-trimethylcyclohex-1-en-1-yl)nona-2,4,6,8-tetraen-1-yl)oxy)-5-oxopentanoic acid as a yellowish oil (700 mg, 78%). Material characterized by NMR.

Preparation of DSPE-PEG2000-Glu-VA

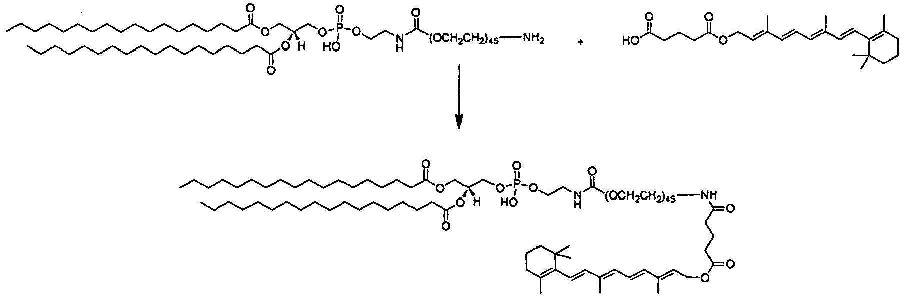

5-(((2E,4E,6E,8E)-3,7-dimethyl-9-(2,6,6-trimethylcyclohex-1-en-1-yl)nona-2,4,6,8-tetraen-1-yl)oxy)-5-oxopentanoic acid (43 mg, 0.108 mmol), DSPE-PEG2000-NH₂ (250 mg, 0.090 mmol) and N,N,N',N'-tetramethyl-O-(7-azabenzotriazol-1-yl)uronium hexafluorophosphate (45 mg, 0.117 mmol) were dissolved in N,N-dimethylformamide (2 mL) in an amber-colored scintillation vial flushed with argon gas. N,N-diisopropylethylamine (47 *u*L, 0.270 mmol) was added and the reaction was allowed to stir overnight at room temperature, then purified via silica gel chromatography with a dichloromethane/methanol gradient to yield yellowish oil (59 mg, 20.7%). Verified product by NMR. ¹H NMR (400 MHz), δ_{H}: 706 (m, 1H), 6.59-6.66 (dd, 1H), 6.06-6.30 (m 5H), 5.56-5.60 (t, 1H), 5.17-5.23 (m, 2H), 4.35-4.42 (dd, 2H), 4.12-4.25 (m, 5H), 3.96-3.97 (m, 6H), 3.79-3.81 (t, 1H), 3.66 (m, ∼180H), 3.51-3.58 (m, 2H), 3.4-3.48 (m, 4H), 3.3-3.38 (m, 2H), 2.25-2.45 (m, 14H), 1.5-2.0 (m, 15H), 1.23-1.32 (m, ∼56H), 1.01 (s, 3H), 0.85-0.88 (t, 12H).

### Example 18: DOPE-Gly₃-VA (preparative example)

Preparation of (Z)-(2R)-3-(((((14E,16E,18E,20E)-15,19-dimethyl-4,7,10,13-tetraoxo-21-(2,6,6-trimethylcyclohex-1-en-1-yl)-3,6,9,12-tetraazahenicosa-14,16,18,20-tetraen-1-yl)oxy)(hydroxy)phosphoryl)oxy)propane-1,2-diyl dioleate (DOPE-Gly₃-VA)

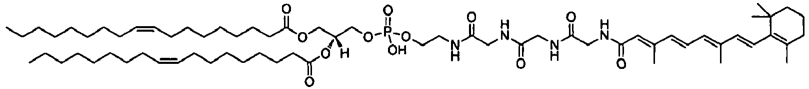

Preparation of Intermediate 1: (Z)-(2R)-3-(((2-(2-(2-(2-aminoacetamido)acetamido)acetamido)ethoxy)(hydroxy)phosphoryl)oxy)propane-1,2-diyl dioleate

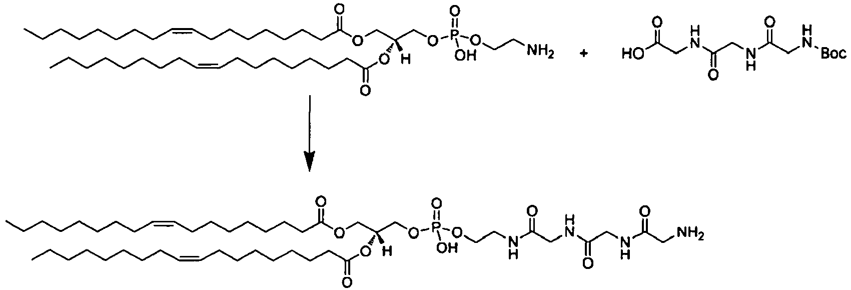

Boc-Gly-Gly-Gly-OH (382 mg, 1.34 mmol) and N,N,N',N'-Tetramethyl-O-(7-azabenzotriazol-1-yl)uronium hexafluorophosphate (532 mg, 1.4 mmol) were dissolved in DMF (5 mL). N,N-Diisopropylethylamine (488 *u*L, 2.8 mmol) was added and the mixture was allowed to stir at room temperature for 10-15 minutes. Afterwards, a solution of 1,2-dioleoyl-sn-glycero-3-phosphoethanolamine (833 mg, 1.12 mmol) in chloroform (5 mL) was added and the reaction vessel was flushed with argon. After 16 hours at room temperature, the reaction mixture was concentrated and partitioned between dichloromethane (50 mL) and H₂O (50 mL), extracted with dichloromethane (3 x 50 mL), dried with sodium sulfate, filtered and concentrated. Material was purified via silica gel chromatography using a dichloromethane/methanol gradient to yield colorless oil residue. To this, 2 M HCl/Diethyl Ether (5 mL) was added and the reaction mixture was allowed to stir in a H₂O bath for approximately 2 hours. The reaction mixture was concentrated and the residue was taken up in dichloromethane (75 mL), washed with saturated sodium bicarbonate solution (75 mL), extracted product with dichloromethane (3x75 mL), dried with sodium sulfate, filtered and concentrated to yield (Z)-(2R)-3-(((2-(2-(2-(2-aminoacetamido)acetamido)acetamido)ethoxy)(hydroxy)phosphoryl)oxy)propane-1,2-diyl dioleate as a semi-solid (765 mg, 90%). Verified by NMR.

Preparation of DOPE-Gly3-VA: (Z)-(2R)-3-(((((14E,16E,18E,20E)-15,19-dimethyl-4,7,10,13-tetraoxo-21-(2,6,6-trimethylcyclohex-1-en-1-yl)-3,6,9,12-tetraazahenicosa-14,16,18,20-tetraen-1-yl)oxy)(hydroxy)phosphoryl)oxy)propane-1,2-diyl dioleate

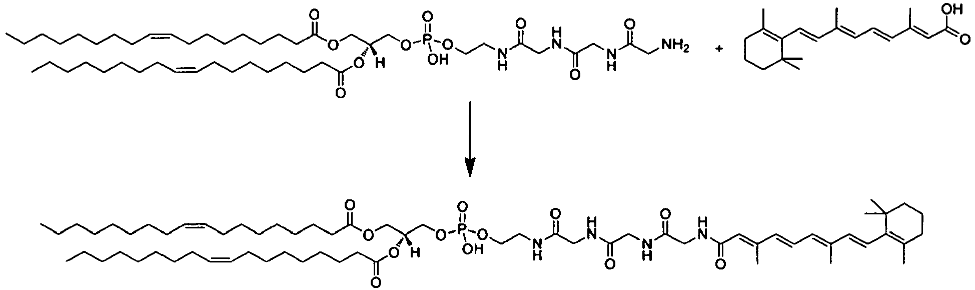

(Z)-(2R)-3-(((2-(2-(2-(2-aminoacetamido)acetamido)acetamido)ethoxy)(hydroxy)phosphoryl)oxy)propane-1,2-diyl dioleate (765 mg, 0.836 mmol), retinoic acid (301 mg, 1.00 mmol), and N,N,N',N'-Tetramethyl-0-(7-azabenzotriazol-1-yl)uronium hexafluorophosphate (413 mg, 1.09 mmol) were suspended in N,N-Dimethylformamide (5 mL). N,N-Diisopropylethylamine (437*u*L, 2.51 mmol) was added and the reaction vessel was flushed with argon gas. Added chloroform (5 mL) to aid in the solvation of materials. Reaction was allowed to stir for ∼4 hours at room temperature in a round bottom flask covered with aluminum foil. Partitioned material between H₂O (100 mL) and dichloromethane (100 mL). Extracted with dichloromethane (3 x 100 mL), dried with sodium sulfate, filtered and concentrated. Material was then purified via silica gel chromatography using a dichloromethane/methanol gradient to yield (Z)-(2R)-3-(((((14E,16E,18E,20E)-15,19-dimethyl-4,7,10,13-tetraoxo-21-(2,6,6-trimethylcyclohex-1-en-1-yl)-3,6,9,12-tetraazahenicosa-14,16,18,20-tetraen-1-yl)oxy)(hydroxy)phosphoryl)oxy)propane-1,2-diyl dioleate as an orange oil (704 mg, 70%). Verified product by LCMS & NMR. ¹H NMR (400 MHz), δ_{H}: 6.90 (t, 1H), 6.21 (q, 2H), 6.08-6.12 (d, 2H), 5.83 (s, 1H), 5.31 (s, 4H), 5.30 (s, 2H), 4.37 (d, 1H), 4.15 (m, 1H), 3.91 (m, 8H), 3.59 (m, 2H), 3.29 (m, 2H), 3.01 (m, 2H), 2.28 (m, 6H), 1.95-1.98 (m, 12H), 1.44 (s, 3H), 1.5-1.6 (m, 2H), 1.44 (m, 6H), 1.24 (m, ∼48H), 1.00 (s, 6H), 0.86 (t, 3H). MS: *m*/*z* 1198.42 (M + H⁺).

### Example 19: VA-PEG-VA (preparative example)

Preparation of N1,N19-bis((16E,18E,20E,22E)-17,21-dimethyl-15-oxo-23-(2,6,6-trimethylcyclohex-1-en-1-yl)-4,7,10-trioxa-14-azatricosa-16,18,20,22-tetraen-1-yl)-4,7,10,13,16-pentaoxanonadecane-1,19-diamide (VA-PEG-VA)

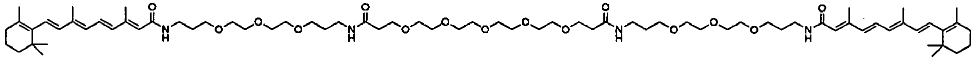

Preparation of VA-PEG-VA: N1,N19-bis((16E,18E,20E,22E)-17,21-dimethyl-15-oxo-23-(2,6,6-trimethylcyclohex-1-en-1-yl)-4,7,10-trioxa-14-azatricosa-16,18,20,22-tetraen-1-yl)-4,7,10,13,16-pentaoxanonadecane-1,19-diamide

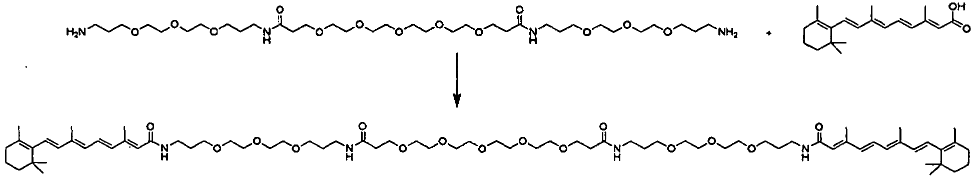

Retinoic acid (2913 mg, 9.70 mmol), N,N,N',N'-Tetramethyl-O-(7-azabenzotriazol-1-yl)uronium hexafluorophosphate (3992mg, 10.50 mmol) and diamido-dPEG₁₁-diamine (3000 mg, 4.04 mmol) were suspended in N,N-dimethylformamide (10 mL). N,N-Diisopropylethylamine (4222 µL, 24.24 mmol) was added and the vessel was flushed with argon. Reaction was allowed to stir at room temperature overnight in a round bottom flask covered with aluminum foil. Next day, partitioned material between ethyl acetate (125 mL) and water (125 mL). Extracted with ethyl acetate (3x125 mL), dried with sodium sulfate, filtered and concentrated. Material was then purified via silica gel chromatography with a dichloromethane/methanol gradient. Pooled fractions and concentrated to yield yellow oil (2900 mg, 54.9%). Verified product by LCMS & NMR. ¹H NMR (400 MHz), δ_{H}: 7.1 (s, 2H), 6.87 (t, 2H), 6.51 (t, 2H), 6.12-6.20 (dd, 8H), 5.66 (s, 2H), 3.6-3.8 (m, ∼44H), 3.4 (q, 4H), 3.3 (q, 4H), 2.46 (t, 4H), 2.32 (s, 6H), 1.9-2.05 (m, 10H), 1.7-1.85 (m, 15H), 1.6 (m, 4H), 1.3-1,5 (m, 6H), 1.01 (s, 12H). QTOF MS: *m*/*z* 1306 (M + H⁺).

### Example 20: VA-PEG2000-VA (preparative example)

Preparation of (2E,2'E,4E,4'E,6E,6'E,8E,8'E)-N,N'-(3,6,9,12,15,18,21,24,27,30,33,36,39,42,45,48,51,54,57,60,63,66,69,72,75,78,81,84,87,90,93,96, 99,102,105,108,111,114,117,120,123,126,129,132,135,138-hexatetracontaoxatetracontahectane-1,140-diyl)bis(3,7-dimethyl-9-(2,6,6-trimethylcyclohex-1-en-1-yl)nona-2,4,6,8-tetraenamide) (VA-PEG2000-VA)

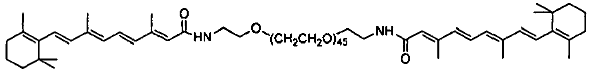

Preparation of VA-PEG2000-VA: (2E,2'E,4E,4'E,6E,6'E,8E,8'E)-N,N'-(3,6,9,12,15,18,21,24,27,30,33,36,39,42,45,48,51,54,57,60,63,66,69,72,75,78,81,84,87,90,93,96, 99,102,105,108,111,114,117,120,123,126,129,132,135,138-hexatetracontaoxatetracontahectane-1,140-diyl)bis(3,7-dimethyl-9-(2,6,6-trimethylcyclohex-1-en-1-yl)nona-2,4,6,8-tetraenamide)

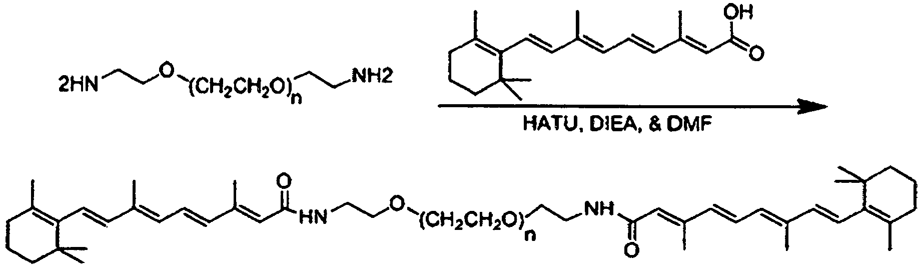

Retinoic acid (109mg, 0.362 mmol), N,N,N',N'-Tetramethyl-O-(7-azabenzotriazol-1-yl)uronium hexafluorophosphate (149 mg, 0.392 mmol) and amine-PEG_{2K}-amine (333 mg, 0.151 mmol) were suspended in N,N-Dimethylformamide (3 mL). N,N-Diisopropylethylamine (158 µL, 0.906 mmol) was added and the vessel was flushed with argon. Reaction was allowed to stir at room temperature overnight in a round bottom flask covered with aluminum foil. Next day, partitioned material between ethyl acetate (30 mL) and water (30 mL). Extracted with ethyl acetate (3x30 mL), dried with sodium sulfate, filtered and concentrated. Material was then purified via silica gel chromatography with a dichloromethane/methanol gradient. Pooled fractions and concentrated to yield (2E,2'E,4E,4'E,6E,6'E,8E,8'E)-N,N'-(3,6,9,12,15,18,21,24,27,30,33,36,39,42,45,48,51,54,57,60,63,66,69,72,75,78,81,84,87,90,93,96, 99,102,105,108,111,114,117,120,123,126,129,132,135,138-hexatetracontaoxatetracontahectane-1,140-diyl)bis(3,7-dimethyl-9-(2,6,6-trimethylcyclohex-1-en-1-yl)nona-2,4,6,8-tetraenamide) as a yellow oil (97 mg, 23%). Verified product by LCMS & NMR. ¹H NMR (400 MHz), δ_{H}: 6.85-6.92 (t, 2h), 6.20-6.32 (M, 6H), 6.08-6.12 (d, 4H), 5.72 (s, 2H), 3.55-3.70 (m, ∼180H), 3.4-3.5 (m, 4H), 2.79 (m, 4H), 2.78 (s, 6H), 2.33 (s, 6H), 2.05 (m, 4H), 1.97 (s, 6H), 1.80 (m, 2H), 1.79 (s, 6H), 1.69 (s, 6H), 1.60 (m, 4H), 1.45 (m, 4H), 1.01 (s, 12H). QTOF MS: *m*/*z* 2651 (M + H⁺)..

### Example 21: DSPE-PEG2000-VA (preparative example)

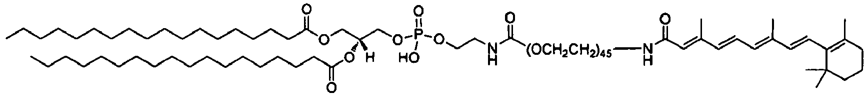

Preparation of DSPE-PEG2000-VA

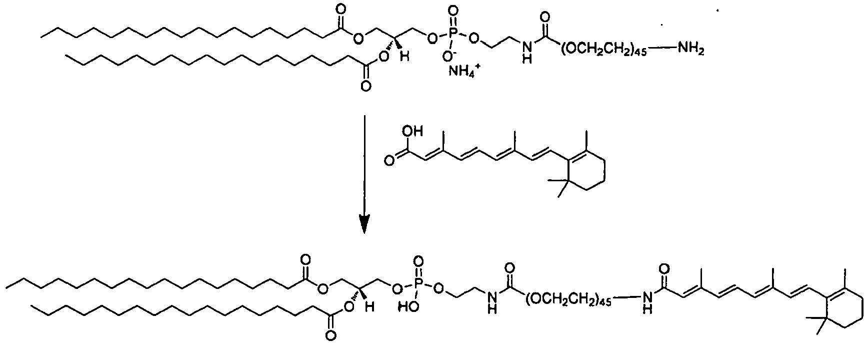

DSPE-PEG2000-NH₂ (250 mg, 0.090 mmol), retinoic acid (3 3 mg, 0.108 mmol) and N,N,N',N'-Tetramethyl-O-(7-azabenzotriazol-1-yl)uronium hexafluorophosphate (45 mg, 0.117 mmol) were dissolved in N,N-Dimethylformamide. N,N-Diisopropylethylamine (47 µL, 0.270 mmol) was added to the mixture. The amber colored scintillation vial was flushed with argon and allowed to stir 3 days at room temperature. Material was then purified silica gel chromatography using a dichloromethane/methanol gradient. Pooled fractions and concentrated to yield DSPE-PEG2000-VA as a yellow oil (245 mg, 89%). Verified product by NMR. ¹H NMR (400 MHz), δ_{H}: 6.86 (dd, 1H), 6.25 (m, 1H), 6.09-6.21 (dd, 4H), 5.71 (s, 1H), 5.1-5.2 (m, 1H), 4.3-4.4 (d, 1H), 4.1-4.2 (m, 3H), 3.85-4.0 (m, 4H), 3.8 (t, 1H), 3.5-3.75 (m, ∼180H), 3.4-3.5 (m, 8H), 3.3 (m, 2H), 2.35 (s, 3H), 2.26 (m, 4H), 1.70 (s, 3H), 1.55-1.65 (m, 6H), 1.47 (m, 2H), 1.23 (s, ∼60H), 1.01 (s; 6H), 0.85 (t, 6H).

### Example 22: diVA-PEG-diVA, also known as "DiVA" (preparative example)

Preparation of N1,N19-bis((S,23E,25E,27E,29E)-16-((2E,4E,6E,8E)-3,7-dimethyl-9-(2,6,6-trimethylcyclo-hex-1-en-1-yl)nona-2,4,6,8-tetraenamido)-24,28-dimethyl-15,22-dioxo-30-(2,6,6-trimethylcyclohex-1-en-1-yl)-4,7,10-trioxa-14,21-diazatriaconta-23,25,27,29-tetraen-1-yl)-4,7,10,13,16-pentaoxanonadecane-1,19-diamide (diVA)

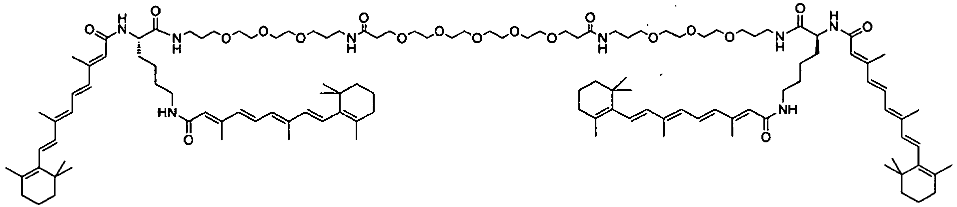

Preparation of Intermediate 1: tetrabenzyl ((5S,57S)-6,22,40,56-tetraoxo-11,14,17,25,28,31,34,37, 45,48,51-undecaoxa-7,21,41,55-tetraazahenhexacontane-1,5,57,61-tetrayl) tetracarbamate, also known as Z-DiVA-PEG-DiVA-IN

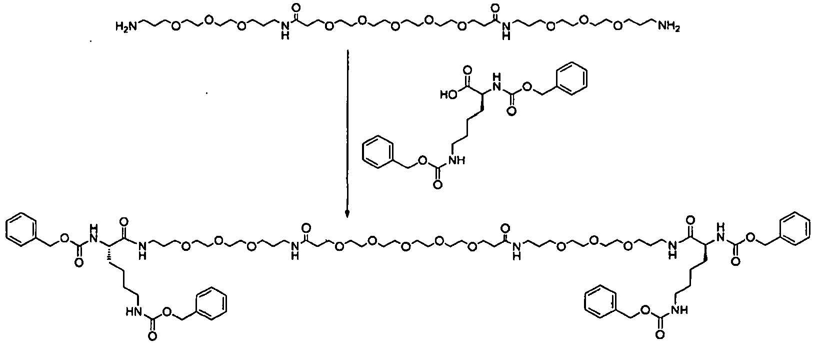

AIL reaction flask cooled to 5 - 10°C was purged with nitrogen and charged with dichloromethane (300 mL), d-PEG-11-diamine (Quanta lot EK1-A-1100-010, 50.0 g, 0.067 mol), Z-(L)-Lys(Z)-OH (61.5 g, 0.15 mol), and HOBt hydrate (22.5 g, 0.15 mol). 4-Methylmorpholine (4-MMP) (15.0 g, 0.15mol) was added to the suspension and a light exothermic reaction was observed. A suspension of EDC hydrochloride (43.5 g, 0.23 mol) and 4-MMP (20.0 g, 0.20 mol) in dichloromethane (150 mL) was added over a period of 30 minutes, and moderate cooling was required in order to maintain a temperature of 20-23°C. The slightly turbid solution was stirred overnight at ambient temperature, and HPLC indicates completion of reaction. Deionized water (300 mL) was added and after having stirred for 10 minutes, a quick phase separation was observed. The aqueous phase was extracted with dichloromethane (150 mL) - with a somewhat slower phase separation. The combined organic extracts are washed with 6% sodium bicarbonate (300 mL) and dried with magnesium sulphate (24 g). Evaporation from a 40-45°C water bath under reduced pressure gives 132 g of crude product. A solution of crude product (131 g) in 8% methanol in ethyl acetate in loaded onto a column of Silica Gel 60 (40-63µ), packed with 8% methanol in ethyl acetate. The column was eluted with 8% methanol in ethyl acetate (7.5 L). The fractions containing sufficiently pure product (5.00-7.25 L) was evaporated from a 45°C water bath under reduced pressure and 83.6 g of purified product. A solution of purified product (83.6 g) in dichloromethane (200 mL) was loaded onto a column of Dowex 650 C (H⁺) (200 g), which has been washed with dichloromethane (250 mL). The column was eluted with dichloromethane (200 mL). The combined product containing fractions (300-400 mL) were dried with magnesium sulphate (14 g) and evaporated from a 45°C water bath under reduced pressure to yield tetrabenzyl ((5S,57S)-6,22,40,56-tetraoxo-11,14,17,25,28,31,34,37,45,48,51-undecaoxa-7,21,41,55-tetraazahenhexacontane-1,5,57,61-tetrayl)tetracarbamate, also known as Z-DiVA-PEG-DiVA-IN (77.9 g, HPLC purity 94.1%).

Preparation of Intermediate 2: N1,N19-bis((S)-16,20-diamino-15-oxo-4,7,10-trioxa-14-azaicosyl)-4,7,10,13,16-pentaoxanonadecane-1,19-diamide, also known as DiVA-PEG-DiVA-IN

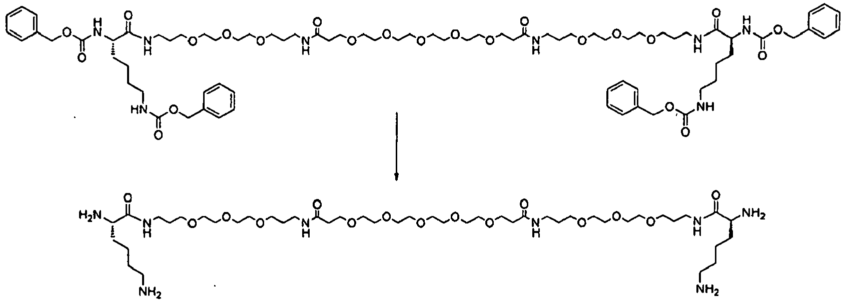

A 1 L reaction flask was purged with nitrogen and charged with methanol (600 mL) and Z-DiVA-PEG-DiVA-IN (92.9, 60.5 mmol). The mixture was stirred under nitrogen until a solution was obtained. The catalyst, 10% Pd/C/50%water (Aldrich, 10 g) was added. The mixture was evacuated, and then the pressure was equalized by nitrogen. The mixture was evacuated, and then the pressure was equalized by hydrogen. Ensuring a steady, low flow of hydrogen over the reaction mixture, the stirrer was started. Hydrogenation was continued in a flow of hydrogen for one hour. The system was then closed, and hydrogenation was continued at ∼0.1bar for one hour. The mixture was evacuated and then re-pressurized to ∼0.1 bar with hydrogen. After another hour of hydrogenation, the mixture was evacuated and then re-pressurized to 0.1bar with hydrogen. Stirring under hydrogen was continued for 15 hours after which time no starting material could be detected by HPLC. The mixture was evacuated, and then the pressure was equalized by nitrogen. The mixture was evacuated, and then the pressure was equalized by nitrogen. The reaction mixture was then filtered on a pad of celite 545. The filter cake was washed with methanol (100 mL). The combined filtrate was concentrated, finally at 45°C and at a pressure of less than 50 mbar. Toluene (100 mL) was added and the resulting mixture was again concentrated finally at 45°C and at a pressure of less than 40 mbar to yield N1,N19-bis((S)-16,20-diamino-15-oxo-4,7,10-trioxa-14-azaicosyl)-4,7,10,13,16-pentaoxanonadecane-1,19-diamide, also known as DiVA-PEG-DiVA-IN (63.4 g), as an oil that solidifies upon standing.

Preparation of DiVA-PEG-DiVA: N1,N19-bis((S,23E,25E,27E,29E)-16-((2E,4E,6E,8E)-3,7-dimethyl-9-(2,6,6-trimethylcyclohex-1-en-1-yl)nona-2,4,6,8-tetraenamido)-24,28-dimethyl-15,22-dioxo-30-(2,6,6-tri-methylcyclohex-1-en-1-yl)-4,7,10-trioxa-14,21-diazatriaconta-23,25,27,29-tetraen-1-yl)-4,7,10,13,16-pentaoxanonadecane-1,19-diamide

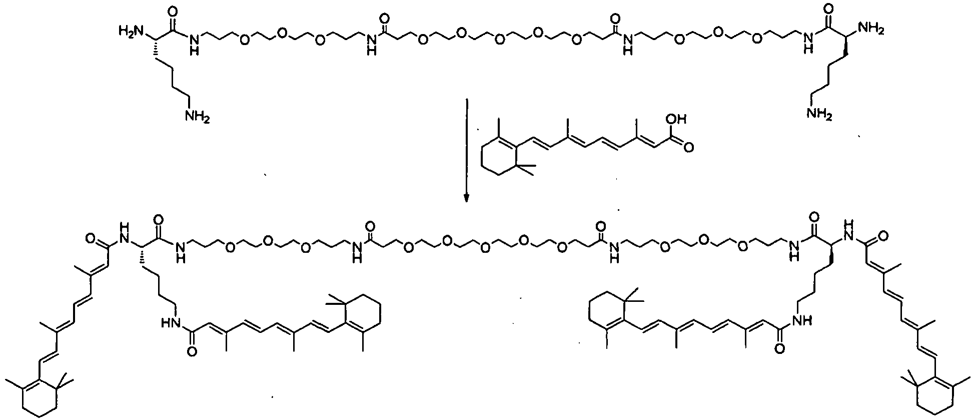

A 2 L reactor was filled with argon and charged with dichloromethane (500 mL), DiVA-PEG-DiVA-IN (52.3 g, 52.3 mmol), retinoic acid (70.6 g, 235 mmol) and 4-N,N-dimethylaminopyridine (2.6 g, 21.3 mmol). The mixture was stirred under argon until dissolved (∼20 minutes). Keeping the temperature of the reaction at 10 - 20°C, 1-ethyl-3-(3-dimethylaminopropyl) carbodiimide) (EDCI) (70.6 g, 369 mmol) was added portion wise over a period of 10 - 15 minutes (the reaction was slightly exothermic for the first 30 - 60 minutes). The reactor was covered with aluminium foil and the mixture was stirred at 18 - 21°C for 15-20 hours. Butylated hydroxytoluene (BHT) (25 mg) was added and the reaction mixture was then poured onto aqueous 6% sodium hydrogen carbonate (500 mL) while keeping an argon atmosphere over the mixture. The organic phase was separated. The aqueous phase was washed with dichloromethane (50 mL). The combined organic phase was dried with of magnesium sulphate (150 g) under inert atmosphere and protected from light. The drying agent was filtered off (pressure filter preferred) and the filter cake was washed with dichloromethane (500 mL). The filtrate was concentrated by evaporation at reduced pressure using a water bath of 35-40°C. The oily residue was added toluene (150 mL) and evaporated again to yield a semi-solid residue of 210 g. This residue was dissolved in dichloromethane (250 mL) and applied onto a column prepared from silica gel 60 (1.6 kg) and 0.5% methanol in dichloromethane) (4 L). The column was eluted with dichloromethane (7.2 L), 2), 3% methanol in dichloromethane (13 L), 5% methanol in dichloromethane (13 L), 10% methanol in dichloromethane (18 L). One 10 L fraction was taken, and then 2.5 L fractions were taken. The fractions, protected from light were sampled, flushed with argon and sealed. The fractions taken were analyzed by TLC (10% methanol in dichloromethane, UV). Fractions holding DiVA-PEG-DiVA were further analyzed by HPLC. 5 Fractions < 85% pure (gave 32 g of evaporation residue) were re-purified in the same manner, using only 25% of the original amounts of silica gel and solvents. The fractions >85% pure by HPLC were combined and evaporated at reduced pressure, using a water bath of 35 - 40°C. The evaporation residue (120 g) was re-dissolved in dichloromethane (1.5 L) and slowly passed (approximately 1 hour) through a column prepared from ion exchanger Dowex 650C, H⁺ form (107 g). The column was then washed with dichloromethane (1 L). The combined eluate (3277.4 g) was mixed well and a sample (25 mL, 33.33 g) was evaporated, finally atroom temperature and a pressure of < 0.1 mBar to afford 0.83 g of a foam. From this figure the total amount of solid material was thus calculated to a yield of 80.8 g (72.5%). The remaining 3.24 kg of solution was concentrated to 423 g. 266 g of this solution was concentrated further to yield a syrup and then re-dissolved in abs. ethanol (200 mL). Evaporation at reduced pressure, using a water bath of 35 - 40°C, was continued to yield a final ethanol solution of 94.8 g holding 50.8 g (53.6% w/w) of N1,N19-bis((S,23E,25E,27E,29E)-16-((2E,4E,6E,8E)-3,7-dimethyl-9-(2,6,6-trimethylcyclohex-1-en-1-yl)nona-2,4,6,8-tetraenamido)-24,28-dimethyl-15,22-dioxo-30-(2,6,6-trimethylcyclohex-1-en-1-yl)-4,7,10-trioxa-14,21-diazatriaconta-23,25,27,29-tetraen-1-yl)-4,7,10,13,16-pentaoxanonadecane-1,19-diamide, also known as DiVA-PEG-DiVA, also known as "DiVA". Characterized by NMR & QTOF. ¹H NMR (400 MHz), δ_{H}: 7.07 (t, 2H), 7.01 (t, 2H), 6.87-6.91 (m, 4.0H), 6.20-6.24 (m, 10H), 6.10-6.13 (m, 8H), 5.79 (s, 2H), 5.71 (s, 2H), 4.4 (q, 2H), 3.70 (t, 6H), 3.55-3.65 (m, ∼34H), 3.59 (t, 6H), 3.4 (m, 2H), 3.25-3.33 (m, 10H), 3.16 (m, 2H), 2.44 (t, 4H), 2.33 (s, 12H), 1.97-2.01 (m, 12H), 1.96 (s, 6H), 1.7-1.9 (m, 12H), 1.69 (s, 12H), 1.5-1.65 (m, 12H), 1.35-1.5(m, 24H), 1.01 (s, 24H). QTOF MS ESI+: *m*/*z* 2128 (M + H⁺).

### Example 23: DOPE-VA (preparative example)

Prepartion of (Z)-(2R)-3-(((2-((2E,4E,6E,8E)-3,7-dimethyl-9-(2,6,6-trimethylcyclohex-1-en-1-yl)nona-2,4,6,8-tetraenamido)ethoxy)(hydroxy)phosphoryl)oxy)propane-1,2-diyl dioleate (DOPE-VA)

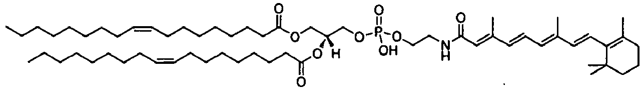

Preparation of DOPE-VA: (Z)-(2R)-3-(((2-((2E,4E,6E,8E)-3,7-dimethyl-9-(2,6,6-trimethylcyclohex-1-en-1-yl)nona-2,4,6,8-tetraenamido)ethoxy)(hydroxy)phosphoryl)oxy)propane-1,2-diyl dioleate

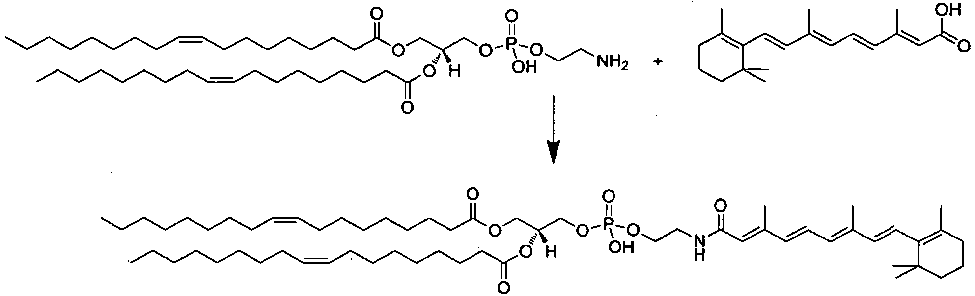

To a solution of retinoic acid (250 mg, 0.83 mmol) in diethyl ether stirring (20 mL) at -78° C, a solution of (diethylamino)sulfur trifluoride (130 µl, 0.90 mmol) in cold ether (20 mL) was added through a syringe. The reaction mixture was taken out of the cold bath and the stirring was continued at room temperature for an additional 2 hr. At the end, the solvent was removed by rotary evaporation. The residue was redissolved chloroform (50 mL) in the presence of solid Na₂CO₃(50 mg). To this solution was added 1,2-dioleoyl-sn-glycero-3-phosphoethanolamine (600mg, 0.81 mmol) and the reaction mixture was stirred at room temperature for an additional 24 hrs. The solvent was removed by rotary evaporation. The residue was purified by silica gel chromatography with a dichloromethane/methanol gradient to yield Z)-(2R)-3-(((2-((2E,4E,6E,8E)-3,7-dimethyl-9-(2,6,6-trimethylcyclohex-1-en-1-yl)nona-2,4,6,8-tetraenamido)ethoxy)(hydroxy)phosphoryl)oxy)propane-1,2-diyl dioleate (240 mg, 28%). 1H NMR (400 MHz, CDCl₃) δ 0.87 (t, 6H, CH₃), 1.01 (s, 6H, CH₃) 1.20-1.40 (m, 40H, CH₂), 1.40-1.60 (m, 8H, CH₂), 1.70 (s, 3H, CH₃-C=C), 1.80-2.10 (m, 8H), 2.32 (m, 4H, CH₂C(=O)), 3.50 (m, 2H), 3.92-4.18 (m, 5H), 4.35 (m, 2H), 5.20 (m, 1H, NHC(=O)), 5.31 (m, 4H, CH=CH), 5.80-6.90 (m, 6H, CH=CH).

### Example 24: DC-VA (preparative example)

Preparation of (((2E,4E,6E,8E)-3,7-dimethyl-9-(2,6,6-trimethylcyclohex-1-en-1-yl)nona-2,4,6,8-tetraenoyl)azanediyl)bis(ethane-2,1-diyl) ditetradecanoate (DC-VA)

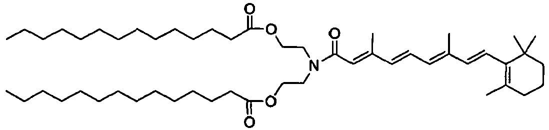

Preparation of DC-VA: (((2E,4E,6E,8E)-3,7-dimethyl-9-(2,6,6-trimethylcyclohex-1-en-1-yl)nona-2,4,6,8-tetraenoyl)azanediyl)bis(ethane-2,1-diyl) ditetradecanoate

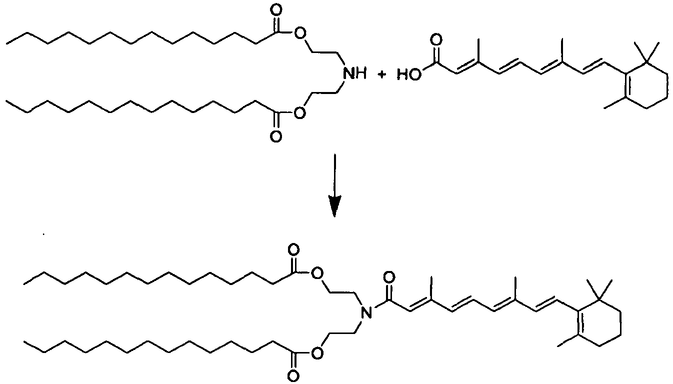

To a solution of retinoic acid (600 mg, 2.0 mmol) in diethyl ether (25 mL) stirring at -78° C, a solution of (diethylamino)sulfur trifluoride (0.3 ml, 2.1 mmol) in 5 mL of cold ether was added through a syringe. The reaction mixture was taken out of the cold bath and the stirring was continued at room temperature for an additional 1 hr. After the solvent was removed by rotary evaporation, the residue was re-dissolved in dichloromethane (20 mL) in the presence of 2 solid Na₂CO₃ (25 mg). To this solution was added the azanediylbis(ethane-2,1-diyl) ditetradecanoate (1.05 g, 2.0 mmol), and the reaction mixture was stirred at room temperature for an additional 24 hrs. The reaction mixture was diluted with dichloromethane (50mL) and was dried over MgSO₄. After the solvent was removed by rotary evaporation, the residue was purified by silica gel chromatography with a dichloromethane/methanol gradient to yield (((2E,4E,6E,8E)-3,7-dimethyl-9-(2,6,6-trimethylcyclohex-1-en-1-yl)nona-2,4,6,8-tetraenoyl)azanediyl)bis(ethane-2,1-diyl) ditetradecanoate (800 mg, 50%). 1H NMR (400 MHz, CDCl₃) δ 0.87 (t, 6H, CH₃), 1.02 (s, 6H, CH₃) 1.20-1.40 (m, 40H, CH₂), 1.40-1.60 (m, 8H, CH₂), 1.70 (s, 3H, CH₃-C=C), 1.97(s, 3H, CH₃-C=C), 2.05 (m, 2H, CH₂), 2.15(s, 3H, CH₃-C=C), 2.32 (m, 4H, C**H**₂C(=O)), 3.67 (m, 4H, NC**H**₂CH₂O), 4.15-4.30 (m, 4H, NCH₂C**H**₂O), 5.80-6.90 (m, 6H, CH=CH).

### Example 25: DC-6-VA (preparative example)

Preparation of ((6-((2E,4E,6E,8E)-3,7-dimethyl-9-(2,6,6-trimethylcyclohex-1-en-1-yl)nona-2,4,6,8-tetraenamido)hexanoyl)azanediyl)bis(ethane-2,1-diyl) ditetradecanoate (DC-6-VA)

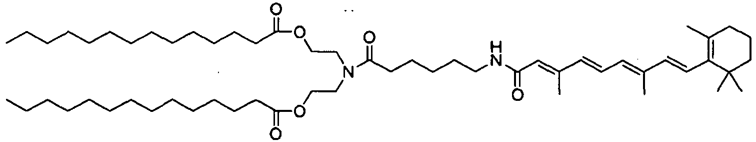

Preparation of Intermediate 1: ((6-aminohexanoyl)azanediyl)bis(ethane-2,1-diyl) ditetradecanoate TFA salt

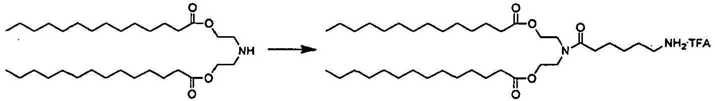

A mixture of azanediylbis(ethane-2,1-diyl) ditetradecanoate (2.5 g, 4.8 mmol), Boc-amino caproic acid (1.3 g, 5.6 mmol), N,N'-dicyclohexylcarbodiimide (1.3 g, 6.3 mmol) and N,N-diisopropylethylamine (2.6 mL, 0.015 mmol) were dissolved in pyridine (40 mL). The solution was stirred at 60° C for overnight. The mixture was diluted with dichloromethane (50 mL) and washed with saline (3x50 mL). After being concentrated by rotary evaporation, the residue was treated with trifluoroacetic acid/dichloromethane (100mL, 1:1). The mixture was concentrated and was re-dissolved in dichloromethane (50 mL) and washed with saline (3x50 mL). The organic layer was isolated and concentrated to yield ((6-aminohexanoyl)azanediyl)bis(ethane-2,1-diyl) ditetradecanoate TFA salt (1.5 g, 33%).

Preparation of DC-6-VA: ((6-((2E,4E,6E,8E)-3,7-dimethyl-9-(2,6,6-trimethylcyclohex-1-en-1-yl)nona-2,4,6,8-tetraenamido)hexanoyl)azanediyl)bis(ethane-2,1-diyl)

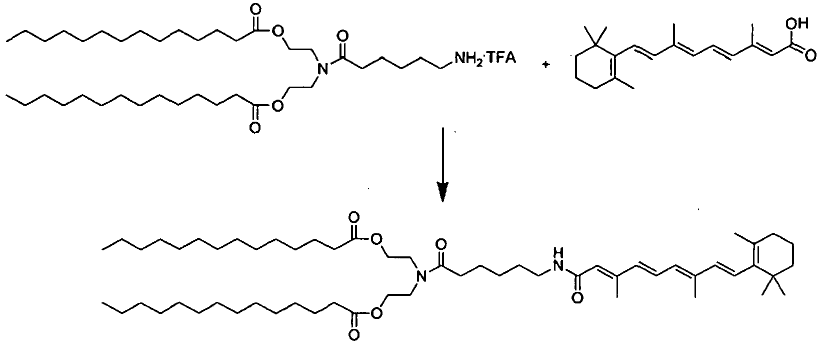

To a solution of retinoic acid (800 mg, 2.67 mmol) in diethyl ether (40 mL) stirring at -78°C, a solution of (diethylamino)sulfur trifluoride (0.4 mL, 22.80 mmol) in cold ether (7 mL) was added through a syringe. The reaction mixture was taken out of the cold bath and the stirring was continued at room temperature for an additional 1 hr. After the solvent was removed by rotary evaporation, the residue was re-dissolved in dichloromethane (25 mL) in the presence of solid Na₂CO₃ (40 mg). To this solution was added the ((6-aminohexanoyl)azanediyl)bis(ethane-2,1-diyl) ditetradecanoate TFA salt (1.5 g, 1.6 mmol) and the reaction mixture was stirred at room temperature for an additional 24 hrs. The reaction mixture was diluted with dichloromethane (50 mL) and dried over MgSO₄. After the solvent was removed by rotary evaporation, the residue was purified by column chromatography using 5% methanol/dichloromethane as eluent to yield ((6-((2E,4E,6E,8E)-3,7-dimethyl-9-(2,6,6-trimethylcyclohex-1-en-1-yl)nona-2,4,6,8-tetraenamido)hexanoyl)azanediyl)bis(ethane-2,1-diyl) (360 mg, 24%). 1H NMR (400 MHz, CDCl₃) δ 0.87 (t, 6H, CH₃), 1.02 (s, 6H, CH₃) 1.20-1.40 (m, 42H, CH₂), 1.40-1.60 (m, 12H, CH₂), 1.70 (s, 3H, CH₃-C=C), 1.97(s, 3H, CH₃-C=C), 2.05 (m, 2H, CH₂), 2.15(s, 3H, CH₃-C=C), 2.32 (m, 6H, C**H**₂C(=O)), 3.20 (m, 2H, C**H**₂NHC(=O)), 3.56 (m, 4H, NC**H**₂CH₂O), 4.15-4.30 (m, 4H, NCH₂C**H**₂O), 5.10 (m, 1H), 5.80-6.90 (m, 6H, CH=CH).

### Example 26: SYNTHESIS OF satDiVA (preparative example)

Preparation of N1,N19-bis((16S)-16-(3,7-dimethyl-9-(2,6,6-trimethylcyclohex-1-en-1-yl)nonanamido)-24,28-dimethyl-15,22-dioxo-30-(2,6,6-trimethylcyclohex-1-en-1-yl)-4,7,10-trioxa-14,21-diazatriacontyl)-4,7,10,13,16-pentaoxanonadecane-1,19-diamide (satDIVA).

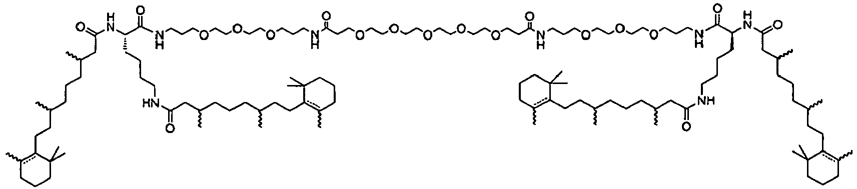

Preparation of Intermediate 1: 3,7-dimethyl-9-(2,6,6-trimethylcyclohex-1-en-1-yl)nonanoic acid

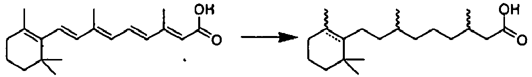

All*-trans* retinoic acid (2000 mg, 6.66 mmol) was dissolved in hexanes/IPA (3:1, 40mL) with the aid of sonication. Material was placed in a Parr-shaker bottle and flushed with inert gas. 10% Pd/C (200mg) was added and the vessel was once again flushed with inert gas. Material was placed on the Parr-Shaker overnight with >70 psi Hydrogen gas. The reaction mixture was then filtered through a pad of celite and concentrated to yield 3,7-dimethyl-9-(2,6,6-trimethylcyclohex-1-en-1-yl)nonanoic acid (2 g).

Preparation of satDIVA: N1,N19-bis((16S)-16-(3,7-dimethyl-9-(2,6,6-trimethylcyclohex-1-en-1-yl)nonanamido)-24,28-dimethyl-15,22-dioxo-30-(2,6,6-trimethylcyclohex-1-en-1-yl)-4,7,10-trioxa-14,21-diazatriacontyl)-4,7,10,13,16-pentaoxanonadecane-1,19-diamide

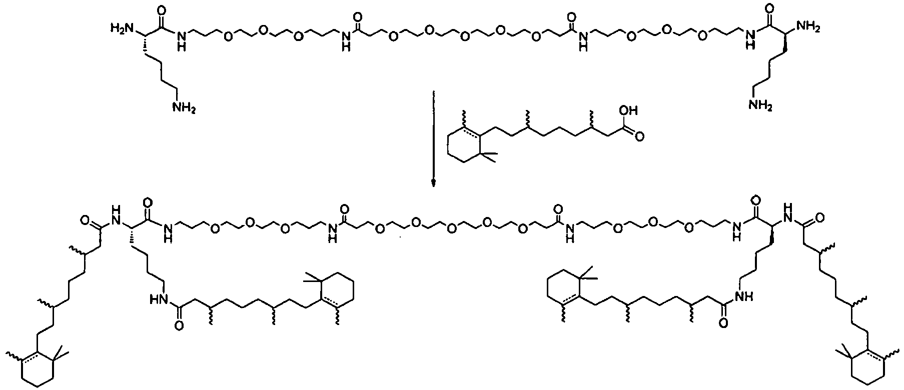

N1,N19-bis((16S)-16-(3,7-dimethyl-9-(2,6,6-trimethylcyclohex-1-en-1-yl)nonanamido)-24,28-dimethyl-15,22-dioxo-30-(2,6,6-trimethylcyclohex-1-en-1-yl)-4,7,10-trioxa-14,21-diazatriacontyl)-4,7,10,13,16-pentaoxanonadecane-1,19-diamide, also known as satDIVA, was prepared in similar fashion as diva-PEG-diVA from previously described N1,N19-bis((S)-16,20-diamino-15-oxo-4,7,10-trioxa-14-azaicosyl)-4,7,10,13,16-pentaoxanonadecane-1,19-diamide with the substitution of 3,7-dimethyl-9-(2,6,6-trimethylcyclohex-1-en-1-yl)nonanoic acid for all*-trans* retinoic acid. QTOF MS ESI+: *m*/*z* 2161, 2163, 2165 & 2167 (M + H+)

### Example 27: SYNTHESIS OF simDiVA (preparative example)

Preparation of N1,N19-bis((S)-15,22-dioxo-30-(2,6,6-trimethylcyclohex-1-en-1-yl)-16-(9-(2,6,6-trimethylcyclohex-1-en-1-yl)nonanamido)-4,7,10-trioxa-14,21-diazatriacontyl)-4,7,10,13,16-pentaoxanonadecane-1,19-diamide (simDiVA).

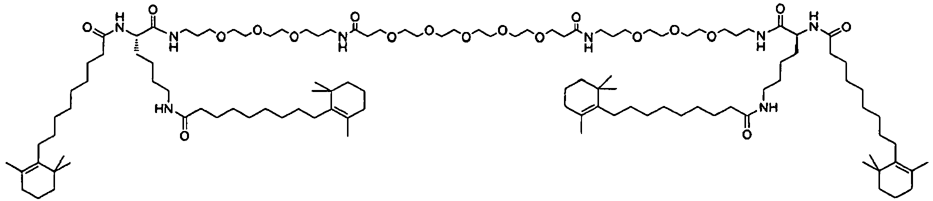

Preparation of Intermediate 1: 2,6,6-trimethylcyclohex-1-en-1-yl trifluoromethanesulfonate

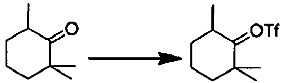

To a solution of 2,2,6-trimethylcyclohexanone in dry THF at -78 °C under nitrogen was added dropwise a 2 M lithium diisopropylamide solution. The mixture was stirred at -78 °C for 3 h. A solution of *N*-phenyl-bis(trifluoromethanesulfonimide) in THF was then added dropwise (at -78 °C). The reaction flask was packed in dry-ice and stirred overnight. The stirring was continued at room temperature for 3 h under which time all material had dissolved. The reaction mixture was concentrated and the residue was added slowly to hexane (350 mL) under vigorous stirring. The solid material was removed by filtration and washed with hexane (2 x 50 mL). The filtrate was concentrated and more hexane (150 mL) was added. The solid material was removed by filtration and the filtrate was concentrated. The precipitation was repeated one more time after which the residue was purified by flash chromatography (silica, hexane) to give 2,6,6-trimethylcyclohex-1-en-1-yl trifluoromethanesulfonate as a colorless oil (23.2 g, 60% yield).

Preparation of Intermediate 2: ethyl 9-(bromozincio)nonanoate

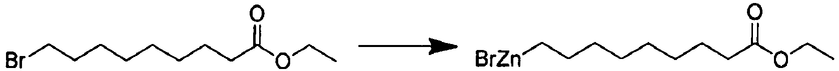

In a dry reaction tube under nitrogen were charged zinc dust (3.70 g, 56.6 mmol), iodine (479 mg, 1.89 mmol) and dry DMA (20 mL). The mixture was stirred at room temperature until the color of iodine disappeared. Ethyl 9-bromononanoate was added, and the mixture was stirred at 80 °C for 4 hours and then at room temperature overnight. (Completion of the zinc insertion reaction was checked by GCMS analysis of the hydrolyzed reaction mixture.) The reaction mixture was used without further treatment in the subsequent step. GCMS m/z 186 [M]+ (ethyl nonanoate).

Preparation of Intermediate 3: ethyl 9-(2,6,6-trimethylcyclohex-1-en-1-yl)nonanoate

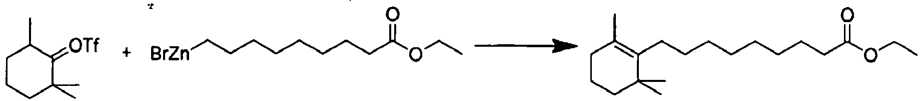

To freshly prepared ethyl 9-(bromozincio)nonanoate (37.7 mmol) in dimethylacetamide under nitrogen in a reaction tube was added 2,6,6-trimethylcyclohex-1-en-1-yl trifluoromethanesulfonate (10.8 g, 39.6 mmol) followed by tetrakis(triphenylphosphine)palladium(0) (872 mg, 0.754 mmol). The tube was sealed and the mixture was stirred at 95 °C for 2 h. The reaction mixture was allowed to cool and was then poured into diethyl ether (100 mL). The upper layer was decanted and the lower layer was washed twice with diethyl ether (2 x 25 mL). The combined ether layers were washed with sat NH₄Cl and brine, dried (MgSO₄) and concentrated to give crude material (∼12 g). The material was purified by flash chromatography (silica, 0 to 1.5% EtOAc in hexane). The obtained oil was stirred under vacuum for 8 h in order to remove most of the side-product, ethyl nonanoate, and was then purified by a second flash chromatography (silica, 0 to 15% toluene in hexane). The fractions were analyzed by LCMS and GCMS. The purest fractions were collected and concentrated at a temperature below 25 °C to give ethyl 9-(2,6,6-trimethylcyclohex-1-en-1-yl)nonanoate as a colorless oil (6.16 g, 53% yield over two steps). LCMS ESI+ m/z 309 [M+H]+; GCMS m/z 308 [M]+.

Preparation of Intermediate 4: 9-(2,6,6-trimethylcyclohex-1-en-1-yl)nonanoic acid

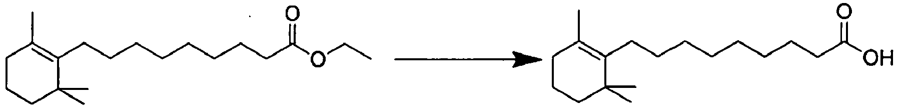

To ethyl 9-(2,6,6-trimethylcyclohex-1-en-1-yl)nonanoate (13.2 g, 42.9 mmol) in ethanol (80 mL) was added 4 M KOH (43 mL). The mixture was stirred at room temperature for 1.5 h. Water (350 mL) was added and the solution was washed with *tert-butyl* methyl ether (2 x 100 mL). The *SimVA,* aqueous phase was cooled, acidified with 4 M HCl (∼45 mL) and extracted with pentane (3 x100 mL). The combined pentane extracts were washed with water (200 mL), dried (MgSO4), filtered, concentrated and dried under high vacuum. The material was redissolved in pentane (100 mL), concentrated and dried under high vacuum one more time to give 9-(2,6,6-trimethylcyclohex-1-en-1-yl)nonanoic acid as a colorless oil (11.1 g, 92% yield). MS ESI- m/z 279 [M-H]-.

Preparation of simdiVA: N1,N19-bis((S)-15,22-dioxo-30-(2,6,6-trimethylcyclohex-1-en-1-yl)-16-(9-(2,6,6-trimethylcyclohex-1-en-1-yl)nonanamido)-4,7,10-trioxa-14,21-diazatriacontyl)-4,7,10,13,16-pentaoxanonadecane-1,19-diamide.

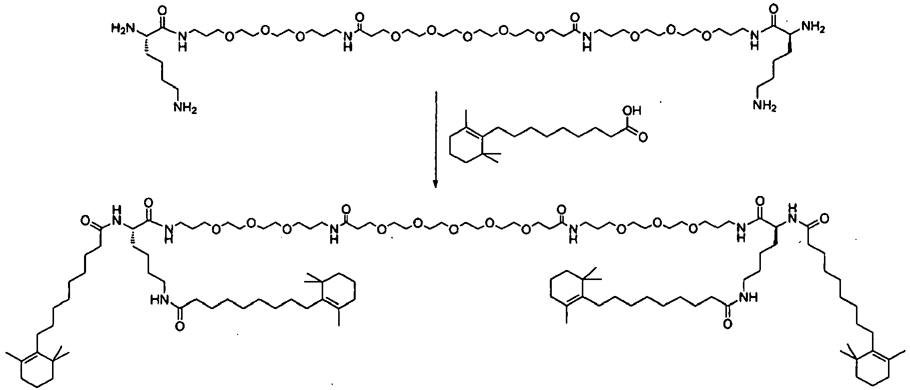

simDIVA was prepared in similar fashion as diVA from previously described N1,N19-bis((S)-16,20-diamino-15-oxo-4,7,10-trioxa-14-azaicosyl)-4,7,10,13,16-pentaoxanonadecane-1,19-diamide with the substitution of 9-(2,6,6-trimethylcyclohex-1-en-1-yl)nonanoic acid for all*-trans* retinoic acid. QTOF MS ESI+: *m*/*z* 2050 (M + H+)

### Example 28: SYNTHESIS OF DiVA-PEG18 (preparative example)

Preparation of (2E,2'E,2"E,4E,4'E,4"E,6E,6'E,6"E,8E,8'E,8"E)-N,N',N"-((5R,69R,76E,78E,80E,82E)-77,81-dimethyl-6,68,75-trioxo-83-(2,6,6-trimethylcyclohex-1-en-1-yl)-10,13,16,19,22,25,28,31,34,37,40,43,46,49,52,55,58,61,64-nonadecaoxa-7,67,74-triazatrioctaconta-76,78,80,82-tetraene-1,5,69-triyl)tris(3,7-dimethyl-9-(2,6,6-trimethylcyclohex-1-en-1-yl)nona-2,4,6,8-tetraenamide) (DIVA-PEG18).

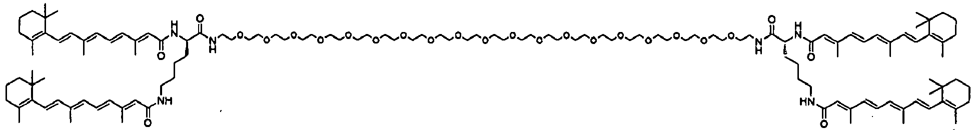

(2E,2'E,2"E,4E,4'E,4"E,6E,6'E,6"E,8E,8'E,8"E)-N,N',N"-((5R,69R,76E,78E,80E,82E)-77,81-dimethyl-6,68,75-trioxo-83-(2,6,6-trimethylcyclohex-1-en-1-yl)-10,13,16,19,22,25,28,31,34,37,40,43,46,49,52, 55,58,61,64-nonadecaoxa-7,67,74-triazatrioctaconta-76,78,80,82-tetraene-1,5,69-triyl)tris(3,7-dimethyl-9-(2,6,6-trimethylcyclohex-1-en-1-yl)nona-2,4,6,8-tetraenamide), also known as DIVA-PEG18 was prepared in similar fashion as diVA with the substitution of PEG₁₈ diamine for diamido-dPEG₁₁-diamine. LCMS ESI+: *m*/*z* 2305 (M + Na).

### Example 29: SYNTHESIS OF TriVA (preparative example)

Preparation of TriVA

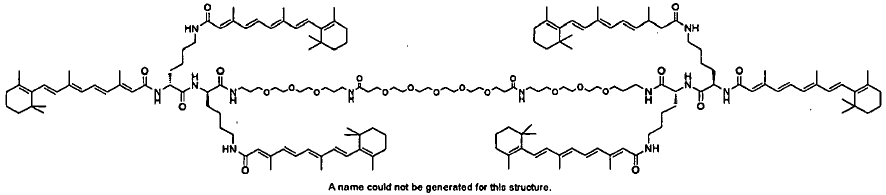

Preparation of Intermediate 1: (S)-methyl 6-(((benzyloxy)carbonyl)amino)-2-((S)-2,6-bis(((benzyloxy) carbonyl)amino)hexanamido) hexanoate

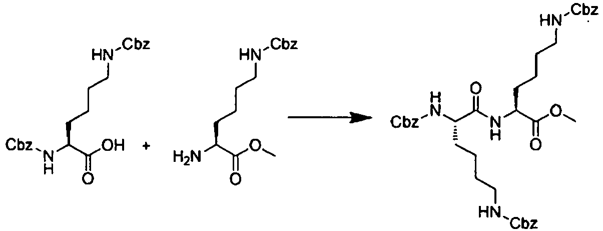

A flask was purged with inert gas and H-Lys(Z)-OMe HCl salt (4g, 12.1mmol), HOBt hydrate (1.84 g, 13.6 mmol), Z-Lys(Z)-OH (5.64g, 13.6mmol) are suspended in dichloromethane (50 mL). NMM (1.5mL, 13.6mmol)was added to the suspension and the solution became clear. A suspension EDC HCl salt (4.01 g, 20.9 mmol) and NMM (2.0 mL, 18.2 mmol) in dichloromethane (50 mL) was added over a period of 10 minutes. The reaction was stirred overnight at room temperature, then washed with 1M HCl (100 mL), H₂O (100 mL), saturated bicarbonate solution (100 mL) and saturated brine solution (100 mL). All aqueous washes were back extracted with dichloromethane (50 mL). Dried organics with Na₂SO₄, filtered and concentrated. Material was purified by silica gel chromatography with a dichloromethane/methanol gradient to yield (S)-methyl 6-(((benzyloxy)carbonyl)amino)-2-((S)-2,6-bis(((benzyloxy)carbonyl)amino)hexanamido) hexanoate (6.91 g).

Preparation of Intermediate 2: (S)-6-(((benzyloxy)carbonyl)amino)-2-((S)-2,6-bis(((benzyloxy)carbonyl) amino)hexanamido)hexanoic acid

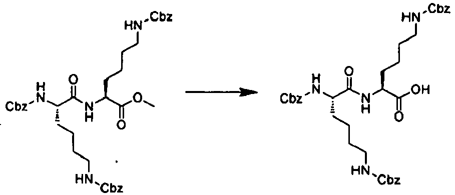

6-(((benzyloxy)carbonyl)amino)-2-((S)-2,6-bis(((benzyloxy)carbonyl)amino)hexanamido) hexanoate (6.91 g, 10mmol) was dissolved with methanol (50mL). Added KOH (2.24g, 40mmol) and allowed mixture to stir at 35°C. After 2 hours, quenched reaction by adding H₂O (200mL) and washed mixture with diethyl ether (50mL). Afterwards, adjusted the pH to ∼2 with 1M HCl acid. Extracted product with dichloromethane (3x100mL), dried with Na₂SO₄, filtered and concentrated to yield (S)-6-(((benzyloxy)carbonyl)amino)-2-((S)-2,6-bis(((benzyloxy)carbonyl)amino)hexanamido)hexanoic acid (4g).

Preparation of Intermediate 3: (Cbz)₆-protected N1,N19-bis((16S,19S)-19,23-diamino-16-(4-aminobutyl)-15,18-dioxo-4,7,10-trioxa-14,17-diazatricosyl)-4,7,10,13,16-pentaoxanonadecane-1,19-diamide

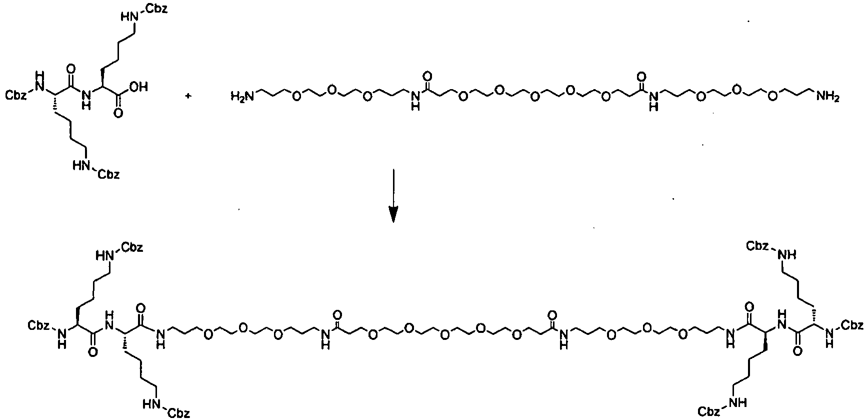

A round bottom flaskwas purged with inert gas and diamido-dPEG₁₁-diamine (1g, 1.35mmol), (S)-6-(((benzyloxy)carbonyl)amino)-2-((S)-2,6-bis(((benzyloxy)carbonyl)amino)hexanamido)hexanoic acid (2.05 g, 3.03 mmol), HOBt hydrate (409 mg, 3.03mmol) are suspended in dichloromethane (25 mL). NMM (333 uL, 3.03 mmol) was added to the suspension and the solution became clear. A suspension EDC HCl salt (893 mg, 4.66 mmol) and NMM (445 uL, 4.05 mmol) in dichloromethane (25 mL)was added over a period of 10 minutes. The reaction was allowed to stir overnight at room temperature, then washed with 1M HCl (100 mL), H2O (100 mL), saturated bicarbonate solution (100 mL) and saturated brine solution (100 mL). All aqueous washes were back extracted with dichloromethane (50 mL). Dried organics with Na₂SO₄, filtered and concentrated. Material was purified by silica gel chromatography with a dichloromethane/methanol gradient to yield (Cbz)₆-protected N1,N19-bis((16S,19S)-19,23-diamino-16-(4-aminobutyl)-15,18-dioxo-4,7,10-trioxa-14,17-diazatricosyl)-4,7,10,13,16-pentaoxanonadecane-1,19-diamide (480 mg).

Preparation of Intermediate 4: N1,N19-bis((16S,19S)-19,23-diamino-16-(4-aminobutyl)-15,18-dioxo-4,7,10-trioxa-14,17-diazatricosyl)-4,7,10,13,16-pentaoxanonadecane-1,19-diamide

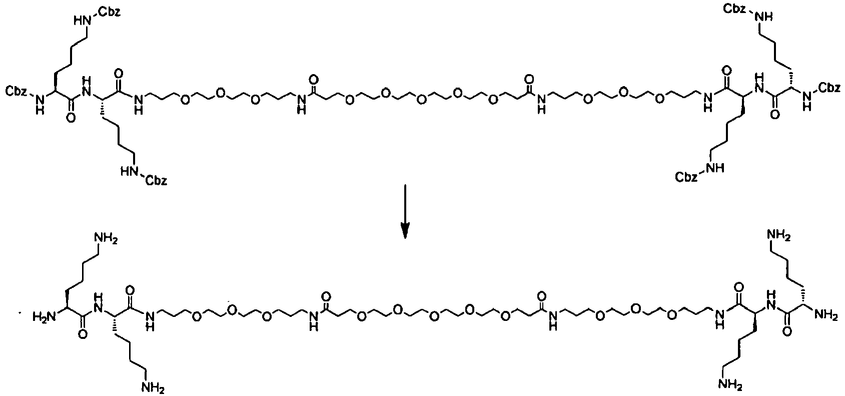

(Cbz)₆-protected N1,N19-bis((16S,19S)-19,23-diamino-16-(4-aminobutyl)-15,18-dioxo-4,7,10-trioxa-14,17-diazatricosyl)-4,7,10,13,16-pentaoxanonadecane-1,19-diamide was dissolved in methanol (30mL) in a round bottom flask and flushed with an inert gas. 10% Pd/C (135mg) was added and the flask was once again flushed with inert gas and then all air was removed via vacuum pump. An 8" H₂ balloon was added and the reaction was allowed to stir at room temperature. After 2 hours, the Pd/C was removed by filtering through a pad of celite washing with methanol, and concentrated to yield N1,N19-bis((16S,19S)-19,23-diamino-16-(4-aminobutyl)-15,18-dioxo-4,7,10-trioxa-14,17-diazatricosyl)-4,7,10,13,16-pentaoxanonadecane-1,19-diamide (823 mg).

Preparation of TriVA

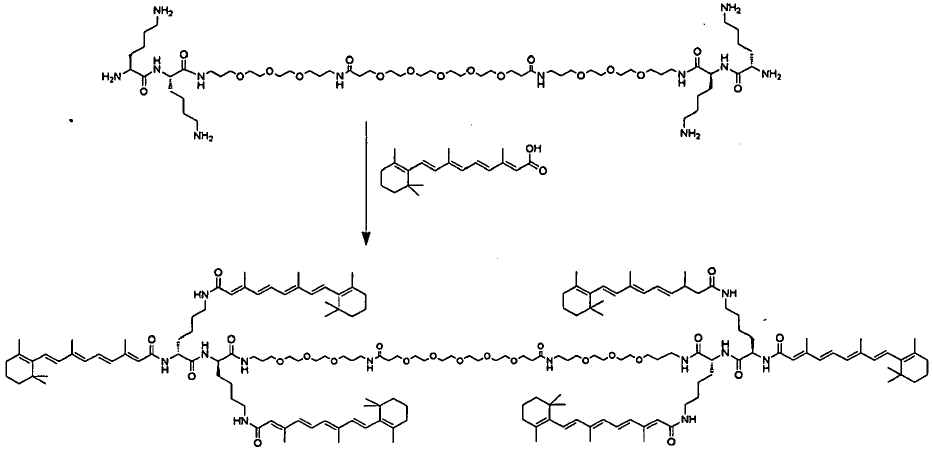

N1,N19-bis((16S,19S)-19,23-diamino-16-(4-aminobutyl)-15,18-dioxo-4,7,10-trioxa-14,17-diazatricosyl)-4,7,10,13,16-pentaoxanonadecane-1,19-diamidewas stirred in dichloromethane and DMAP and retinoic acid was added. NMM was added and the solution was stirred in an aluminum foil covered round bottom flask flushed with inert gas at room temperature. A suspension of EDC HCl salt & NMM in dichloromethane (20 mL) was slowly added to reaction over a period of 10 minutes. Reaction was allowed to stir overnight at room temperature. Next day, diluted with dichloromethane to 100 mL. Washed with H₂O (100 mL), saturated bicarbonate solution (100 mL) and saturated brine solution (100 mL). All aqueous washes were back extracted with dichloromethane (50 mL). Dried organics with Na₂SO₄, filtered and concentrated. Material was purified by basic alumina chromatography eluating with dichloromethane/ethanol gradient to yield TriVA (780 mg). LCMS ESI+: *m*/*z* 2972 (M + Na).

### Example 30: SYNTHESIS OF 4TTNPB (preparative example)

Preparation of N1,N19-bis((R)-1,8-dioxo-7-(4-((E)-2-(5,5,8,8-tetramethyl-5,6,7,8-tetrahydro-naphthalen-2-yl)prop-1-en-1-yl)benzamido)-1-(4-((E)-2-(5,5,8,8-tetramethyl-5,6,7,8-tetrahydronaphthalen-2-yl)prop-1-en-1-yl)phenyl)-13,16,19-trioxa-2,9-diazadocosan-22-yl)-4,7,10,13,16-pentaoxanonadecane-1,19-diamide (4TTNPB).

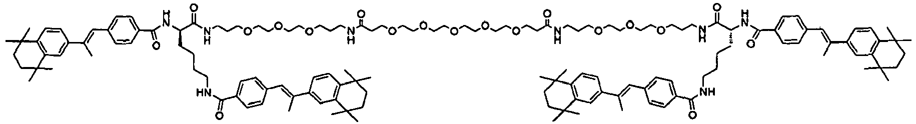

N1N19-bis((R)-1,8-dioxo-7-(4-((E)-2-(5,5,8,8-tetramethyl-5,6,7,8-tetrahydro-naphthalen-2-yl)prop-1-en-1-yl)benzamido)-1-(4-((E)-2-(5,5,8,8-tetramethyl-5,6,7,8-tetrahydronaphthalen-2-yl)prop-1-en-1-yl)phenyl)-13,16,19-trioxa-2,9-diazadocosan-22-yl)-4,7,10,13,16-pentaoxanonadecane-1,19-diamide, also known as 4TTNPB, was prepared in similar fashion as N1,N19-bis((S,23E,25E,27E,29E)-16-((2E,4E,6E,8E)-3,7-dimethyl-9-(2,6,6-trimethylcyclo-hex-1-en-1-yl)nona-2,4,6,8-tetraenamido)-24,28-dimethyl-15,22-dioxo-30-(2,6,6-trimethylcyclohex-1-en-1-yl)-4,7,10-trioxa-14,21-diazatriaconta-23,25,27,29-tetraen-1-yl)-4,7,10,13,16-pentaoxanonadecane-1,19-diamide, also known as diVA, from N1,N19-bis((S)-16,20-diamino-15-oxo-4,7,10-trioxa-14-azaicosyl)-4,7,10,13,16-pentaoxanonadecane-1,19-diamide with the substitution of TTNPB for all*-trans* retinoic acid. LCMS ESI+: *m*/*z* 2343 (M + Na).

### Example 31: SYNTHESIS OF 4Myr (preparative example)

Preparation of N1,N19-bis((R)-15,22-dioxo-16-tetradecanamido-4,7,10-trioxa-14,21-diazapenta-triacontyl)-4,7,10,13,16-pentaoxanonadecane-1,19-diamide (4Myr).

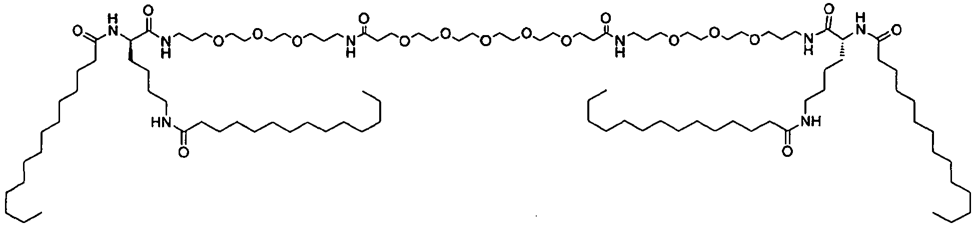

Preparation of 4Myr: N1,N19-bis((R)-15,22-dioxo-16-tetradecanamido-4,7,10-trioxa-14,21-diaza-penta-triacontyl)-4,7,10,13,16-pentaoxanonadecane-1,19-diamide

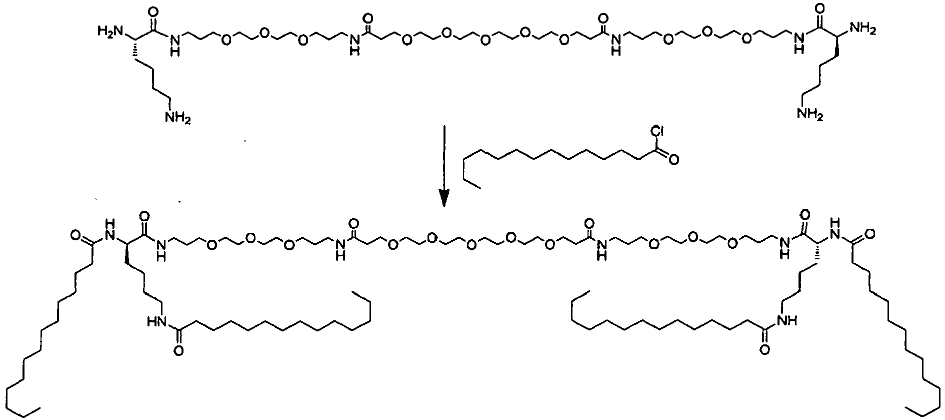

N1,N19-bis((S)-16,20-diamino-15-oxo-4,7,10-trioxa-14-azaicosyl)-4,7,10,13,16-pentaoxanonadecane-1,19-diamide (synthesis previously described) was dissolved in dichloromethane and placed in an ice-bath. Myristoyl chloride was added followed by triethylamine. The ice-bath was removed and the reaction was allowed to stir overnight at room temperature under a blanket of inert gas. Next day, diluted with dichloromethane to 100 mL and washed with 1M HCl (75 mL), H₂O (75 mL), saturated bicarbonate solution (75 mL) and saturated brine solution (75mL). Back extracted all aqueous washes with dichloromethane (25mL). Dried organics with MgSO₄, filtered and concentrated. Purification by silica gel chromatography with a dichloromethane/methanol gradient yielded N1,N19-bis((R)-15,22-dioxo-16-tetradecanamido-4,7,10-trioxa-14,21-diaza-penta-triacontyl)-4,7,10,13,16-pentaoxanonadecane-1,19-diamide (410mg). LCMS ESI+: *m*/*z* 1841 (M + H).

### Example 32: SYNTHESIS OF DiVA-242 (preparative example)

Preparation of N1,N16-bis((R,18E,20E,22E,24E)-11-((2E,4E,6E,8E)-3,7-dimethyl-9-(2,6,6-trimethyl-cyclohex-1-en-1-yl)nona-2,4,6,8-tetraenamido)-19,23-dimethyl-10,17-dioxo-25-(2,6,6-trimethylcyclohex-1-en-1-yl)-3,6-dioxa-9,16-diazapentacosa-18,20,22,24-tetraen-1-yl)-4,7,10,13-tetraoxahexadecane-1,16-diamide, also known as DIVA-242

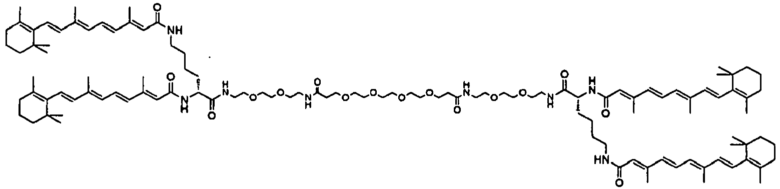

Preparation of Intermediate 1: di-tert-butyl (10,25-dioxo-3,6,13,16,19,22,29,32-octaoxa-9,26-diazatetratriacontane-1,34-diyl)dicarbamate

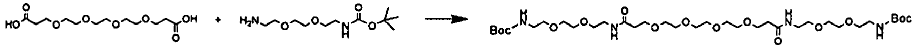

A round bottom flask containing dichloromethane (25 mL) was purged with inert gas and Bis-dPeg₄ acid (1000 mg, 3.40 mmol), N-Boc-3,6-dioxa-1,8-octane diamine (1816 uL, 7.65 mmol) and HOBt hydrate (1034 mg, 7.65 mmol) were added. NMM (841 uL, 7.65 mmol) was added to the suspension and the solution became clear. A suspension of EDC HCl salt (2249 mg, 11.7 mmol) & NMM (1121 uL, 10.2 mmol) in dichloromethane (25 mL) was added followed by DMAP (62 mg, 0.51 mmol). The reaction was allowed to stir overnight at room temperature. It was then diluted with dichloromethane to 100 mL and washed with H₂O (100 mL), 10% K₂CO₃ (100 mL) and saturated brine solution (100 mL), back extracted all aqueous washes with dichloromethane (30 mL), dried with MgSO₄, filtered and concentrated. Purification by silica gel chromatography with a dichloromethane/methanol gradient yielded di-tert-butyl (10,25-dioxo-3,6,13,16,19,22,29,32-octaoxa-9,26-diazatetratriacontane-1,34-diyl)dicarbamate (2.57 g).

Preparation of intermediate 2: N1,N16-bis(2-(2-(2-aminoethoxy)ethoxy)ethyl)-4,7,10,13-tetraoxahexa-decane-1,16-diamide TFA salt

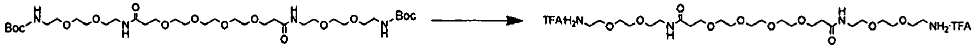

di-tert-butyl (10,25-dioxo-3,6,13,16,19,22,29,32-octaoxa-9,26-diazatetratriacontane-1,34-diyl) dicarbamate was dissolved in dichloromethane (15 mL) and placed into an ice bath, The round bottom flask was flushed with inert gas and TFA (15 mL) was added. Mixture was allowed to stir for 20 minutes. Afterwards, the reaction mixture was concentrated to yield N1,N16-bis(2-(2-(2-aminoethoxy)ethoxy)ethyl)-4,7,10,13-tetraoxahexadecane-1,16-diamide TFA salt (1885 mg).

Preparation of DIVA-242: N1,N16-bis((R,18E,20E,22E,24E)-11-((2E,4E,6E,8E)-3,7-dimethyl-9-(2,6,6-trimethylcyclohex-1-en-1-yl)nona-2,4,6,8-tetraenamido)-19,23-dimethyl-10,17-dioxo-25-(2,6,6-trimethylcyclohex-1-en-1-yl)-3,6-dioxa-9,16-diazapentacosa-18,20,22,24-tetraen-1-yl)-4,7,10,13-tetraoxahexadecane-1,16-diamide

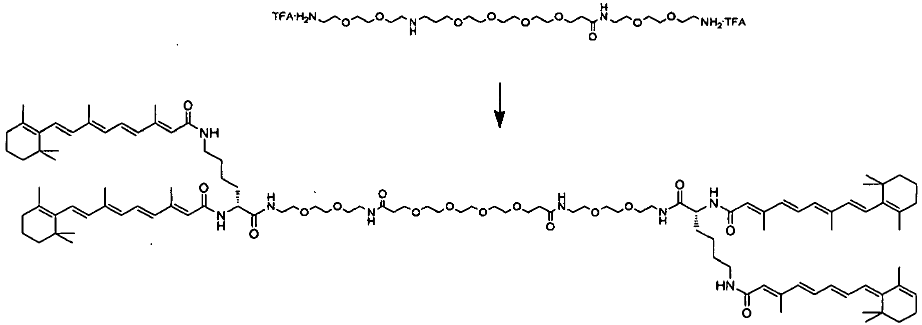

Synthesis of N1,N16-bis((R,18E,20E,22E,24E)-11-((2E,4E,6E,8E)-3,7-dimethyl-9-(2,6,6-trimethylcyclohex-1-en-1-yl)nona-2,4,6,8-tetraenamido)-19,23-dimethyl-10,17-dioxo-25-(2,6,6-trimethylcyclohex-1-en-1-yl)-3,6-dioxa-9,16-diazapentacosa-18,20,22,24-tetraen-1-yl)-4,7,10,13-tetraoxahexadecane-1,16-diamide (DIVA-242) follows the same protocol as diVA from N1,N16-bis(2-(2-(2-aminoethoxy)ethoxy)ethyl)-4,7,10,13-tetraoxahexadecane-1,16-diamide TFA salt. LCMS ESI+: m/z 1940 (M + H).

### Example 33: Formation of nucleic acid-lipid particles

The siRNA referred to in the formulation protocols are double stranded siRNA sequence with 21-mer targeting HSP47/gp46 wherein HSP47 (mouse) and gp46 (rat) are homologs - the same gene in different species:
rat HSP47-C double stranded siRNA used for in vitro assay (rat pHSCs)
   Sense (5'->3') GGACAGGCCUCUACAACUATT (SEQ. ID NO. 2)
   Antisense (3'->5') TTCCUGUCCGGAGAUGUUGAU (SEQ. ID NO. 3)
mouse HSP47-C double stranded siRNA used in formulations for in vivo assay (mouse CC14 model)
   Sense (5'->3') GGACAGGCCUGUACAACUATT (SEQ. ID NO. 4)
   Antisense (3'->5') TTCCUGUCCGGACAUGUUGAU (SEQ. ID NO. 5)

*Cationic Lipid Stock Preparation.* Stock solutions of cationic lipids were prepared by combining the cationic lipid with DOPE, cholesterol, and diVA-PEG-diVA in ethanol at concentrations of 6.0, 5.1 and 2.7 and 2.4 mg/mL respectively. If needed, solutions were warmed up to about 50 °C to facilitate the dissolution of the cationic lipids into solution.

*Empty Liposome Preparation.* A cationic lipid stock solution was injected into a rapidly stirring aqueous mixture at 35-40 °C through injection needle(s) at 1.5 mL/min per injection port. The cationic lipid stock solution to the aqueous solution ratio (v/v) is fixed at 35:65. Upon mixing, empty vesicles formed spontaneously. The resulting vesicles were then allowed to equilibrate at 35-40°C for 10 minutes before the ethanol content was reduced to ∼ 12%. The empty liposomes were then diafiltered against 10X volumes of aqueous buffer to remove ethanol.

*Lipoplex Preparation.* The empty vesicle prepared according to the above method was diluted to the final volume of 1 mM concentration of cationic lipid by 9% sucrose. To the stirring solution, 100 µL of 5% glucose in RNase-free water was added for every mL of the diluted empty vesicle ("EV") and mixed thoroughly. 150 µL of 10 mg/mL siRNA solution in RNase-free water was then added at once and mixed thoroughly. The mixture was then diluted with 5% glucose solution with 1.750 mL for every mL of the EV used. The mixture was stirred at about 200 rpm at room temperature for 10 minutes. Using a semi-permeable membrane with ∼100000 MWCO in a cross-flow ultrafiltration system using appropriately chosen peristaltic pump (e. g. Midgee Hoop, UFP-100-H24LA), the mixture was concentrated to about 1/3 of the original volume (or desired volume) and then diafiltered against 5 times of the sample volume using an aqueous solution containing 3% sucrose and 2.9% glucose. The product was then filtered through a combined filter of 0.8/0.2 micron pore size under aseptic conditions before use.

*Formation of non-di VA siRNA containing liposomes.* Cationic lipid, DOPE, cholesterol, and a PEG-conjugated lipid were solubilized in absolute ethanol (200 proof) at a molar ratio of 50:10:38:2. The siRNA was solubilized in 50 mM citrate buffer and the temperature was adjusted to 35-40°C. The ethanol/lipid mixture was then added to the siRNA-containing buffer while stirring to spontaneously form siRNA loaded liposomes. Lipids were combined with siRNA to reach a final total lipid to siRNA ratio of 15:1 (wt:wt). The range can be 5:1 to 15:1, preferably 7:1 to 15:1. The siRNA loaded liposomes were diafiltered against 10X volumes of PBS (pH 7.2) to remove ethanol and exchange the buffer. Final product was filtered through 0.22 µm, sterilizing grade, PES filter for bioburden reduction. This process yielded liposomes with a mean particle diameter of 50-100 nm, PDI <0.2, and >85% entrapment efficiency.

*Formation of siRNA containing liposomes co-solubilized with diVA. siRNA-*diVA-Liposome formulations were prepared using the method described above. DiVA-PEG-diVA was co-solubilized in absolute ethanol with the other lipids (cationic lipid, DOPE, cholesterol, and PEG-conjugated lipids at a ratio of 50:10:38:2) prior to addition to the siRNA containing buffer. Molar content of diVA-PEG-diVA ranged from 0.1 to 5 molar ratio (50:10:38:2:0.1 to 50:10:38:2:5). This process yielded liposomes with a mean particle diameter of 50-100 nm, PDI <0.2, and >85% entrapment efficiency..

*Formation of siRNA containing liposomes with cationic lipids. siRNA-diVA-*Liposome formulations and siRNA-Liposome formulations were prepared using the method described above. Cationic lipid can be, for example, HEDC, HEDODC, DC-6-14 (comparative), or any combination of these cationic lipids.

*Formation of siRNA containing liposomes decorated with di VA.* siRNA-Liposome formulations were prepared using the method described above and diluted to a siRNA concentration of 0.5 mg/mL in PBS. Cationic lipid can be HEDC, HEDODC, DC-6-14 (comparative), or any combination of these cationic lipids. diVA-PEG-diVA was dissolved in absolute ethanol (200 proof) to a final concentration ranging from 10 to 50 mg/mL. An appropriate amount of ethanol solution was added to the siRNA-Liposome solution to yield a final molar percentage between 2 to 10 mol%. Solution was plunged up and down repeatedly with a pipette to mix. diVA-PEG-diVA concentration and ethanol addition volume were adjusted to keep the addition volume.>1.0 µL and the final ethanol concentration < 3% (vol/vol). Decorated liposomes were then gently shaken at ambient temperature for 1 hr on an orbital shaker prior to *in vitro* or *in vivo* evaluation.

The following tables set forth preferred embodiments of the invention expressed in terms of molar ratios, as well as the equivalent mol % and weight percentages.

| | **Molar Ratio** | | | | |
|---|---|---|---|---|---|
| **Formulation** | **Cationic Lipid** | **DOPE** | **Cholesterol** | **PEG-lipid** | **diVA** |
| HEDODC Liposome | 50 | 10 | 38 | 2 | - |
| HEDODC Liposome +diVA | 50 | 10 | 38 | 2 | 5 |
| DC-6-14 Lipoplex* | 40 | 30 | 30 | - | - |
| DC-6-14 Lipoplex + diVA* | 40 | 30 | 30 | - | 5 |
| | | | | | |

| | **Mol.%** | | | | |
|---|---|---|---|---|---|
| **Formulation** | **Cationic Lipid** | **DOPE** | **Cholesterol** | **PEG-lipid** | **diVA** |
| HE-DODC Liposome | 50 | 10 | 38 | 2 | - |
| HE-DODC Liposome +diVA | 47.6 | 9.5 | 36.2 | 1.9 | 4.8 |
| DC-6-14 Lipoplex* | 40 | 30 | 30 | - | - |
| DC-6-14 Lipoplex + diVA* | 38.1 | 28.6 | 28.6 | - | 4.8 |
| | | | | | |

| | **Weight Percent** | | | | |
|---|---|---|---|---|---|
| **Formulation** | **Cationic Lipid** | **DOPE** | **Cholesterol** | **PEG-lipid** | **diVA** |
| HE-DODC Liposome | 61.1 | 10.8 | 21.3 | 6.9 | - |
| HE-DODC Liposome +diVA | 52.9 | 9.3 | 18.4 | 5.9 | 13.4 |
| DC-6-14 Lipoplex* | 43.8 | 37 | 19.2 | - | - |
| DC-6-14 Lipoplex + diVA* | 37.2 | 31.4 | 16.3 | - | 15.0 |

| | | | | | |
|---|---|---|---|---|---|
| * : comparative | | | | | |

### Example 34: Transfection with liposomal formulations

The transfection method is the same for LX-2 and pHSCs. The liposome formulations or lipoplex formulations of the invention were mixed with growth medium at desired concentrations. 100 µl of the mixture was added to the cells in 96-well plate and cells were incubated for 30 min at 37 °C in the incubator with 5% CO₂. After 30 min, medium was replaced with fresh growth medium. After 48 h of transfection, cells were processed using Cell-to-Ct® lysis reagents (Applied Biosystems) according to the manufacturer's instructions.

*Quantitative RT-PCRfor measuring HSP47 mRNA expression (qRT-PCR)* HSP47 and GAPDH TaqMan® assays and One-Step RT-PCR master mix were purchased from Applied Biosystems. Each PCR reaction contained the following composition: One-step RT-PCR mix 5 µl, TaqMan® RT enzyme mix 0.25 µl, TaqMan® gene expression assay probe (HSP47) 0.25 µl, TaqMan® gene expression assay probe (GAPDH) 0.5 µl, RNase-free water 3.25 µl, Cell lysate 0.75 µl, Total volume of 10 µl. GAPDH was used as endogenous control for the relative quantification of HSP47 mRNA levels. Quantitative RT-PCR was performed in ViiA 7 real-time PCR system (Applied Biosciences) using an in-built Relative Quantification method. All values were normalized to the average HSP47 expression of the mock transfected cells and expressed as percentage of HSP47 expression compared to mock.
*In vivo experiments:* Female C57B1/6 retired breeder mice (Charles River) with a weight range of 24-30 grams were used for this study. Animals were randomly distributed by weight into 10 groups of 10 animals each. All animal procedures were approved by Bio-Quant's IACUC and/or Attending Veterinarian as necessary and all animal welfare concerns were addressed and documented. Mice were anesthetized with Isoflurane and exsanguinated via the inferior vena cava.

Up-regulation of heat shock protein 47 (HSP47) was induced via intraperitoneal injecting CCl₄ (CCl₄ in olive oil, 1:7 (vol/vol), 1 µL per gram body weight) given every other day for 7 days (day 0, 2, 4, 6). On day 3 mice were treated for 4 consecutive days (day 3, 4, 5, 6) with liposome or lipoplex formulations of the invention or PBS by IV injection into the tail vein. One group of ten mice (naive) received neither CCl₄ treatment nor IV injection and served as the control group for normal HSP47 gene expression.

### Experimental Timeline

| **Day** | **0** | **1** | **2** | **3** | **4** | **5** | **6** | **7** |
|---|---|---|---|---|---|---|---|---|
| **CCl₄ IP Injection** | **X** | **X** | **X** | **X** | **X** | **X** | **X** | |
| **Test Article IV Injection** | | | | **X** | **X** | **X** | **X** | |
| **Sample Collection (n=10)** | | | | | | | | **X** |

On day 7 and approximately 24 hours after final IV injection, all remaining mice were sacrificed and the livers were perfused with PBS prior to collecting liver samples for PCR analysis. An approximate 150 mg sample from each mouse liver was collected and placed in 1.5 mL RNAlater stabilization reagent (Qiagen) and stored at 2-8°C until analysis. Liver samples were not collected from areas of clear and marked liver damage and/or necrosis.

Total RNA from mouse livers was extracted using RNeasy columns (Qiagen) according to the manufacturer's protocol. 20 ng of total RNA was used for quantitative RT- PCR for measuring HSP47 expression. HSP47 and GAPDH TaqMan® assays and One-Step RT-PCR master mix were purchased from Applied Biosystems. Each PCR reaction contained the following composition: One-step RT-PCR mix 5 µl, TaqMan® RT enzyme mix 0.25 µl, TaqMan® gene expression assay probe (HSP47) 0.25 µl, TaqMan® gene expression assay probe (GAPDH) 0.5 µl, RNase-free water 3.25 µl, RNA 0.75 µl, Total volume of 10 µl. GAPDH was used as endogenous control for the relative quantification of HSP47 mRNA levels. Quantitative RT-PCR was performed in ViiA 7 realtime PCR system (Applied Biosciences) using an in-built Relative Quantification method. All values were normalized to the average HSP47 expression of the naïve animal group and expressed as percentage of HSP47 expression compared to naive group.

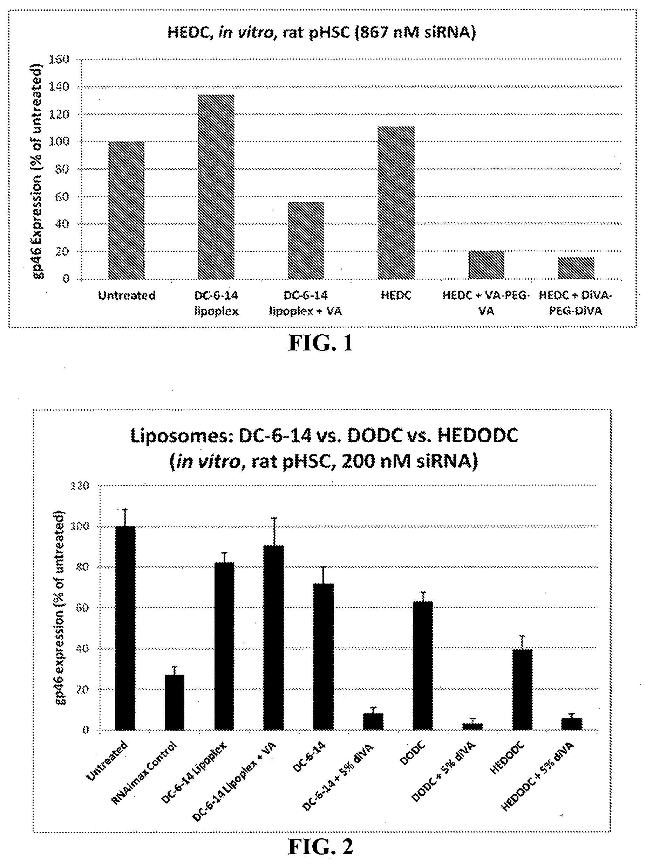

The formulations described in Fig. 1 are as follows:

| | **Molar Ratio** | | | | |
|---|---|---|---|---|---|
| **Formulation** | **Cationic Lipid** | **DOPE** | **Cholesterol** | **PEG-lipid** | **VA or VA-conjugate** |
| DC-6-14 Lipoplex# | 40 | 30 | 30 | - | - |
| DC-6-14 Lipoplex + VA*# | 40 | 30 | 30 | - | 40 |
| HEDC Liposome | 50 | 10 | 38 | 2 | - |
| HEDC Liposome + VA-PEG-VA* | 50 | 10 | 38 | 2 | 5 |
| HEDC Liposome + diVA-PEG-diVA* | 50 | 10 | 38 | 2 | 5 |

| | | | | | |
|---|---|---|---|---|---|
| *VA-PEG-VA and diVA-PEG-diVA were added via co-solubilization. VA was added via decoration post-process #: comparative | | | | | |

The formulations described in Fig. 2 are as follows

| | **Molar Ratio** | | | | |
|---|---|---|---|---|---|
| **Formulation** | **Cationic Lipid** | **DOPE** | **Cholesterol** | **PEG-lipid** | **VA or VA-conjugate** |
| DC-6-14 Lipoplex# | 40 | 30 | 30 | - | - |
| DC-6-14 Lipoplex + VA*# | 40 | 30 | 30 | - | 40 |
| DC-6-14 Liposome# | 50 | 10 | 38 | 2 | |
| DC-6-14 Liposome + #diVA-PEG-diVA* | 50 | 10 | 38 | 2 | 5 |
| HEDODC Liposome | 50 | 10 | 38 | 2 | - |
| HEDODC Liposome + diva-PEG-diVA* | 50 | 10 | 38 | 2 | 5 |

| | | | | | |
|---|---|---|---|---|---|
| *diVA-PEG-diVA was added via co-solubilization. VA was added via decoration post-process. #: comparative | | | | | |

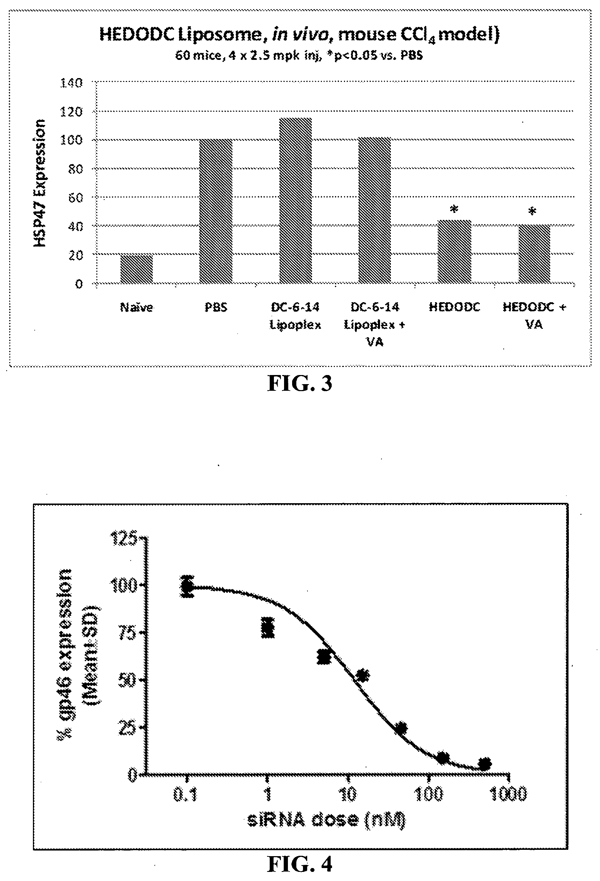

The formulations described in Fig. 3 are as follows.

| | **Molar Ratio** | | | | |
|---|---|---|---|---|---|
| **Formulation** | **Cationic Lipid** | **DOPE** | **Cholesterol** | **PEG-lipid** | **VA or VA-conjugate** |
| DC-6-14 Lipoplex# | 40 | 30 | 30 | - | - |
| DC-6-14 Lipoplex + VA*# | 40 | 30 | 30 | - | 40 |
| HEDODC Liposome | 50 | 10 | 38 | 2 | - |
| HEDODC Liposome + diVA-PEG-diVA* | 50 | 10 | 38 | 2 | 5 |

| | | | | | |
|---|---|---|---|---|---|
| *diVA-PEG-diVA was added via co-solubilization. VA was added via decoration post-process #: comparative | | | | | |

### Example 35: in vitro efficacy (pHSC) - dose response

### pHSC in vitro assay description:

Primary hepatic stellate cells (pHSCs) in a 96-well plate were incubated with formulations (HEDC:S104:DOPE:Chol:Peg-DMPE:DiVA, 20:20:30:25:5:2) of increasing siRNA concentration. After 30 minutes, cells were washed and treated with fresh growth medium and incubated at 37°C for 48 hours. At that time, cells were lysed and gp46 and GAPDH mRNA levels were measured by quantitative RT-PCR (TaqMan®) assay. mRNA levels of gp46 were normalized to GAPDH levels. Normalized gp46 levels are expressed as the percent of untreated control cells. Fig. 4 shows the results. Error bars indicate standard deviations (n=3). Fitting data to a sigmoidal dose-response curve using Graphpad yielded an EC₅₀ of 11.8 nM.

### Example 36: Toxicity

### HepG2 cytotoxicity assay description:

HepG2 cells, an adherent cell line derived from human hepatocellular carcinoma, was cultured in MEM/EBSS (Hyclone, Logan, Utah, Cat# SH30024.01) supplemented with 10% FBS (Hyclone, Logan, Utah Cat# SH30910). HepG2 cells were seeded in 96-well Optilux black plates (BD Falcon, Cat # BD353220) at the density of 5000 cells/well overnight. Formulations were added to each well to final indicated siRNA concentration (n=3). At 48 h post formulation addition, cell viability was determined using CellTiter-Glo Luminescent Cell Viability Assay Kit (Promega, Cat #G7572) following manufacture's instruction. Chemiluminescent signal were measured on Clarity Luminescence Microplate Reader (502-Biotek, Winooski, Vermont). Viability was calculated based on % of chemiluminescent signal in formulation treated well normalized against mock treated wells.

After exploring formulations with our most potent quaternary amine cationic lipids of formula I, we evaluated combinations of quaternary amine cationic lipids of formula I with their respective ionizable synthetic precursors (as shown in the examples below, i-DC* and HEDC, INT4* and DODC*, S104* and HES104 (*: compound not in accordance with formula I of appended claims)).

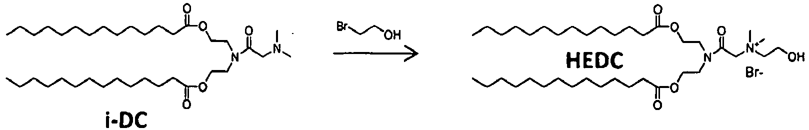

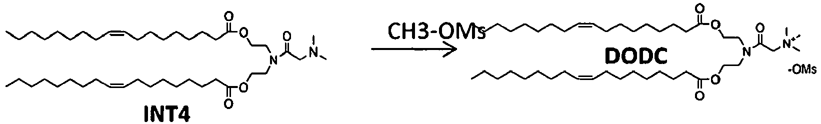

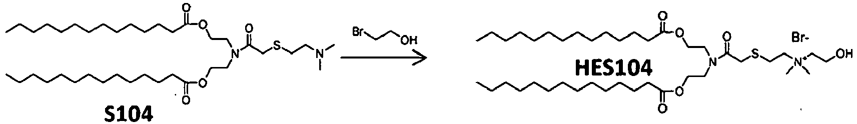

The following table provides exemplary results from different formulations. Combinations of quaternary amine cationic lipids with ionizable cationic lipids surprisingly and unexpectedly were less toxic than liposomes containing a single cationic lipid (see examples HEDC vs. HEDC+ iDC; and DODC vs. DODC+INT4 in table below). The HEDC+S104 combination was identified as another preferred formulation.

| **Variant Description** | **Formulation Variants** | ***in vitro* KD* (%)** | ***in vitro* tox (% cell viability, HepG2 @ 200 nM)** |
|---|---|---|---|
| Cationic Lipid Content | 40 mol% HEDC, no ionizable lipid | 90% @ 200 nM | 27% |
| (2mol% PEG-Lipid) | 50 mol% DODC, no ionizable lipid | 90% @ 200 nM | 55% |
| | 25 mol% DODC: 25 mol% INT4 | 90% @ 200 nM | 90% |
| | 20 mol% HEDC: 20 mol% i-DC | 89% @ 200 nM | 57% |
| | 20 mol% HEDC: 20 mol% S104 | 90% @ 200 n M | 52% |
| | 10 mol% HEDC: 30 mol% S104 | 90% @ 200 n M | 71% |
| | 5 mol% HEDC: 35 mol% S104 | 90% @ 200 nM | 80% |
| PEG Lipid Content | 2 mol% | 70% | |
| (DMPE-PEG) | 5 mol% | 60% | |
| | 7 mol% | 55% | |
| | 10 mol% | 45% | |
| DiVA Content | 0.25 mol% | 35% | |
| (w/ 5mol% DMPE-PEG) | 0.5 mol% | 40% | |
| | 1.0 mol% | 60% | |
| | 2.0 mol% | 70% | |
| DOPE:Cholesterol Ratio | DOPE-0%: Cholesterol 55% | 89% | |
| (w/ 5mol% DMPE-PEG) | DOPE-5%: Cholesterol 50% | 82% | |
| | DOPE-10%: Cholesterol 45% | 77% | |
| | DOPE-15%: Cholesterol 40% | 74% | |
| | DOPE-20%: Cholesterol 35% | 80% | |
| | DOPE-25%: Cholesterol 30% | 79% | |
| | DOPE-30%: Cholesterol 25% | 82% | |
| | DOPE-35%: Cholesterol 20% | 80% | |
| | DOPE-40%: Cholesterol 15% | 84% | |
| | DOPE-45%: Cholesterol 10% | 80% | |
| | DOPE-50%: Cholesterol 5% | 78% | |
| | DOPE-55%: Cholesterol 0% | 72% | |
| siRNA:Total Lipid Ratio | 0.07 | 80% | |
| (w/ 5 mol% DMPE-PEG) | 0.09 | 75% | |
| | 0.11 | 82% | |
| * All data with 20 mol% HEDC, 20 mol% S104, and 2 mol % DIVA @ 50 nM sIRNa dose unless otherwise noted | | | |

### in vivo toxicity

The HEDC:S104 (20:20) formulation is exceptionally well tolerated in preliminary *in vivo* toxicity studies. No toxicity is observed when the formulation is injected intravenously at doses up to **25 mg/kg (rat)** and 12 mg/kg (monkey). This is considered to be superior in this field. For example, Alnylam Pharmaceuticals on March 1, 2012 disclosed at the AsiaTIDES conference that their least toxic third generation lipid nanoparticles had a NOAEL of 10 mg/kg (single dose, rat).

### Example 37: in vivo efficacy (rat DMNQ)

In vivo activity of target formulation was evaluated in the short-term liver damage model (referred to as the Quick Model, DMNQ). In this model, the short-term liver damage induced by treatment with a hepatotoxic agent such as dimethylnitrosamine (DMN) is accompanied by the elevation of gp46 mRNA levels. To induce these changes, male Sprague-Dawley rats were injected intraperitoneally with DMN on six consecutive days. At the end of the DMN treatment period, the animals were randomized to groups based upon individual animal body weight. Formulations were administered as a single intravenous dose, one hour after the last injection of DMN. Twenty four hours after, liver lobes were excised and both gp46 and MRPL19 mRNA levels were determined by quantitative RT-PCR (TaqMan®) assay. Levels of gp46 mRNA were normalized to MRPL19 levels.

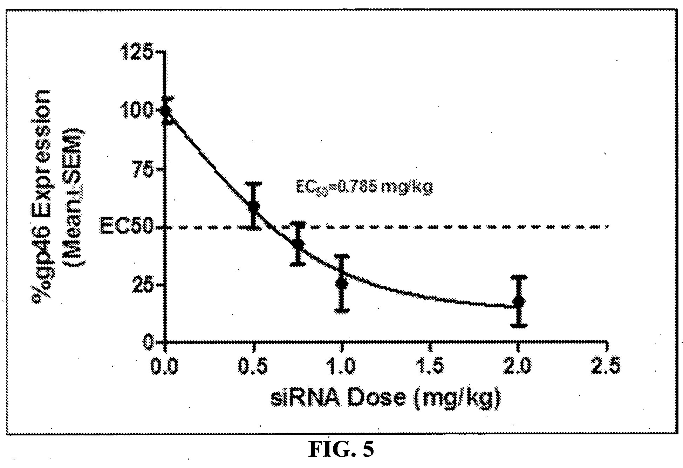

Male Sprague-Dawley rats were treated with DMN at 10 mg/kg on day 1, 2, 3 and 5 mg/kg on day 4, 5, 6 through intraperitoneally dosing to induce liver damage. Animals (n = 8/group) were injected intravenously either with formulations at a dose of 0.5, 0.75, 1.0, 2 mg/kg siRNA in a formulation consisting of HEDC:S104:DOPE:Chol:Peg-DMPE:DiVA (20:20:30:25:5:2), or PBS (vehicle), one hour after the last injection of DMN. Twenty four hours later, total siRNA was purified from a section of the right liver lobe from each animal and stored at 4°C until RNA isolation. Control groups included a PBS vehicle group (DMN-treated) and naive (untreated; no DMN) group. Fig. 5 shows the results of measurements. After subtracting background gp46 mRNA levels determined from the naive group, all test group values were normalized to the average gp46 mRNA of the vehicle group (expressed as a percent of the vehicle group). The mean gp46 mRNA level following treatment showed dose-dependent response and curve fitting to sigmoidal dose response curve yielded EC₅₀ of 0.79 mg/kg.

### Example 38: in vivo efficacy (rat DMNC)

Male Sprague Dawley rats (130-160 g) were treated DMN through intraperitoneally dosing to induce liver fibrosis. The DMN treatment regimen was 3 times each week (Mon, Wed, and Fri) with 10 mg/kg (i.e., 5.0 mg/mL of DMN at a dose of 2.0 mL/kg body weight) for first 3 weeks and half dose of 5 mg/kg (i.e., 5 mg/mL of DMN at a dose of 1.0 mL/kg) from day 22 to 57. The sham group animals were injected with PBS (solvent for DMN) using the same schedule. On day 22, 24 h post the last DMN treatment, blood samples were collected and assayed for liver disease biomarkers to confirm the effectiveness of the DMN treatment. DMN treated animals were assigned to different treatment groups based on body weight to ensure that the mean body weights and the range of body weights of the animals in each group have no significant difference. Animals from pretreatment group were sacrificed on day 25 to evaluate the disease progression stage prior to treatment begins. Treatments with formulations containing gp46 siRNA were started at day 25 with 2 treatments/week at specified siRNA dose for a total of 10 times. On day 59, 48 hours after last formulation treatment and 72 hours after last DMN treatment, animals were sacrificed by CO₂ inhalation. Liver lobes were excised and both gp46 and MRPL19 mRNA levels were determined by quantitative RT-PCR (TaqMan®) assay. mRNA levels for gp46 were normalized to MRPL19 levels.

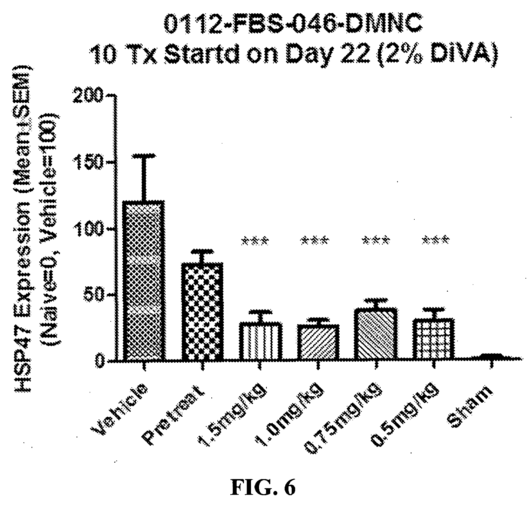

Male Sprague-Dawley rats were treated with DMN at 10 mg/kg for three weeks (three times/week) and then 5 mg/kg from day 22 to 57 (three times/week) through intraperitoneal dosing to induce liver fibrosis. Animals (n = ten/group) were injected intravenously either with formulations consisting of HEDC:S104:DOPE:Chol:Peg-DMPE:DiVA (20:20:30:25:5:2) at 1.5, 1.0, 0.75, and 0.5 mg/kg siRNA or PBS (vehicle) for 10 times (2 times/week), one hour after the last injection of DMN. At day 59, total siRNA was purified from a section of the right liver lobe from each animal and stored at 4°C until analysis. Control groups included a PBS vehicle group (DMN-induced, PBS treated, n=7) and sham group (PBS treated in place of DMN and formulation, n=10) group. Fig. 6 shows the results of measurements. After subtracting background gp46 mRNA levels determined from the naive group, all test group values were normalized to the average gp46 mRNA of the vehicle group (expressed as a percent of the vehicle group). Animal from pretreat group (n=7) were sacrificed on day 25 to evaluate disease progression level prior to treatment began. One-way Anova analysis followed by Dunnett's test showed significant gp46 gene knockdown in all treatment groups as compared to vehicle group (***, P<0.001).

The following table summarizes the compounds described herein, and the results obtained by testing these compounds *in vivo* and *in vitro.*

| **Cationic Lipid Name** | **Structure** | ***in vitro* (pHSC) % KD** | **in vivo (rat DMNQ) % KD** |
|---|---|---|---|
| **Pr-HEDC** | 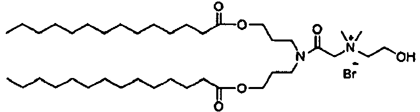 | 75% @ 50 nM | |
| **Pr-HE-DODC** | 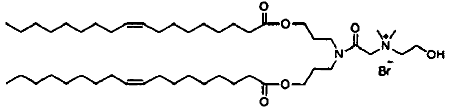 | 73% @ 50 nM | |
| **HE-Et-DC** | 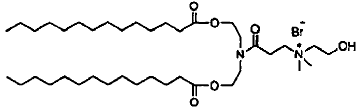 | 70% @ 50 nM | |
| **HE-Et-DODC** | 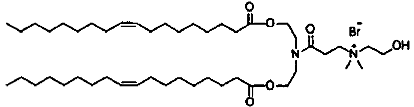 | 71% @ 50 nM | |
| **HE-Pr-DC** | 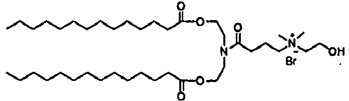 | 47% @ 50 nM | |
| **HE-Pr-DODC** | 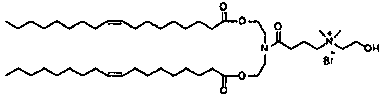 | 75% @ 50 nM | |
| **HE2DODC** | 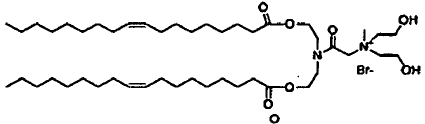 | 78% @ 50 nM | |
| **HEDC-DLin** | 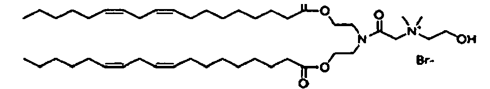 | 50% @ 50 nM | |
| **HEDC** | 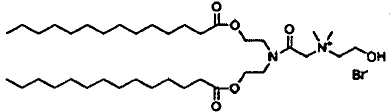 | 68% @ 50 nM | 52% @ 0.5 mpk |
| **HEDC-12*** | 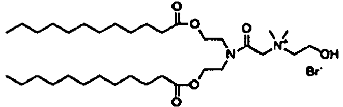 | 0% @ 50 nM | |
| **HES104** | 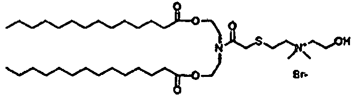 | | |
| **HES104DO** | 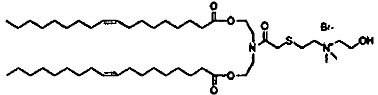 | | |
| **HETU104DO** | 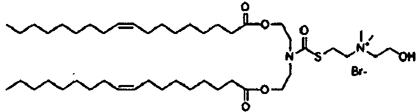 | | |

| | | | |
|---|---|---|---|
| *: comparative | | | |

### Example 39: In vivo anti-pulmonary-fibrosis

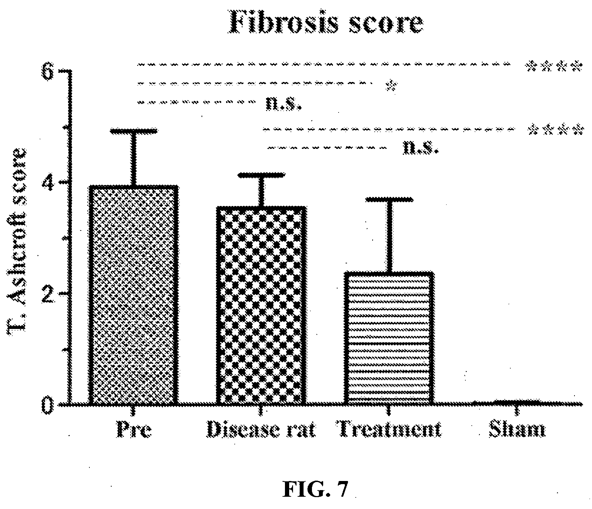

Male S-D rats (8 rats/group, 8 weeks old, Charles River Laboratories Japan, Inc.) were administered once with 0.45 mg bleomycin (BLM) dissolved in 0.1 mL of saline into the lung by intratracheally cannulating (MicroSprayer, Penn-Century, Inc.) under anesthesia, to produce a bleomycin pulmonary fibrosis model. With this method, a significant fibrosis occurs in the lung generally after approximately 2 weeks. The Liposome formulation (1.5 mg/kg as an amount of siRNA, 1 ml/kg in volume, i.e., 200 µl for a rat of 200 g) or PBS (1 ml/kg in volume) was administered to the rats via the tail vein, starting from the 2 weeks after the bleomycin administration, for total of ten times (every other day). The rats were sacrificed at two days post last treatment, histological investigation of the lung tissue was performed (see Fig. 7). One way ANOVA and Bonferroni multi comparison test was used for the evaluation of statistically-significant difference.

A part of the removed lung was formalin-fixed in accordance with a routine method, and subjected to azan staining (azocarmine, aniline blue orange G solution)..

As shown by the results of histological scoring (T. Ashcroft score) in Fig. 7, in the formulation administration group (Treatment), fibrosis score was significantly decreased.

### Example 40: In vitro evaluation of VA-siRNA-liposome formulations for knockdown efficiency in LX-2 cell line and rat primary hepatic stellate cells (pHSCs)

LX2 cells (Dr. S.L. Friedman, Mount Sinai School of Medicine, NY) were grown in DMEM (Invitrogen) supplemented with 10 % fetal bovine serum (Invitrogen) at 37 °C in the incubator with 5% CO₂. Cells were trypsinized using TryPLExpress solution (Invitrogen) for 3 min at 37 °C in the incubator. The cell concentration was determined by cell counting in hemocytometer and 3000 cells/well were seeded into the 96-well plates. The cells were grown for 24 h prior to transfection.

Rat primary hepatic stellate cells (pHSCs) were isolated from Sprague-Dawley rats according to the previously published method (Nat. Biotechnol. 2008, 26(4):431-42). pHSCs were grown in DMEM supplemented with 10% fetal bovine serum. Cells were grown up to two passages after isolation before using them for *in vitro* screening. Cells were seeded at the cell density of 1000 cells/well in 96-well plates and grown for 48 h before using them for transfection.

Transfection with VA-siRNA-Liposome formulations. The transfection method is the same for LX-2 and pHSC cells. The VA-siRNA-Liposome or VA-siRNA-Lipoplex formulations were mixed with growth medium at desired concentrations. 100 µl of the mixture was added to the cells in 96-well plate and cells were incubated for 30 min at 37 °C in the incubator with 5% CO₂. After 30 min, medium was replaced with fresh growth medium after. After 48 h of transfection, cells were processed using Cell-to-Ct lysis reagents (Applied Biosystems) according to the manufacturer's instructions.

Quantitative (q) RT-PCR for measuring HSP47 mRNA expression. HSP47 and GAPDH TaqMan® assays and One-Step RT-PCR master mix were purchased from Applied Biosystems. Each PCR reaction contained the following composition: One-step RT-PCR mix 5 µl, TaqMan® RT enzyme mix 0.25 µl, TaqMan® gene expression assay probe (HSP47) 0.25 µl, TaqMan® gene expression assay probe (GAPDH) 0.5 µl, RNase-free water 3.25 µl, Cell lysate 0.75 µl, Total volume of 10 µl. GAPDH was used as endogenous control for the relative quantification of HSP47 mRNA levels. Quantitative RT-PCR was performed in ViiA™ 7 realtime PCR system (Applied Biosciences) using an in-built Relative Quantification method. All values were normalized to the average HSP47 expression of the mock transfected cells and expressed as percentage of HSP47 expression compared to mock.

The siRNA referred to in the formulation protocols are double stranded siRNA sequence with 21-mer targeting HSP47/gp46 wherein HSP47 (mouse) and gp46 (rat) are homologs - the same gene in different species:
Rat HSP47-C double stranded siRNA used for *in vitro* assay (rat pHSCs)
Sense (5'->3') GGACAGGCCUCUACAACUATT (SEQ. ID NO. 1)
Antisense (3'->5') TTCCUGUCCGGAGAUGUUGAU (SEQ. ID NO. 2).

Cationic Lipid Stock Preparation. Stock solutions of cationic lipids were prepared by combining the cationic lipid with DOPE, cholesterol, and diVA-PEG-DiVA in ethanol at concentrations of 6.0, 5.1 and 2.7 and 2.4 mg/mL respectively. If needed, solutions were warmed up to about 50° C to facilitate the dissolution of the cationic lipids into solution.

Empty Liposome Preparation. A cationic lipid stock solution was injected into a rapidly stirring aqueous mixture of 9% sucrose at 40±1° C through injection needle(s) at 1.5 mL/min per injection port. The cationic lipid stock solution to the aqueous solution ratio (v/v) is fixed at 35:65. Upon mixing, empty vesicles formed spontaneously. The resulting vesicles were then allowed to equilibrate at 40° C for 10 minutes before the ethanol content was reduced to ∼ 12%.

Lipoplex Preparation. The empty vesicle prepared according to the above method was diluted to the final volume of 1 mM concentration of cationic lipid by 9% sucrose. To the stirring solution, 100 µL of 5% glucose in RNase-free water was added for every mL of the diluted empty vesicle ("EV") and mixed thoroughly. 150 µL of 10 mg/mL siRNA solution in RNase-free water was then added at once and mixed thoroughly. The mixture was then diluted with 5% glucose solution with 1.750 mL for every mL of the EV used. The mixture was stirred at about 200 rpm at room temperature for 10 minutes. Using a semi-permeable membrane with ∼100000 MWCO in a cross-flow ultrafiltration system using appropriately chosen peristaltic pump (e. g. Midgee Hoop, UFP-100-H24LA), the mixture was concentrated to about 1/3 of the original volume (or desired volume) and then diafiltered against 5 times of the sample volume using an aqueous solution containing 3% sucrose and 2.9% glucose. The product was then filtered through a combined filter of 0.8/0.2 micron pore size under aseptic conditions before use.

Formation of non-diVA siRNA containing liposomes. Cationic lipid, DOPE, cholesterol, and PEG conjugated lipids (e.g., Peg-Lipid) were solubilized in absolute ethanol (200 proof) at a molar ratio of 50:10:38:2. The siRNA was solubilized in 50 mM citrate buffer, and the temperature was adjusted to 35-40° C. The ethanol/lipid mixture was then added to the siRNA-containing buffer while stirring to spontaneously form siRNA loaded liposomes. Lipids were combined with siRNA to reach a final total lipid to siRNA ratio of 15:1 (wt:wt) The range can be 5:1 to 15:1, preferably 7:1 to 15:1. The siRNA loaded liposomes were diafiltered against 10X volumes of PBS (pH 7.2) to remove ethanol and exchange the buffer. Final product was filtered through 0.22 µm, sterilizing grade, PES filter for bioburden reduction. This process yielded liposomes with a mean particle diameter of 50-100 nm, PDI <0.2, >85% entrapment efficiency.

Formation of siRNA containing liposomes co-solubilized with diVA. siRNA-diVA-Liposome formulations were prepared using the method described above. diVA-PEG-diVA was co-solubilized in absolute ethanol with the other lipids (cationic lipid, DOPE, cholesterol, and PEG-conjugated lipids at a ratio of 50:10:38:2) prior to addition to the siRNA containing buffer. Molar content of diVA-PEG-diVA ranged from 0.1 to 5 molar ratio. This process yielded liposomes with a mean particle diameter of 50-100 nm, PDI <0.2, >85% entrapment efficiency.

Formation of siRNA containing_liposomes with cationic lipids. siRNA-diVA-Liposome formulations and siRNA-Liposome formulations were prepared using the method described above. Cationic lipid can be, for example, DODC*, HEDC, HEDODC, DC-6-14*, or any combination of these cationic lipids (*: comparative).

Formation of siRNA containing liposomes decorated with diVA. siRNA-Liposome formulations were prepared using the method described above and diluted to a siRNA concentration of 0.5 mg/mL in PBS. Cationic lipid can be DODC*, HEDC, HEDODC, DC-6-14*, or any combination of these cationic lipids (*: comparative). diVA-PEG-diVA was dissolved in absolute ethanol (200 proof) to a final concentration ranging from 10 to 50 mg/mL. An appropriate amount of ethanol solution was added to the siRNA-Liposome solution to yield a final molar percentage between 2 to 10 mol%. Solution was plunged up and down repeatedly with a pipette to mix. diVA-PEG-diVA concentration and ethanol addition volume were adjusted to keep the addition volume >1.0 µL and the final ethanol concentration < 3% (vol/vol). Decorated liposomes were then gently shaken at ambient temperature for 1 hr on an orbital shaker prior to *in vitro* or *in vivo* evaluation.

### Results

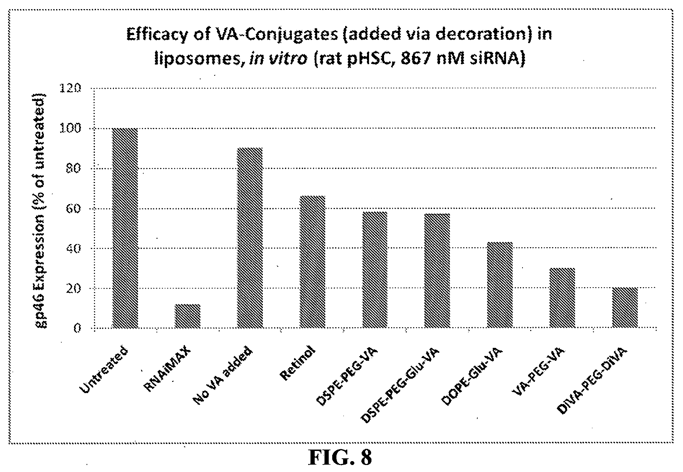

Fig. 8 shows that addition of the VA-conjugate to liposomes via decoration improved the knockdown efficacy of siRNA, enhancing siRNA activity. Peg-Lipid. The dose for all samples was 867 nM siRNA HSP47-C. The results showed that in every instance where a VA-conjugate was added to liposomes, siRNA activity was enhanced compared to liposomes without a retinoid and compared to liposomes decorated with free (non-conjugated) retinol. RNAiMAX was a commercial transfection reagent.

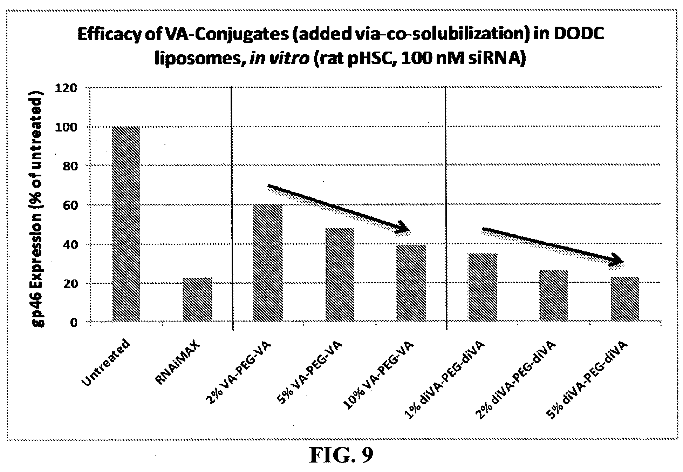

Fig. 9 shows that addition of VA-conjugates to liposomes via co-solubilization improves knockdown efficacy of siRNA. These were DODC containing liposomes* with VA-conjugates added via co-solubilization (*: comparative). The formulation is 50:10:38:2:X, where X = 1 to 10 (DODC:DOPE:cholesterol:PEG-Lipid:VA-conjugate, mole ratio). The concentration in every instance was 100 nM siRNA HSP47-C. The results show that addition of VA-conjugates to liposomes via cosolubilization enhances siRNA activity.

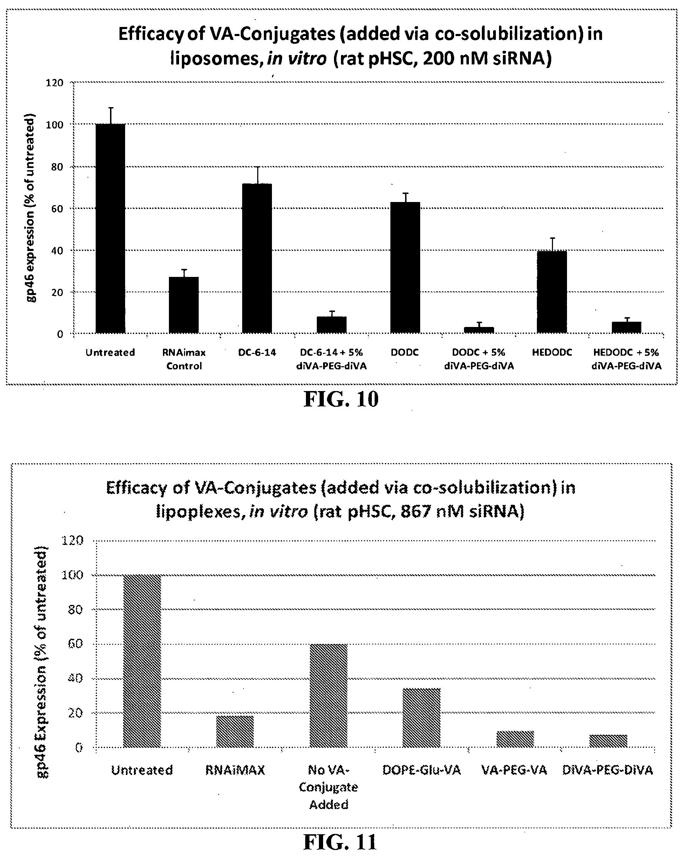

Fig. 10 shows that addition of VA-conjugate to liposomes via co-solubilization dramatically improves the knockdown efficacy of siRNA. Results include three different liposomes ,DC-6-14*, DODC*, HEDODC with VA-conjugates added via co-solubilization (*: comparative). The formulation is the same for all, 50:10:38:2, cationic lipid:DOPE:cholesterol:Peg-Lipid, with only the cationic lipid varying. The concentration of siRNA is 200 nM siRNA HSP47-C is the same for all. The results show in that VA-conjugate addition to liposomes having different cationic lipids significantly enhanced siRNA activity, when prepared by co-solubilization.

Fig. 11 shows that addition of VA-conjugates to lipoplexes having DC-6-14 cationic lipid* via co-solubilization, and siRNA coating the exterior of the liposome enhances siRNA activity (*: comparative). The formulation is a 40% lipoplex formulation, 40:30:30, DC-6-14:DOPE:cholesterol. The concentration for all samples is 867 nM siRNA HSP47-C. The results show that VA-conjugate addition to lipoplexes via co-solubilization enhance siRNA activity.

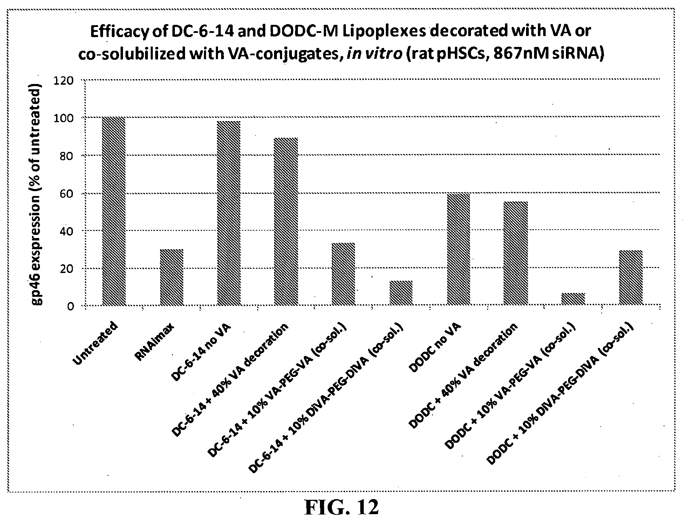

Fig. 12 shows that addition of VA-conjugate to lipoplexes formed via co-solubilization compared to lipoplexes with VA-conjugate added via decoration. These results are from DC-6-14* and DODC* lipoplexes (*: comparative). The formulation consists of 40:30:30, DC-6-14:DOPE:cholesterol. The concentration in each sample is 867 nM siRNA HSP47-C. VA-conjugate addition via co-solubilization significantly improves knockdown efficacy *in vitro,* relative to VA-conjugates added by decoration.

### Example 41: In vivo experiments

Female C57B1/6 retired breeder mice (Charles River) with a weight range of 24-30 grams were used. Animals were randomly distributed by weight into 10 groups of 10 animals each. All animal procedures were approved by Bio-Quant's IACUC and/or Attending Veterinarian as necessary and all animal welfare concerns were addressed and documented. Mice were anesthetized with Isoflurane and exsanguinated via the inferior vena cava.

Mouse HSP47-C double stranded siRNA used in formulations for *in vivo* assay (mouse CC14 model)
Sense (5'->3') GGACAGGCCUGUACAACUATT (SEQ. ID NO. 3)
Antisense (3'->5') TTCCUGUCCGGACAUGUUGAU (SEQ. ID NO. 4)

Upregulation of heat shock protein 47 (HSP47) was induced via intraperitoneal injection of CCl₄ (CCl₄ in olive oil, 1:7 (vol/vol), 1 µL per gram body weight) given every other day for 7 days (day 0, 2, 4, 6). On day 3 mice were treated for 4 consecutive days (day 3, 4, 5, 6) with liposome or lipoplex formulations of the invention or PBS by IV injection into the tail vein. One group of ten mice (naive) received neither CClCCl₄ treatment nor IV injection and served as the control group for normal HSP47 gene expression.

### Experimental Timeline

| **Day** | **0** | **1** | **2** | **3** | **4** | **5** | **6** | **7** |
|---|---|---|---|---|---|---|---|---|
| **CCl₄ IP Injection** | **X** | **X** | **X** | **X** | **X** | **X** | **X** | |
| **Test Article IV Injection** | | | | **X** | **X** | **X** | **X** | |
| **Sample Collection (n=10)** | | | | | | | | **X** |

On day 7 and approximately 24 hours after final IV injection, all remaining mice were sacrificed and the livers were perfused with PBS prior to collecting liver samples for PCR analysis. An approximate 150 mg sample from each mouse liver was collected and placed in 1.5 mL RNAlater stabilization reagent (Qiagen) and stored at 2-8° C until analysis. Liver samples were not collected from areas of clear and marked liver damage and/or necrosis.

Total RNA from mouse livers was extracted using RNeasy® columns (Qiagen) according to the manufacturer's protocol. 20 ng of total RNA was used for quantitative RT-PCR for measuring HSP47 expression. HSP47 and GAPDH TaqMan® assays and One-Step RT-PCR master mix were purchased from Applied Biosystems. Each PCR reaction contained the following composition: One-step RT-PCR mix 5 µl, TaqMan® RT enzyme mix 0.25 µl, TaqMan® gene expression assay probe (HSP47) 0.25 µl, TaqMan® gene expression assay probe (GAPDH) 0.5 µl, RNase-free water 3.25 µl, RNA 0.75 µl, Total volume of 10 µl. GAPDH was used as endogenous control for the relative quantification of HSP47 mRNA levels. Quantitative RT-PCR was performed in ViiA™ 7 realtime PCR system (Applied Biosciences) using an in-built Relative Quantification method. All values were normalized to the average HSP47 expression of the naïve animal group and expressed as percentage of HSP47 expression compared to naive group.

### Example 42: IN VITRO EFFICACY OF FAT-SOLUBLE VITAMIN TARGETING CONJUGATE HEDC:S104:DOPE:Chol:PEG-DMPE:DiVA

Liposome formulations with 50 nM siRNA were tested. The liposomes were either: HEDC:S104:DOPE:Chol:PEG-DMPE:DiVA (+DiVA) or controls lacking vitamin A moieties (-DiVA) and were incubated in 96-well culture plates containing rat hepatic stellate cells for 30 minutes. After 30 minutes, medium was replaced with fresh growth medium. Forty eight hours later, cells were lysed and gp46 and GAPDH mRNA levels measured by quantitative RT-PCR (TaqMan®®) assay, and gp46 levels were normalized to GAPDH levels.

*In vitro* efficacy (pHSC), effect of 2% DiVA siRNA was efficacious with 2 % diVA and had an EC₅₀ of 14 nM. PHSCs in 96-well plate were incubated with formulation that lacked vitamin A moieties for targeting (-DiVA), or formulation that included vitamin A moieties (+DiVA) at 50 nM siRNA. After 30 minutes, medium was replaced with fresh growth medium. Forty eight hours later, cells were lysed and gp46 and GAPDH mRNA levels measured by quantitative RT-PCR (TaqMan®) assay, and gp46 levels were normalized to GAPDH levels. Normalized gp46 levels were expressed as percent of mock control cells. Error bars indicate standard deviations (n=3). The mean gp46 level following DiVA containing treatment is significantly different from the mock control treatment (P < 0.001) based on one-tailed t-test.

### COMPARISON OF DiVA AND satDiVA

Liposome formulations were transfected into rat pHSCs for 30 min in 96-well plates. After 48 hours, the cells were processed using Cells-to-Ct® lysis reagents (Applied Biosystems) and HSP47 mRNA levels were quantified by qRT-PCR. HSP47 expression was normalized mock control. EC₅₀ was determined by measuring HSP47 knockdown (KD) at six half-log doses of siRNA and fitting the data to the "Classic sigmoidal dose response function" in Graphpad Prism® 5.04.

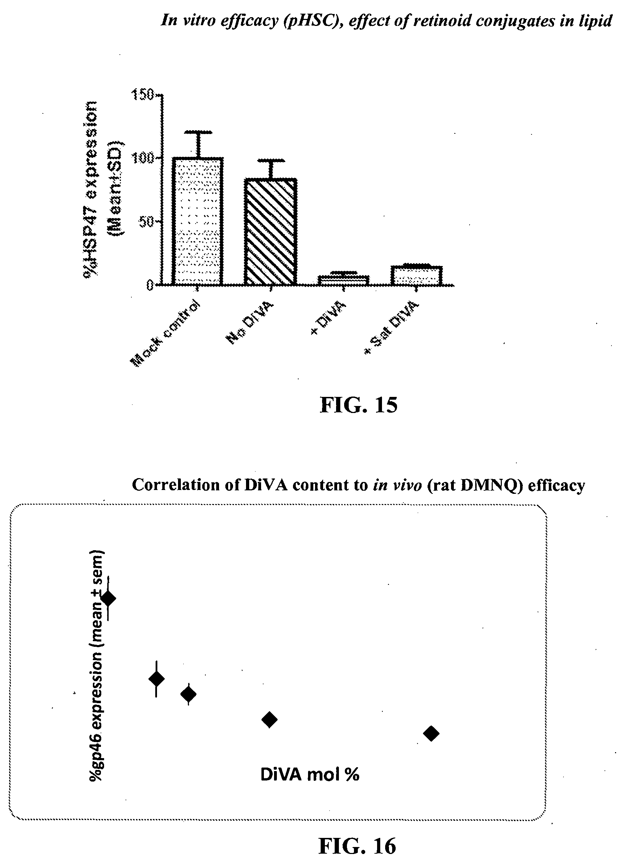

Results shown that both DiVA and Sat DiVA increased KD efficacy (table below and also Fig 15). The EC₅₀ is 12 nM for DiVA and the EC₅₀ is 14 nM for Sat DiVA.

| **Retinoid Conjugate** | **Formulation** | ***in vitro* (pHSC) EC₅₀ or % KD** | **in vivo (rat DMNQ) %KD** |
|---|---|---|---|
| DiVA | 20:20 HEDC:S104 with 2% DiVA | EC₅₀ = 12 nM | 60% @ 0.75 mpk |
| satDiVA | 20:20 HEDC:S104 with 2% satDiVA | EC₅₀ = 14 nM | 74% @ 0.75 mpk |

### Retinoid conjugate vs non-retinoid conjugate

Retinoid conjugates were found to be consistently more potent *in vitro* relative to the non-reti2noid equivalents (see 4TTNBB and 4Myr vs. the retinoid conjugate equivalents satDiVA and DiVA

| **Compound (Type of Conjugate)** | **Formulation** | ***in vitro* (pHSC) EC₅₀ or % KD** |
|---|---|---|
| DiVA (retinoid) | 20:20 HEDC:S104 with 2% DiVA | 74% @ 50 nM |
| satDiVA (retinoid) | 20:20 HEDC:S104 with 2% satDiVA | 73% @ 50 nM |
| 4TTNPB (non-retinoid) | 20:20 HEDC:S104 with 2% 4TTNPB | 34% @ 50 nM |
| 4Myr (non-retinoid) | 20:20 HEDC:S104 with 2% 4Myr | 27% @ 50 nM |

### Example 43: IN VIVO EFFICACY OF FAT-SOLUBLE VITAMIN TARGETING CONJUGATE HEDC:S104:DOPE:Chol:PEG-DMPE:DiVA

*In vivo* activity of target formulation was evaluated in the short-term liver damage model (referred to as the Quick Model, DMNQ). In this model, short-term liver damage is induced by treatment with a hepatotoxic agent such as dimethylnitrosamine (DMN), and is accompanied by the elevation of gp46 mRNA levels. To induce these changes, male Sprague-Dawley rats were injected intraperitoneally with DMN on six consecutive days. At the end of the DMN treatment period, the animals were randomized to groups based upon individual animal body weight. Formulations were administered as a single IV dose, and given one hour after the last injection of DMN. Twenty four hours later, liver lobes were excised and both gp46 and MRPL19 mRNA levels were determined by quantitative RT-PCR (TaqMan®®) assay. mRNA levels for gp46 were normalized to MRPL19 levels.

The results (Fig. 16) show a correlation between the amount of retinoid conjugate and efficacy is evident. Only 0.25 mol% is required to see a significant effect in the rat DMNQ model. With 2 mol % DiVA a robust knockdown of gp46 expression is observed. Fig 16 shows male Sprague-Dawley rats that were treated with DMN at 10 mg/kg on day 1, 2, 3 and 5 mg/kg on day 4, 5, 6 through intraperitoneal dosing to induce liver damage. Animals (n = 8/group) were injected intravenously either with formulations containing 0, 0.25, 0.5, 1, and 2% DiVA at a dose of 0.75 mg/kg siRNA, or PBS (vehicle), one hour after the last injection of DMN. Twenty four hours later, total siRNA was purified from a section of the right liver lobe from each animal and stored at 4° C until RNA isolation. Control groups included a PBS vehicle group (DMN-treated) and naive (untreated; no DMN) group. After subtracting background gp46 mRNA levels determined from the naive group, all test group values were normalized to the average gp46 mRNA of the vehicle group (expressed as a percent of the vehicle group).

Male Sprague Dawley rats (130-160 g) were treated DMN through intraperitoneal dosing to induce liver fibrosis. The DMN treatment regimen was 3 times each week (Mon, Wed, and Fri) with 10 mg/kg (i.e., 5.0 mg/mL of DMN at a dose of 2.0 mL/kg body weight) for first 3 weeks and half dose of 5 mg/kg (i.e., 5 mg/mL of DMN at a dose of 1.0 mL/kg) from day 22 to 57. The sham group animals were injected with PBS (solvent for DMN) using the same schedule. On Day 22, 24 h post the last DMN treatment, blood samples were collected and assayed for liver disease biomarkers to confirm the effectiveness of the DMN treatment. DMN treated animals were assigned to different treatment groups based on body weight and ensure that the mean body weights and the range of body weights of the animals in each group have no significant difference. Animals from pretreatment group were sacrificed on day 25 to evaluate the disease progression stage prior to treatment begins. Treatments with formulations containing gp46 siRNA were started at day 25 with 2 treatments/week at specified siRNA dose for a total of 10 times. On day 59, 48 hours after last formulation treatment and 72 hours after last DMN treatment, animals were sacrificed by CO₂ inhalation. Liver lobes were excised and both gp46 and MRPL19 mRNA levels were determined by quantitative RT-PCR (TaqMan®) assay. mRNA levels for gp46 were normalized to MRPL19 levels.

It will be readily apparent to one skilled in the art that varying substitutions and modifications can be made to the description disclosed herein without departing from the scope and spirit of the description. Such additional embodiments are within the scope of the present invention provided they are covered by the following claims. The present description teaches one skilled in the art to test various combinations and/or substitutions of chemical modifications described herein toward generating nucleic acid constructs with improved activity for mediating RNAi activity. Such improved activity can include improved stability, improved bioavailability, and/or improved activation of cellular responses mediating RNAi. Therefore, the specific embodiments described herein are not limiting and one skilled in the art can readily appreciate that specific combinations of the modifications described herein can be tested without undue experimentation toward identifying nucleic acid molecules with improved RNAi activity within the scope of the appended claims.

The descriptions illustratively described herein may suitably be practiced in the absence of any element or elements, limitation or limitations, not specifically disclosed herein. Thus, for example, the terms "a" and "an" and "the" and similar referents in the context of describing the description (especially in the context of the following claims) are to be construed to cover both the singular and the plural, unless otherwise indicated herein or clearly contradicted by context. The terms "comprising", "having," "including," containing", etc. shall be read expansively and without limitation (*e.g.,* meaning "including, but not limited to,"). Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g*., "such as") provided herein, is intended merely to better illuminate the description and does not pose a limitation on the scope of the description unless otherwise claimed. No language in the specification should be construed as indicating any non-claimed element as essential to the practice of the description. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the description claimed. Thus, it should be understood that although the present description has been specifically disclosed by preferred embodiments and optional features, modification and variation of the descriptions embodied therein herein disclosed may be resorted to by those skilled in the art, and that such modifications and variations are considered to be within the scope of this description.

The description has been described broadly and generically herein. Each of the narrower species and subgeneric groupings falling within the generic disclosure also form part of the description. This includes the generic description of the description with a proviso or negative limitation removing any subject matter from the genus, regardless of whether or not the excised material is specifically recited herein. Other embodiments are within the following claims. In addition, where features or aspects of the description are described in terms of Markush groups, those skilled in the art will recognize that the description is also thereby described in terms of any individual member or subgroup of members of the Markush group.

### SEQUENCE LISTING

'<110> NITTO DENKO CORPORATION
<120> COMPOUNDS FOR TARGETING DRUG DELIVERY AND ENHANCING SIRNA ACTIVITY
<130> F3137WO (NITT-0017)
<140> PCT/US2012/041753
   <141> 2012-06-08
<150> 61/494,840
   <151> 2011-06-08
<150> 61/494,710
   <151> 2011-06-08
<160> 5
<170> PatentIn version 3.5
<210> 1
   <211> 2208
   <212> RNA
   <213> Homo sapiens
<400> 1
   ucuuuggcuu uuuuuggcgg agcuggggcg cccuccggaa gcguuuccaa cuuuccagaa      60
   guuucucggg acgggcagga gggggugggg acugccauau auagaucccg ggagcagggg     120
   agcgggcuaa gaguagaauc gugucgcggc ucgagagcga gagucacguc ccggcgcuag     180
   cccagcccga cccaggccca ccguggugca cgcaaaccac uuccuggcca ugcgcucccu     240
   ccugcuucuc agcgccuucu gccuccugga ggcggcccug gccgccgagg ugaagaaacc     300
   ugcagccgca gcagcuccug gcacugcgga gaaguugagc cccaaggcgg ccacgcuugc     360
   cgagcgcagc gccggccugg ccuucagcuu guaccaggcc auggccaagg accaggcagu     420
   ggagaacauc cuggugucac ccgugguggu ggccucgucg cuagggcucg ugucgcuggg     480
   cggcaaggcg accacggcgu cgcaggccaa ggcagugcug agcgccgagc agcugcgcga     540
   cgaggaggug cacgccggcc ugggcgagcu gcugcgcuca cucagcaacu ccacggcgcg     600
   caacgugacc uggaagcugg gcagccgacu guacggaccc agcucaguga gcuucgcuga     660
   ugacuucgug cgcagcagca agcagcacua caacugcgag cacuccaaga ucaacuuccg     720
   cgacaagcgc agcgcgcugc aguccaucaa cgagugggcc gcgcagacca ccgacggcaa     780
   gcugcccgag gucaccaagg acguggagcg cacggacggc gcccugcuag ucaacgccau     840
   guucuucaag ccacacuggg augagaaauu ccaccacaag augguggaca accguggcuu     900
   cauggugacu cgguccuaua ccgugggugu caugaugaug caccggacag gccucuacaa     960
   cuacuacgac gacgagaagg aaaagcugca aaucguggag augccccugg cccacaagcu    1020
   cuccagccuc aucauccuca ugccccauca cguggagccu cucgagcgcc uugaaaagcu    1080
   gcuaaccaaa gagcagcuga agaucuggau ggggaagaug cagaagaagg cuguugccau 1140
   cuccuugccc aagggugugg uggaggugac ccaugaccug cagaaacacc uggcugggcu 1200
   gggccugacu gaggccauug acaagaacaa ggccgacuug ucacgcaugu caggcaagaa 1260
   ggaccuguac cuggccagcg uguuccacgc caccgccuuu gaguuggaca cagauggcaa 1320
   ccccuuugac caggacaucu acgggcgcga ggagcugcgc agccccaagc uguucuacgc 1380
   cgaccacccc uucaucuucc uagugcggga cacccaaagc ggcucccugc uauucauugg 1440
   gcgccugguc cggccuaagg gugacaagau gcgagacgag uuauagggcc ucagggugca 1500
   cacaggaugg caggaggcau ccaaaggcuc cugagacaca ugggugcuau ugggguuggg 1560
   ggggagguga gguaccagcc uuggauacuc cauggggugg ggguggaaaa acagaccggg 1620
   guucccgugu gccugagcgg accuucccag cuagaauuca cuccacuugg acaugggccc 1680
   cagauaccau gaugcugagc ccggaaacuc cacauccugu gggaccuggg ccauagucau 1740
   ucugccugcc cugaaagucc cagaucaagc cugccucaau caguauucau auuuauagcc 1800
   agguaccuuc ucaccuguga gaccaaauug agcuaggggg gucagccagc ccucuucuga 1860
   cacuaaaaca ccucagcugc cuccccagcu cuaucccaac cucucccaac uauaaaacua 1920
   ggugcugcag ccccugggac caggcacccc cagaaugacc uggccgcagu gaggcggauu 1980
   gagaaggagc ucccaggagg ggcuucuggg cagacucugg ucaagaagca ucgugucugg 2040
   cguugugggg augaacuuuu uguuuuguuu cuuccuuuuu uaguucuuca aagauaggga 2100
   gggaaggggg aacaugagcc uuuguugcua ucaauccaag aacuuauuug uacauuuuuu 2160
   uuuucaauaa aacuuuucca augacauuuu guuggagcgu ggaaaaaa 2208
<210> 2
   <211> 21
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<400> 2
   ggacaggccu cuacaacuat t 21
<210> 3
   <211> 21
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<400> 3
   uaguuguaga ggccugucct t 21
<210> 4
   <211> 21
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<400> 4
   ggacaggccu guacaacuat t 21
<210> 5
   <211> 21
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<400> 5
   uaguuguaca ggccugucct t 21

## Claims

1. A compound of formula I 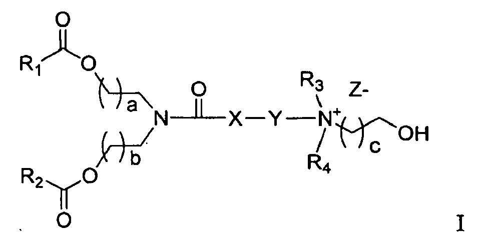 wherein R₁ and R₂ is independently selected from a group consisting of C₁₂ to C₁₈ alkyl, C₁₂ to C₁₈ alkenyl, and oleyl group; wherein R₃ and R₄ are independently selected from a group consisting of C₁ to C₆ alkyl, and C₂ to C₆ alkanol; wherein X is selected from a group consisting of -CH₂-, -S-, and -O- or absent; wherein Y is selected from -(CH₂)ₙ, -S(CH₂)ₙ, -O(CH₂)ₙ-, thiophene, and -SO₂(CH₂)ₙ-, wherein n = 1-4; wherein a = 1-4; wherein b=1-4; wherein c=1-4; and wherein Z is a counterion, preferably a halogen or mesylate and wherein said alkenyl group is derived from an alkyl group and contains in the straight or branched hydrocarbon chain one or more double bonds.

2. The compound of claim 1 that is selected from the group consisting of

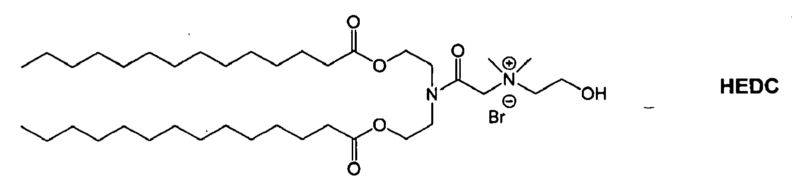

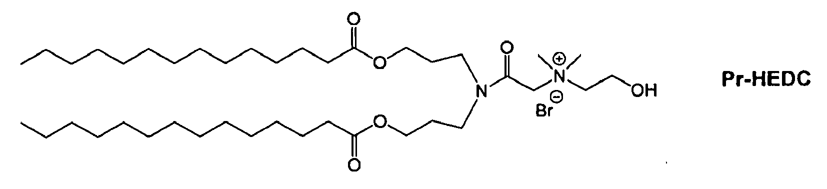

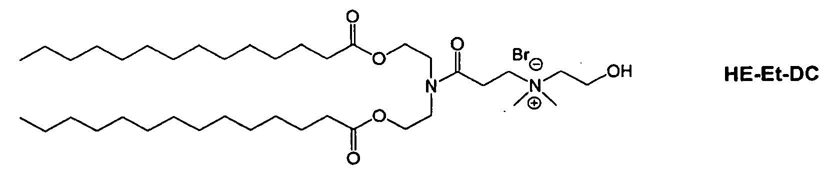

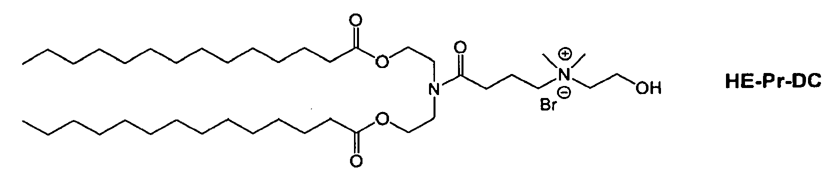

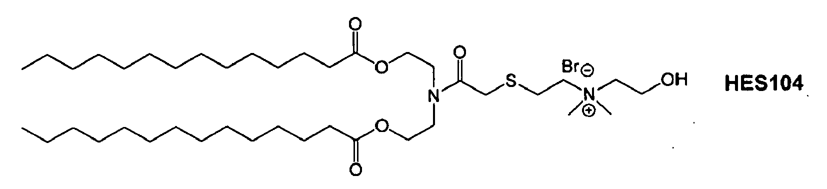

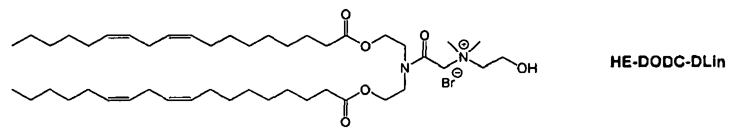

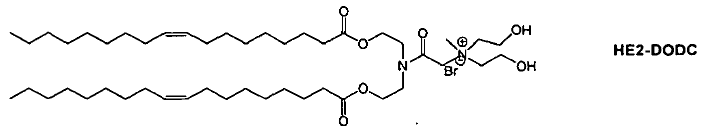

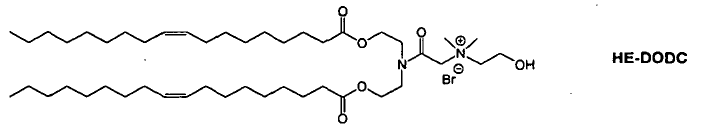

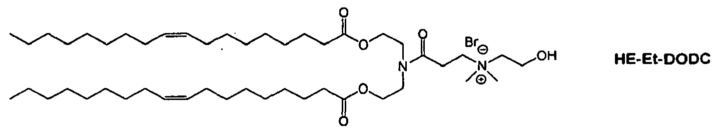

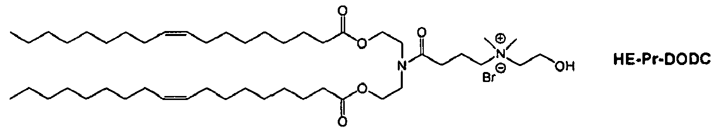

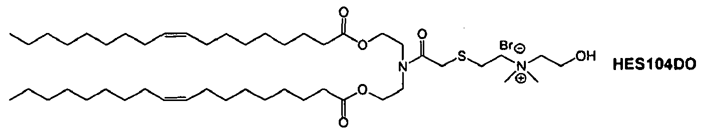

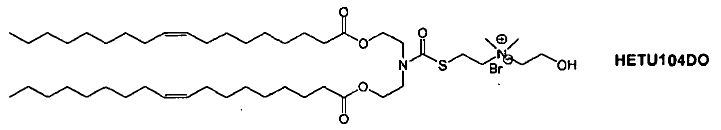

3. A composition comprising a cationic lipid consisting of the compound according to claim 1 or 2, preferably

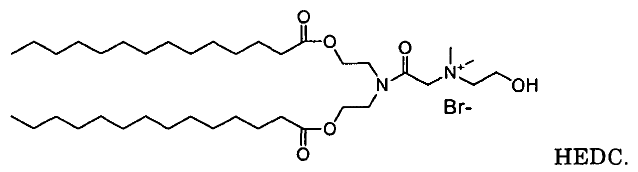

4. The composition of claim 3, comprising a liposomal composition.

5. The composition of claim 4, further comprising a lipid selected from the group consisting of a PEG-lipid, preferably PEG-DMPE (1,2-dimyristoleoyl-sn-glycero-3-phosphoethanolamine-N-PEG), an ionizable cationic lipid, preferably 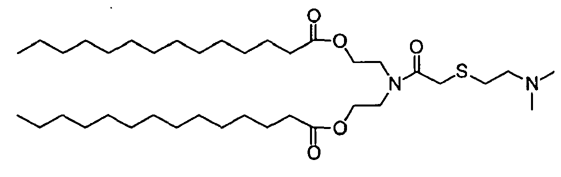 S104, and a non-cationic lipid, preferably selected from cholesterol and phospholipid such as 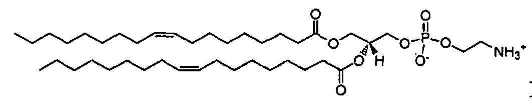 DOPE.

6. The composition of any one of claims 3-5, further comprising a retinoid molecule.

7. The composition of claim 6, wherein the retinoid molecule is a retinoid conjugate selected from the group consisting of retinoid-linker-retinoid or retinoid-linker-lipid.

8. The composition of claim 7, wherein the retinoid conjugate is of formula 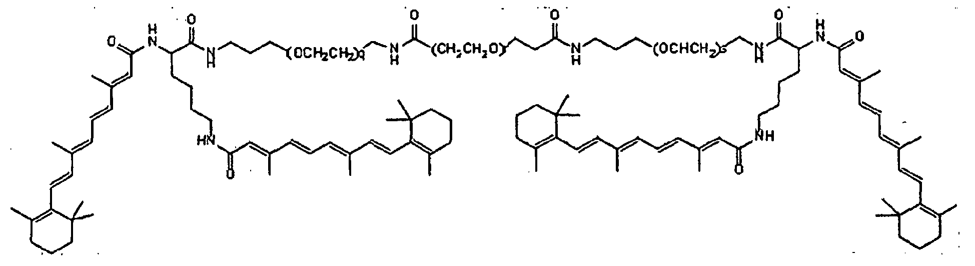 wherein q, r, and s are each independently 1, 2, 3, 4, 5, 6, 7, 8, 9, or 10.

9. The composition of claim 8, wherein the retinoid conjugate is of formula

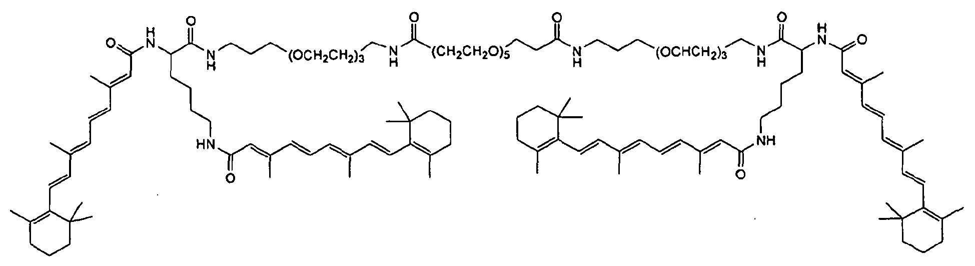

10. The composition of claims 3-9 further comprising a therapeutic agent.

11. The composition of claim 10, wherein the therapeutic agent is a nucleic acid, preferably an siRNA.

12. The composition of any one of claims 10-11 for use in the treatment of a condition **characterized by** abnormal fibrosis, optionally selected from cancer and a fibrotic disease

## Patentansprüche

1. Verbindung der Formel (I): 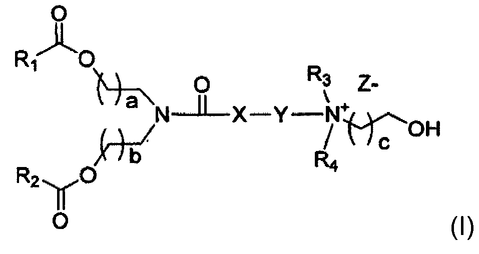 worin R₁ und R₂ unabhängig aus einer Gruppe bestehend aus C₁₂₋₁₈-Alkyl-, C₁₂₋₁₈-Alkenyl- und Oleylgruppen ausgewählt sind; worin R₃ und R₄ unabhängig aus einer Gruppe bestehend aus C₁₋₆-Alkyl und C₂₋₆-Alkanol ausgewählt sind; worin X aus einer Gruppe bestehend aus -CH₂-, -S- und -O- ausgewählt ist oder nicht vorhanden ist; worin Y aus -(CH₂)ₙ, -S(CH₂)ₙ, -O(CH₂)ₙ-, Thiophen und -SO₂(CH₂)ₙ- ausgewählt ist, worin n = 1 bis 4; worin a = 1 bis 4; worin b = 1 bis 4; worin c = 1 bis 4; und worin Z ein Gegenion, vorzugsweise ein Halogen oder Mesylat, ist und wobei die Alkenylgruppe von einer Alkylgruppe abgeleitet ist und in der geradkettigen oder verzweigten Kohlenwasserstoffkette eine oder mehrere Doppelbindungen enthält.

2. Verbindung gemäss Anspruch 1, ausgewählt aus der Gruppe bestehend aus:

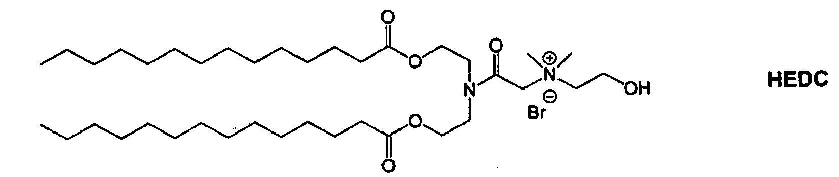

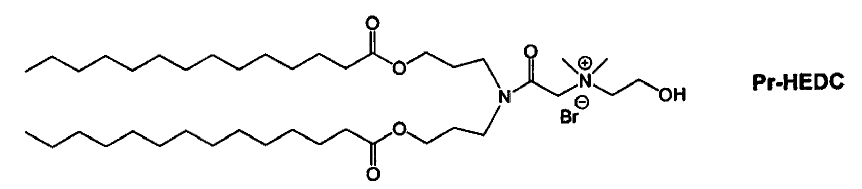

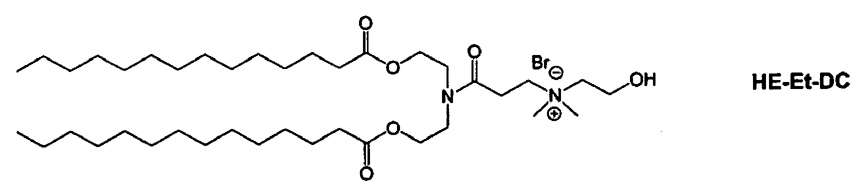

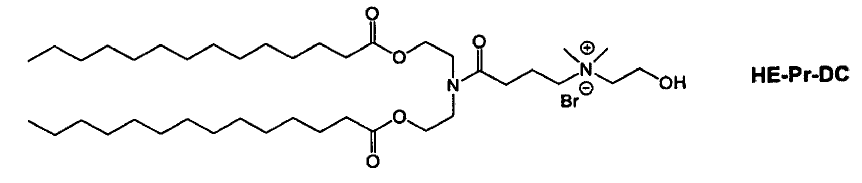

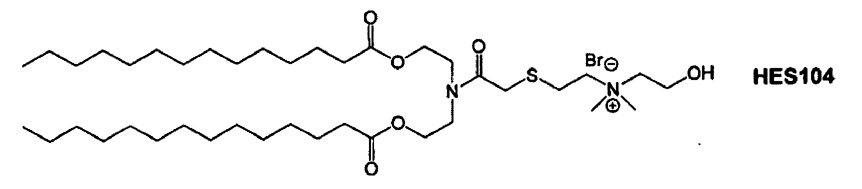

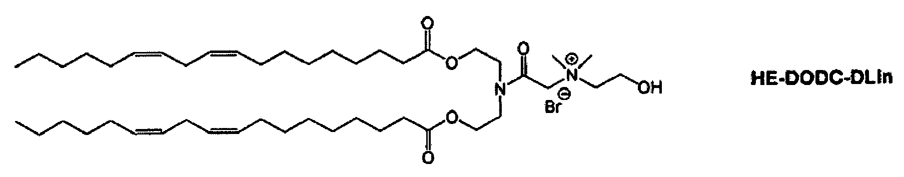

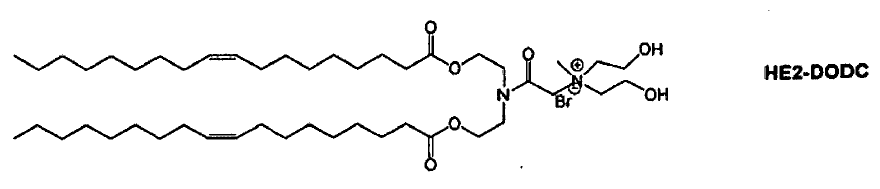

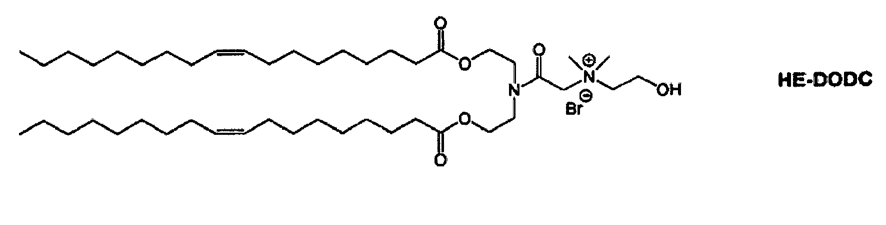

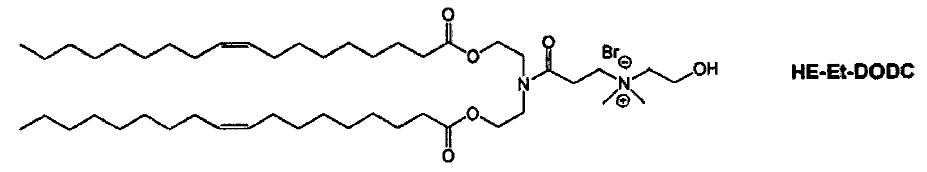

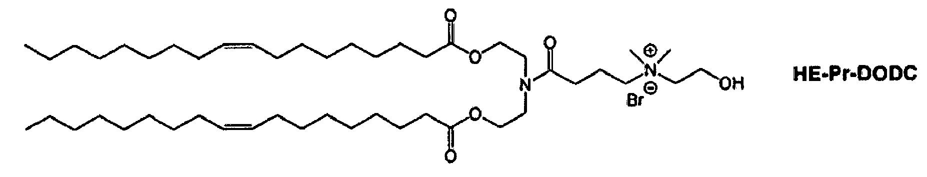

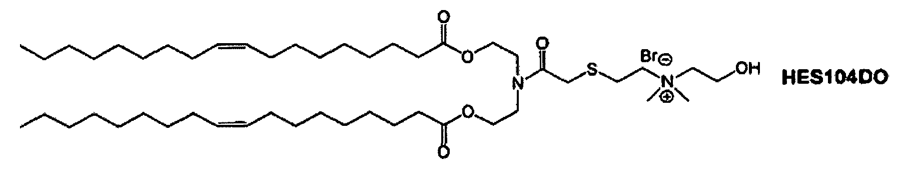

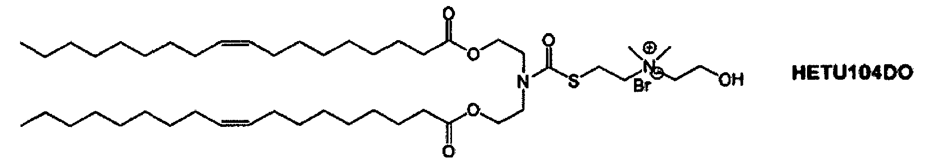

3. Zusammensetzung, umfassend ein kationisches Lipid, das aus einer Verbindung gemäss Anspruch 1 oder 2, vorzugsweise 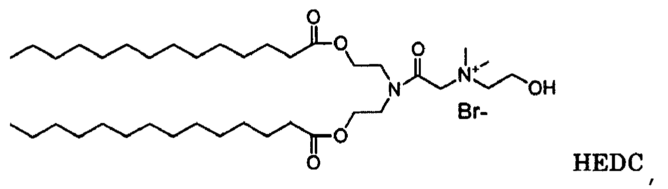 besteht.

4. Zusammensetzung gemäss Anspruch 3, die eine liposomale Zusammensetzung umfasst.

5. Zusammensetzung gemäss Anspruch 4, die ferner ein Lipid, ausgewählt aus der Gruppe bestehend aus einem PEG-Lipid, vorzugsweise PEG-DMPE (1,2-Dimyristoleoyl-sn-glycero-3-phosphoethanolamin-N-PEG), ein ionisierbares kationisches Lipid, vorzugsweise 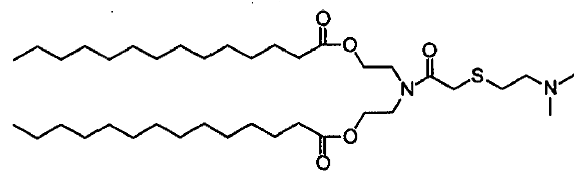 S104, und ein nicht-kationisches Lipid, vorzugsweise ausgewählt aus Cholesterin und Phospholipid, wie 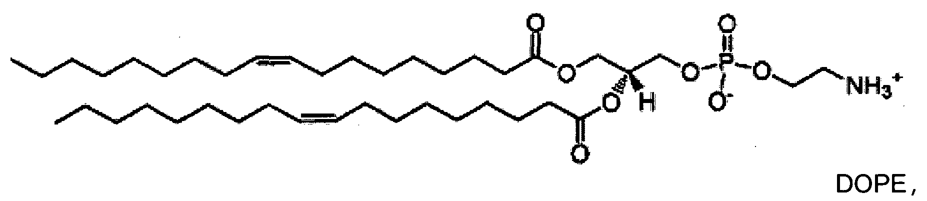 umfasst.

6. Zusammensetzung gemäss irgendeinem der Ansprüche 3 bis 5, die ferner ein Retinoidmolekül umfasst.

7. Zusammensetzung gemäss Anspruch 6, wobei das Retinoidmolekül ein Retinoidkonjugat, ausgewählt aus der Gruppe bestehend aus einem Retinoid-Linker-Retinoid oder einem Retinoid-Linker-Lipid, ist.

8. Zusammensetzung gemäss Anspruch 7, wobei das Retinoidkonjugat die Formel: 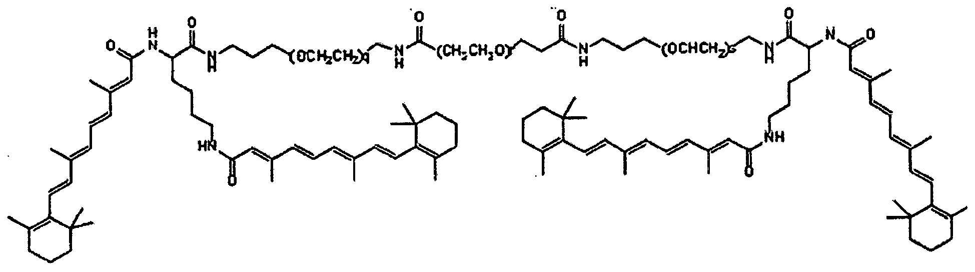 aufweist, worin q, r und s jeweils unabhängig 1, 2, 3, 4, 5, 6, 7, 8, 9 oder 10 sind.

9. Zusammensetzung gemäss Anspruch 8, wobei das Retinoidkonjugat die Formel: 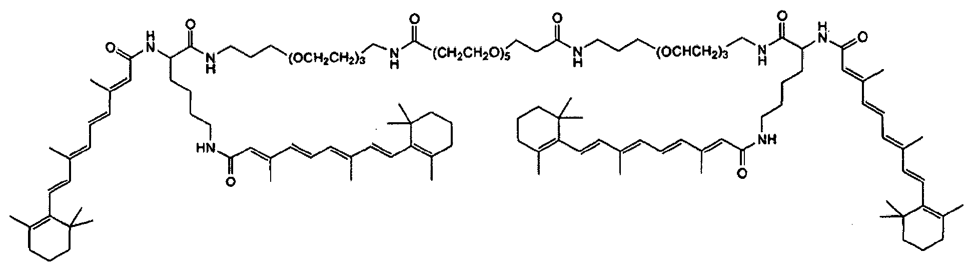 aufweist.

10. Zusammensetzung gemäss den Ansprüchen 3 bis 9, die ferner ein therapeutisches Mittel umfasst.

11. Zusammensetzung gemäss Anspruch 10, wobei das therapeutische Mittel eine Nukleinsäure, vorzugsweise siRNA, ist.

12. Zusammensetzung gemäss irgendeinem der Ansprüche 10 bis 11 zur Verwendung bei der Behandlung eines Zustands, der durch abnorme Fibrose, gegebenenfalls ausgewählt aus Krebs und einer fibrotischen Erkrankung, gekennzeichnet ist.

## Revendications

1. Composé de formule I 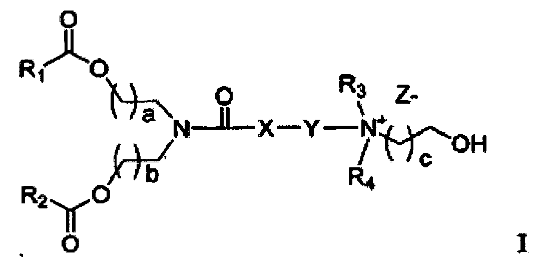 dans laquelle R₁ et R₂ sont choisis indépendamment dans le groupe consistant en un alkyle en C₁₂ à C₁₈, un alcényle en C₁₂ à C₁₈ et un groupe oléyle ; dans laquelle R₃ et R₄ sont choisis indépendamment dans le groupe consistant en un alkyle en C₁ à C₆ et un alcanol en C₂ à C₆ ; dans laquelle X est choisi dans le groupe consistant en CH₂, -S- et -O- ou est absent ; dans laquelle Y est choisi parmi - -(CH₂)ₙ, -S(CH₂)ₙ, -O(CH₂)ₙ, le thiophène et -SO₂(CH₂)ₙ-, où n = 1 à 4 ; dans laquelle a = 1 à 4 ; dans laquelle b = 1 à 4 ; dans laquelle c = 1 à 4 ; et dans laquelle Z est un contre-ion, de préférence un halogène ou un mésylate et dans lequel ledit groupe alcényle est dérivé d'un groupe alkyle et contient dans la chaîne hydrocarbonée linéaire ou ramifiée une ou plusieurs liaisons doubles.

2. Composé selon la revendication 1, qui est choisi dans le groupe consistant en

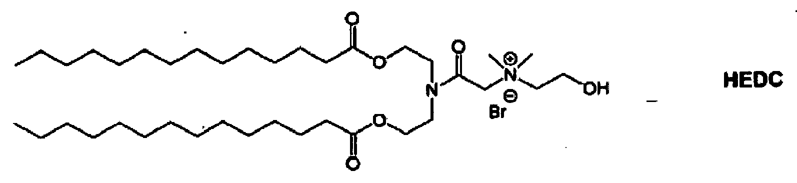

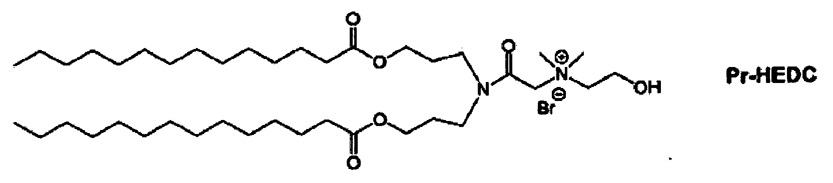

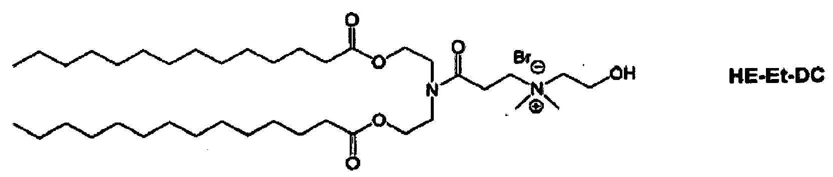

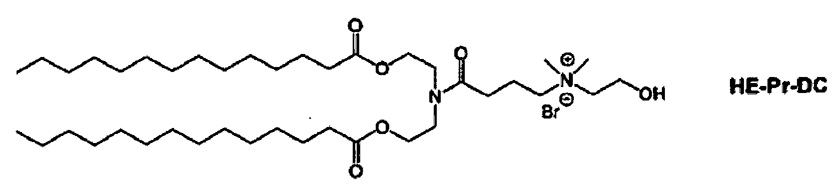

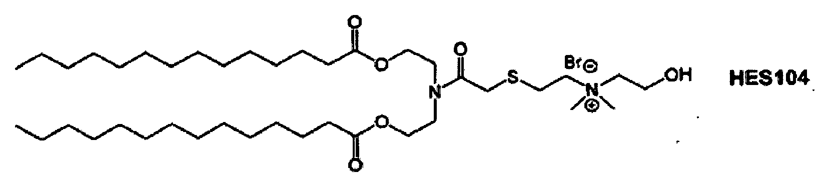

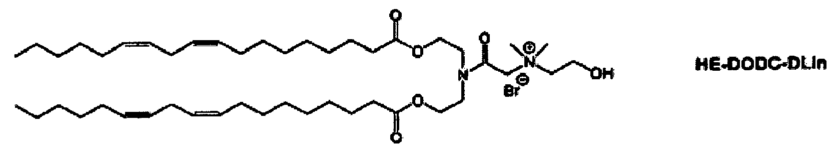

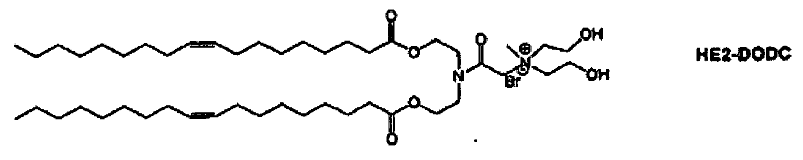

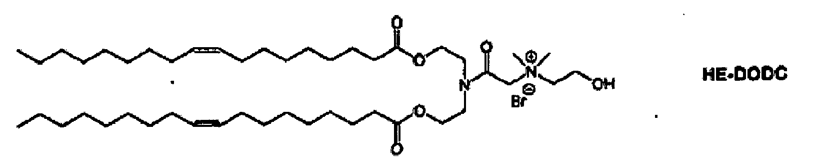

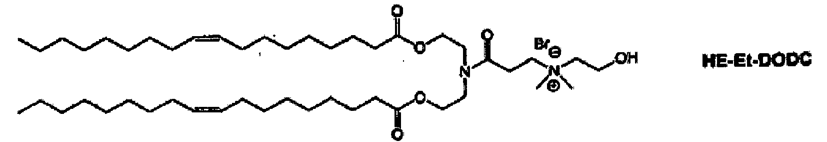

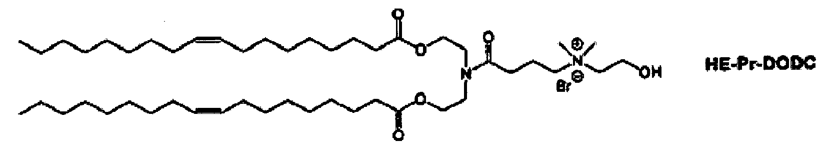

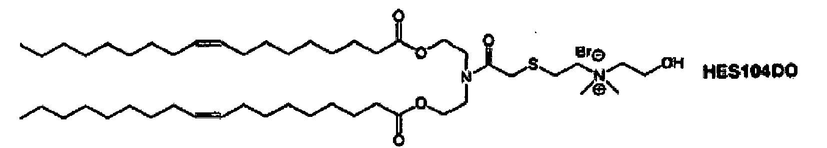

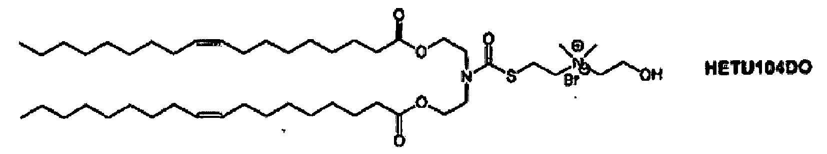

3. Composition comprenant un liquide cationique consistant en le composé selon la revendication 1 ou 2, de préférence

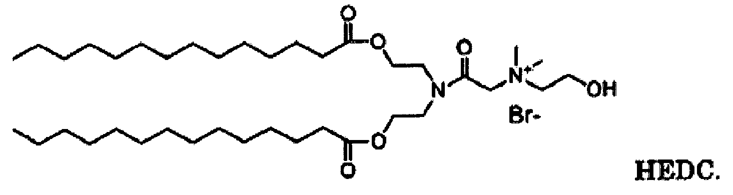

4. Composition selon la revendication 3, comprenant une composition de liposome.

5. Composition selon la revendication 4, comprenant en outre un lipide choisi dans le groupe consistant en un PEG-lipide, de préférence la PEG-DMPE (1,2-dimyristoléoyl-sn-glycéro-3-phosphoéthanolamine-N-PEG), un lipide cationique ionisable, de préférence 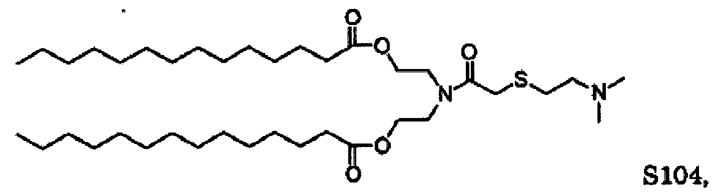 et un lipide non cationique, choisi de préférence parmi le cholestérol et un phospholipide tel que

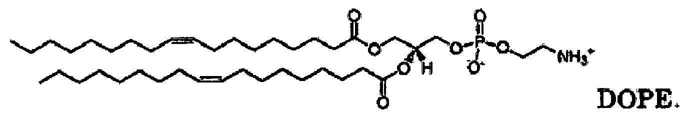

6. Composition selon l'une quelconque des revendications 3 à 5, comprenant en outre une molécule rétinoïde.

7. Composition selon la revendication 6, dans laquelle la molécule rétinoïde est un conjugué rétinoïde choisi dans le groupe consistant en un rétinoïde-lieur-rétinoïde ou un rétinoïde-lieur-lipide.

8. Composition selon la revendication 7, dans laquelle le conjugué rétinoïde est de formule 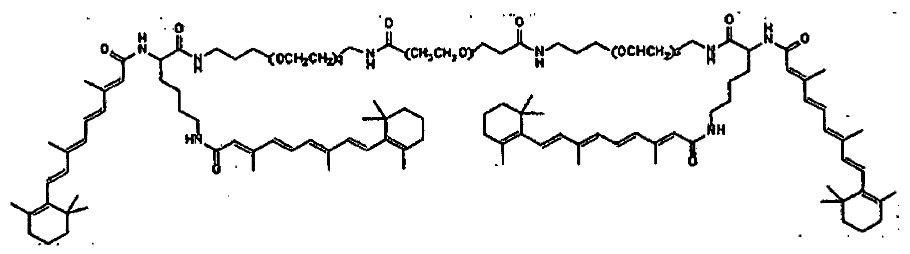 dans laquelle q, r et s sont chacun indépendamment 1, 2, 3, 4, 5, 6, 7, 8, 9 ou 10.

9. Composition selon la revendication 8, dans laquelle le conjugué rétinoïde est de formule

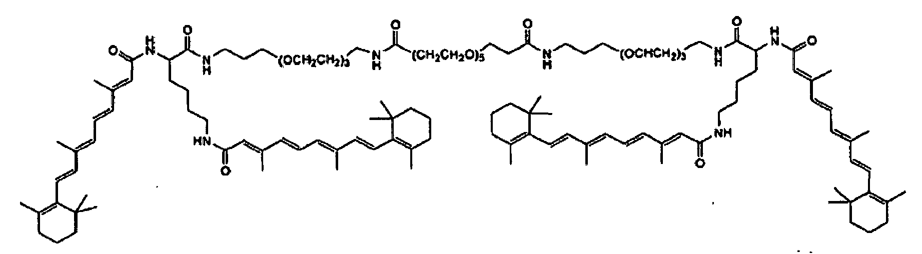

10. Composition selon les revendications 3 à 9, comprenant en outre un agent thérapeutique.

11. Composition selon la revendication 10, dans laquelle l'agent thérapeutique est un acide nucléique, de préférence un ARNsi.

12. Composition selon l'une quelconque des revendications 10 à 11, pour son utilisation dans le traitement d'une affection **caractérisée par** une fibrose anormale, choisie facultativement parmi un cancer et une maladie fibrogène.
